(19) Europäisches Patentamt / European Patent Office / Office européen des brevets

(11) **EP 4 130 252 A1**

(12) **EUROPEAN PATENT APPLICATION**

(43) Date of publication:
**08.02.2023 Bulletin 2023/06**

(21) Application number: **22197497.5**

(22) Date of filing: **15.02.2019**

(51) International Patent Classification (IPC):
*C12N 5/0775* $^{(2010.01)}$     *A61K 35/28* $^{(1974.07)}$
*A61K 35/12* $^{(1974.07)}$     *C12N 5/073* $^{(2010.01)}$

(52) Cooperative Patent Classification (CPC):
**C12N 5/0668;** A61K 35/00; C12N 5/0605

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.02.2018 EP 18157070**

(62) Document number(s) of the earlier application(s) in accordance with Art. 76 EPC:
**19706500.6 / 3 752 598**

(71) Applicant: **NextCell Pharma AB
141 57 Huddinge (SE)**

(72) Inventor: **Svahn, Mathias Gösta
13141 Nacka (SE)**

(74) Representative: **AWA Sweden AB
Box 45086
104 30 Stockholm (SE)**

Remarks:
This application was filed on 23.09.2022 as a divisional application to the application mentioned under INID code 62.

(54) **COMPOSITION OF ALLOGENEIC MESENCHYMAL STEM CELLS**

(57)     The present disclosure relates to allogeneic populations of mesenchymal stem/stromal cells and related compositions, which populations and compositions comprise cells pooled from multiple donors, and their use in therapy and/or prevention of disease, such as diabetes type 1. The present disclosure also relates to methods for obtaining said composition.

Figure 1

EP 4 130 252 A1

**Description**

**TECHNICAL FIELD**

The present disclosure relates to allogeneic populations of mesenchymal stem/stromal cells and related compositions, which populations and compositions comprise cells pooled from multiple donors, and their use in therapy and/or prevention of disease, such as diabetes type 1. The present disclosure also relates to methods for obtaining said composition.

**BACKGROUND**

Mesenchymal stem cells (MSCs) are non-hematopoietic cells expressing the surface markers CD73, CD90, and CD105 while lacking the expression of CD14, CD34, and CD45. When expanded as polyclonal cultures, they are a heterogenous population of cells with retained capacity for self-renewal and differentiation into various forms of mesenchyme (Dominici, et al. (2006), The International Society for Cellular Therapy position statement. Cytotherapy 8: 315-317). *In vitro,* MSCs must be adherent to plastic under standard tissue culture conditions, and have the capacity to differentiate into osteoblasts, adipocytes, and chondroblasts. MSCs can be found not only in bone marrow, in which they were originally found, but also in almost all other forms of tissues e.g., Wharton's jelly and the placenta.

MSCs have the capacity to promote survival, angiogenesis and tissue repair and modulate responses by innate and adaptive immune cells (Berman et al, (2010) Diabetes 59: 2558-2568). Thus, transplantation of MSCs presents an attractive therapeutic option for example in the fields of autoimmune disease, inflammatory disease and transplant rejection.

Diabetes mellitus type 1 (also known as type 1 diabetes, T1D) is a form of diabetes in which insufficient insulin is produced. The underlying mechanism involves an autoimmune destruction of the insulin-producing beta cells in the pancreas. At onset of T1D beta-cell mass has regularly decreased to 20-40% compared to normal levels. Maintenance of residual insulin secretion is important in contributing to lower HbA1c, less blood glucose fluctuations, and diminished risk of ketoacidosis (Madsbad et al., (1979) Br Med J; 2(6200): 1257-9; Steffes et al., (2003) Diabetes Care; 26(3): 832-6). It also substantially decreases the risks of severe hypoglycemic events and late complications. An identified successful intervention may not only be used to prevent disease but also be applied to patients with ongoing disease before overt hyperglycemia, thereby providing means to prevent disease development.

Different intervention strategies have been tested to save residual beta-cells but have at best only temporarily preserved endogenous insulin production.

The first clinical trial that used transplantation of MSCs expanded in culture was published in 1995 (Lazarus et al., (1995) Bone Marrow Transplant 16: 557-564). Since then, many clinical trials with MSCs for the treatment of diverse diseases have been published, and many are ongoing.

However, a limiting factor for the use of MSCs in transplantation is the difficulty to obtain large batches of cells with desired properties. Furthermore, expansion of cells from one donor is generally used for production of a single batch, the next batch will typically use cells from another donor. Hence, the batch-to-batch variability is of major concern for both safety and efficacy.

These challenges cause the cell therapy industry to go through cumbersome manufacturing processes with extensive testing and as consequence of excessive expansion, the cells might lose their potency and/or exhibit an increased risk for genetic instability. Additionally, when expansion of cells is done on a case to case basis, and this it is difficult to ensure optimal dosage of MSCs for the recipient patient and "giving the patient the number of cells we managed to expand" is a common approach. This makes treatment outcomes as well as potential adverse side effects highly unpredictable. Furthermore, the process of finding a suitable donor may be time consuming and laboursome, and carries an uncertainty regarding if a suitable donor will be found or not. Additionally, there is a risk that patients develop antibodies against the transplanted MSCs. In particular, a dose response relation between MSCs administered and the patient developing antibodies directed against the MSCs is expected. This effect can be profound by multiple administrations.

Thus, there is a large need in the field to provide a population of MSCs suitable for treatment and/or prevention of autoimmune disease, inflammatory disease and transplant rejection, which MSC population enables administration of a suitable dosage of cells to a patient in need thereof. The production should ensure a robust manufacturing process with little variations between batches and every batch should yield multiple doses. The cells need to have proven potency and be formulated to minimize the risk of allosensitization and/or donor specific antibodies. It is furthermore desirable that said population is instantaneously available to a patient without the need for donor-recipient matching.

**SUMMARY OF THE DISCLOSURE**

The object of the present disclosure is to provide methods, agents and treatments for inflammatory diseases or conditions, autoimmune disease and transplantation rejection that overcome the drawbacks of the prior art. It is envisioned that treatments with the isolated, pooled allogenic MSC population as described herein are an interesting therapeutic option.

Thus, the present disclosure aims at providing a MSC population suitable for transplantation to a patient in need thereof, which population comprises potent cells, exhibits low, or even no statistically significant, batch-to-batch variability and results in low alloimmunization or allosensitization in treated patients. The present object is achieved by an isolated, pooled allogenic MSC population obtained by the method disclosed, which employs the selection algorithm as described herein.

As used herein, the term "selection algorithm" refers to step 2-5 of the method defined below, in other word to all method steps disclosed except the culturing or providing step and the pooling step. It will be understood that further steps may be added to the selection algorithm without falling outside the scope of the present disclosure.

Thus, in a first aspect of the present disclosure, there is provided a method for obtaining an isolated, pooled allogeneic mesenchymal stem cell (MSC) population comprising MSCs derived from at least 3 individual donors, wherein the number of cells derived from any one donor does not exceed 50% of the total cell number and wherein said MSCs have at most been subject to eight passages;
comprising the steps of:

- culturing or providing MSCs from more than said at least 3 individual donors to obtain more than at least 3 individual donor derived MSC populations;
- assaying each individual donor derived MSC population using at least 3 assays to obtain at least 3 assay results for said each individual donor derived MSC population, wherein said at least 3 assays comprise at least one assay measuring the immunosuppressive capacity each individual donor derived MSC population;
- for each assay allocating an individual ranking score value to said each individual donor derived MSC population based on the assay result and thus obtaining at least 3 individual ranking score values for each individual donor derived MSC population, wherein a higher ranking score value is indicative of more desirable assay result; or wherein a lower ranking score value is indicative of more desirable assay result;
- allocating a total score value to each individual donor derived MSC population based on said at least 3 individual ranking score values, wherein in the case of a higher ranking score value being indicative of more desirable assay result, a higher total score value is indicative of more desirable population properties; or wherein in the case of a lower ranking score value being indicative of more desirable assay result, a lower total score value is indicative of more desirable population properties;
- selecting a subset of individual donor derived MSC populations with desirable population properties based on their total score values; and pooling said selected individual donor derived MSC populations to obtain an isolated, pooled allogeneic MSC population,

wherein said pooled allogeneic MSC population is not further cultured after the pooling step and wherein said at least 3 assays comprise one assay measures indoleamine-2,3-dioxygensase (IDO) activity; one assay measuring prostaglandin E2 secreted by said MSCs; and one assay measuring the effect of said MSCs on the proliferation of peripheral blood mononuclear cells (PBMCs).

The present disclosure provides a method for obtaining an isolated, pooled allogeneic MSC population comprising cells from at least 3 individual donors, wherein the number of cells derived from any one donor does not exceed 50% of the total cell number, thus ensuring that the population comprises a significant number of cells derived from each donor and that cells derived from any one donor are not dominant in the population. It is considered beneficial that the population comprises similar numbers or numbers in the same range of cells derived from different individual donor. The present inventors expect that an isolated, pooled allogeneic MSC population obtained according to the present method will exhibit low immunogenic properties. The selection algorithm is used herein to select cells with desired functionalities. Furthermore, the pooling of cells from multiple donors meeting the criteria of the selection algorithm will decrease batch-to-batch variability. The method also ensures that the isolated, pooled allogeneic MSC population comprises potent cells, as the selection algorithm functions to select cells with desirable properties. Additionally, the method as described herein allows for obtaining large batches of cells due to the pooling step. In particular, large batches of cells may be obtained, which cells have been subjected to a low number of passages. Additionally, large batches also allow for reduction in manufacturing costs. In contrast, if cells are not pooled, it is difficult to obtain large batches of cells, especially if cells are subjected to a low number of passages. Furthermore, a low passage number is associated with high potency in MSCs and it is therefore desirable that cells are not exposed to excessive numbers of passages. For clarity, a subculture is a new cell or microbiological culture made by transferring some or all cells from a previous culture to fresh growth medium. This action is called subculturing or passaging the cells. To record the approximate number of divisions cells have had in culture the number of passages may be recorded. As used herein, the term "passage" refers to transferring cells from a previous culture to fresh growth medium.

Thus, in one embodiment there is provided a method as disclosed herein, wherein said MSC in the isolated, pooled allogeneic MSC population have at most been subject to seven passages, such as at most six passages, such as at most five passages, such as at most four passages, such as at most three passages, such as one, two or three passages, such as two or three passages. It is to be appreciated that the number of passages is related to the number of cells present in the culture. Thus, it may be beneficial to retain a balance between cell number and maintained potency in order to obtain a sufficient number of cells with desirable properties. Thus, in some embodiments the said MSC have been subject to from 2 to 6, such as from 2 to 5, such as from 2 to 4, such as from 2 to 3 passages.

Mesenchymal stem cells (MSCs) are non-hematopoietic cells expressing the surface markers CD73, CD90, and CD105 while lacking the expression of CD14, CD34, and CD45. *In vitro,* MSCs must be adherent to plastic under standard tissue culture conditions, and have the capacity to differentiate into osteoblasts, adipocytes and chondroblasts. As used herein, the terms "MSCs", "mesenchymal stem cells", "mesenchymal stromal cells" and "marrow stromal cells" refer to cells with the above-mentioned properties. The present disclosure adheres to the definition of MSC according to the criteria of the International Society for Cellular Therapy (ISCT). MSCs can be derived from bone marrow, peripheral blood, adipose tissue, dental tissue, placenta, umbilical cord, amniotic fluid, cord blood, Wharton Jelly, decidua, chondrion membrane and amnion membrane.

Thus, in one embodiment, there is provided a method as disclosed herein, wherein said MSCs are selected from the group consisting of bone marrow derived MSCs, peripheral blood derived MSCs, adipose tissue derived MSCs, dental tissue derived MSCs, placenta derived MSCs, umbilical cord derived MSCs, amniotic fluid derived MSC, cord blood derived MSCs, Wharton Jelly derived MSCs, decidua derived MSCs, chondrion membrane derived MSCs and amnion membrane derived MSCs. In particular embodiments, said MSCs are selected from the group consisting of placenta derived MSCs, umbilical cord derived MSCs, amniotic fluid derived MSC, cord blood derived MSCs, Wharton Jelly derived MSCs, decidua derived MSCs, chondroid membrane derived MSCs, dental pulp and amnion membrane derived MSCs; such as placenta derived MSCs, umbilical cord derived MSCs, amniotic fluid derived MSC, cord blood derived MSCs, Wharton Jelly derived MSCs, decidua derived MSCs, dental pulp derived MSCs and amnion membrane derived MSCs; such as placenta derived MSCs, umbilical cord derived MSCs, amniotic fluid derived MSC, cord blood derived MSCs, Wharton Jelly derived MSCs, dental pulp derived MSCs; such as placenta derived MSCs, umbilical cord derived MSCs, cord blood derived MSCs and Wharton Jelly derived MSCs; such as umbilical cord derived MSCs, cord blood derived MSC and Wharton Jelly derived MSCs. In one embodiment, said MSCs are umbilical cord derived MSCs or Wharton Jelly derived MSCs, such as Wharton Jelly derived MSCs.

In some embodiments of the inventive method, it may be beneficial that the isolated, pooled allogeneic MSC population comprises MSCs derived from more than 3 donors in order to ensure that the concentration of any allogenic Human Leukocyte Antigen (HLA) will be lower than when cells from a single donor or from few donors were used. It is envisioned that this will reduce the risk of generation of anti-HLA antibodies (i.e. Donor specific antibodies, DSA) in patients administered the isolated, pooled allogeneic MSC population. The present inventors expect that low concentration of any specific HLA allele in the isolated, pooled allogeneic MSC population will reduce the risk of adverse effect connected to single and multiple administrations of said cells. Additionally, by using cells from multiple donors, low batch variability can be obtained. The donor to donor variability between donors that have qualified for manufacturing and that have passed all GMP quality criteria in the expansion to large scale clinical grade Drug Product is reduced by up to 40 % or even more when comparing the results from all donors with the results from the donors selected for pooling. The reduction in variation for specific assays are presented in Example 12 which generates an overall assessment reduction of variation, based on the selection algorithm, of up to 40 % or oven more between selected donors and all donors evaluated for a specific batch.

The GMP production of MSC will dramatically reduce the donor variability and the Selection algorithm will further reduce the variation by up to 40 % or even more resulting in batch-to-batch variation without statistical significance.

Thus, in one embodiment, said population comprises MSCs derived from at least four individual donors, such as at least five individual donors, such as at least six individual donors, such as at least seven individual donors, such as at least eight individual donors, such as at nine individual donors, such as at least ten individual donors. In another embodiment, the isolated, pooled allogeneic MSC population comprises MSCs derived from 3-20 individual donors, such as 3-15 individual donors, such as 3-10 individual donors, such as 4-8 individual donors, such as 5-7 individual donors, such as 5, 6 or 7 individual donors. In one particular embodiment, said step of assaying each individual donor derived MSC population comprises assaying at least one more, such as at least two more, such as at least three more, such as at least four more, such as at least five more, such as at least six more, such as at least seven more, such as least eight more, such as at least nine more, such as at least ten more individual donor derived MSC population than the number of individual donor derived MSC populations pooled in the pooling step. In one particular embodiment, said the step of assaying each individual donor derived MSC population comprises assaying at least 1-4 times, such as 2-4 times, such as 2-3 or 3-4 times, as many individual donor derived MSC population as the number of individual donor derived MSC populations pooled in the pooling step. Thus, for example, if the isolated, pooled allogeneic MSC population comprises MSC derived from 3 individual donor derived MSC populations, the step of assaying each individual donor derived MSC population comprises assaying 3-12, such as 6-12, such as 6-9 or 9-12 individual donor derived MSC populations. In this example, only 3 individual donor derived MSC populations would be selected for pooling, while the remaining individual donor derived MSC populations would be discarded.

In one embodiment of the disclosure, said step of culturing or providing MSCs comprises culturing or providing MSCs from at least 4 individual donors to obtain said at least 4 individual donor derived MSC populations, such as at least 5, such as at least 6, such as at least 7, such as at least 8, such as at least 9, such as at least 10, such as at least 11, such as at least 12, such as at least 13, such as at least 14, such as at least 15, such as at least 16, such as at least 17, such as at least 18, such as at least 19, such as at least 20 individual donor derived MSC populations. For example, from about 3 to about 50 individual donor derived MSC populations, such as from about 4 to about 50, such as from about 5 to about 50, such as from about 6 to about 50, such as from about 6 to about 30, such as from about 6 to about 20, such as from about 6 to about 15, such as from about 8 to about 12 individual donor derived MSC population may be provided or cultured.

In some embodiments of the method as disclosed herein, the step of assaying said each individual donor derived MSC population, comprises assaying at least 3, such as at least 4, such as at least 5, such as at least 6, such as at least 7, such as at least 8, such as at least 9, such as at least 10, such as at least 11, such as at least 12, such as at least 13, such as at least 14, such as at least 15, such as at least 16, such as at least 17, such as at least 18, such as at least 19, such as at least 20 individual donor derived MSC populations, In another embodiment, the step of assaying said each individual donor derived MSC population comprises assaying from about 3 to about 50 individual donor derived MSC populations, such as from about 4 to about 50, such as from about 5 to about 50, such as from about 6 to about 50, such as from about 6 to about 30, such as from about 6 to about 20, such as from about 6 to about 15, such as from about 8 to about 12 individual donor derived MSC populations. In one embodiment, from about 3 to about 20 individual donor derived MSC populations are assayed, for example, 8, 9, 10, 11, 12, 13, 14 individual donor derived MSC population may be assayed. It is to be understood that the individual donor derived MSC populations assayed in the present step of the method disclosed herein, are obtained in the culture or provision step according to the present method.

In some embodiments, it may be beneficial that said each individual donor derived MSC population is assayed with at least one or more functional assay in order to obtain more information about the properties of each MSC population. Thus, in one embodiment of the method as disclosed herein, the step assaying each individual donor derived MSC population using at least 3 assays comprises using as least one functional assay, such as at least two functional assays, such as at least three functional assays, such at least four functional assays, such least five functional assays. The skilled person will appreciate that said assays may be tailored to reflect the properties which are desired in the MSC population. For example, in the case wherein it is desired that the MSC population is immunosuppressive, the assays may be selected to reflect this property of interest.

In one embodiment, said at least 3 assays comprise at least one assay measuring the immunosuppressive capacity of said MSCs. As used herein, the term "immunosuppressive capacity" refers to the capacity to elicit a reduction of the activation or efficacy of the immune system. The skilled person will appreciate that the immunosuppressive capacity may be measured directly or indirectly in an assay.

In one embodiment, said wherein said at least one assay measuring the immunosuppressive capacity of said MSCs measures indoleamine-2,3-dioxygensase (IDO) activity. An immunosuppressive potential may reported as a measure of IDO activity, determined by measuring tryptophan and kynurenine in the culture supernatant. IDO is a heme-containing enzyme that in humans is encoded by the IDO1 gene. The IDO enzyme converts L-tryptophan to N-formylkynurenine (or kynurenine), an immunosuppressive molecule that acts as an inhibitor of immune cell proliferation, including T cells. The IDO activity may be presented as the ratio of kynurenine/tryptophan and can be determined by calculating the amount of tryptophan and kynurenine present in cell culture supernatants for example using an ELISA kit. When stimulated with interferon gamma (IFNy), mesenchymal stem/stroma cells (MSCs) secrete more IDO than when they are unstimulated.

Inducible IDO activity indicates that the cells have functional potency, related to immunomodulation and/or immunosuppression which the present inventors consider a key quality attribute of the MSCs used in this method.

It is possible to quantitatively measure the immunosuppressing effect the MSCs have on the proliferation of peripheral blood mononuclear cells (PBMC). MSCs have been shown to suppress T-lymphocyte proliferation. Mixed lymphocyte reactions with MSC are frequently used to demonstrate the immunosuppressive activity of MSC. In one embodiment, said least one assay measuring the immunosuppressive capacity of said MSCs measures the effect of said MSCs on the proliferation of peripheral blood mononuclear cells (PBMCs), such as T-lymphocytes. For example, the proliferation of T-lymphocytes, such as proliferation of phytohemagglutinin (PHA) stimulated T-lymphocytes. PHA is used as a mitogen which activates proliferation of T-lymphocytes. Thus, in one embodiment, said proliferation of PBMCs is the proliferation of T-lymphocytes, such as proliferation of PHA stimulated T-lymphocytes. The immunosuppressive activity of MSCs may be quantified as the decrease in proliferation of PHA stimulated T-lymphocytes.

Furthermore, the MSCs may be assayed to measure prostaglandin E2 secreted by said MSCs. Prostaglandin E2 (PGE2) is formed in a variety of cells from prostaglandin H2, which is synthesized from arachidonic acid by the enzyme prostaglandin synthetase. PGE2 has a number of biological actions, including vasodilation, both anti- and proinflammatory action, modulation of sleep/wake cycles, and facilitation of human immunodeficiency virus replication. PGE2 is active in inflammation, immune regulation, generation of fever, pain perception, protection of the gastric muscosa, fertility and parturition, as well as sodium and water retention. PGE2 is rapidly metabolized *in vivo,* the half-life of PGE2 in the circulatory system is approximately 30 seconds and normal plasma levels are 3-12 pg/mL. PGE2 is involved in the regulation of different stages of the immune response and different effector mechanisms of immunity. MSCs constitutively produce PGE2, and their proliferation is regulated by this prostaglandin through the differential activation of cAMP-dependent protein kinase isoforms. This production of PGE2 is sensitive to the local environment, where inflammatory signals stimulate its induction. During co-culture with immune cells, PGE2 production by MSCs is substantially increased and participates in the immunomodulatory effects of MSCs. Moreover, the role of PGE2 in MSC-induced immunosuppressive effects depends on T-cell stimuli, as reported by Rasmusson et al. (Rasmusson et al., (2005) Exp.Cell.Res, 305 (1) (2005), pp. 33-41). PGE2 is effective in the MSC inhibition of T cells activated by PHA rather than by alloantigens. MSCs prevent lymphocyte activation and induce the inhibition of T-cell proliferation through the modulation of COX1/ COX2 expression and ultimately PGE2 production. Therefore, it is possible use the amount of PGE2 secretion found in cell culture supernatants from co-cultures of peripheral blood mononuclear cells (PBMCs) and MSCs as a measure of immunosuppressive capacity. In one embodiment, said wherein said at least one assay measuring the immunosuppressive capacity of said MSCs measures prostaglandin E2 secreted by said MSCs. In one embodiment, said at least one assay measuring prostaglandin E2 secreted by said MSCs comprises measuring prostaglandin E2 secreted by said MSCs when co-cultured with PBMCs, such as PHA stimulated PBMCs, such as PHA stimulated T-lymphocytes.

In yet another assay, the HLA-G expression in the MSCs may be measured. HLA-G has been identified as a naturally occurring tolerance-inducing molecule. It has restricted expression under physiological conditions but can be upregulated e.g. in response to IFNy, IL-10 and PHA. MSC have low levels of intracellular HLA-G and express low levels of soluble HLA-G (sHLA-G) but stimulation with IFNy or IL-10 is expected to result in increased levels. Stimulation with PHA or GABA is expected to increase soluble HLA-G levels. JEG-3, a placenta derived cell line, has a high level of HLA-G expression, both intracellular and soluble, and may be used as a positive control in the assays. The scope may be to compare both intracellular HLA-G expression for example by flow cytometry (FACS) analysis and the release of sHLA-G by for example ELISA between individual donor derived MSC populations.

Thus, in one embodiment of the method as disclosed herein, said at least 3 assays further comprise at least one assay measuring HLA-G expression in said MSCs, for example said at least one assay measures HLA-G expression in said MSCs in response to IFN$\gamma$, IL-10, PHA and/or GABA, for example said at least one assay measures HLA-G expression in said MSCs in response to IFN$\gamma$, IL-10 and/or PHA. In one embodiment, said at least one assay measures HLA-G expression in said MSCs in response one or several selected from the group consisting of IFN$\gamma$, IL-10, PHA and GABA. Said HLA-G expression may be expression of soluble HLA-G.

Additionally, the individual donor derived MSC populations may be evaluated in terms of protein expression and/or cytokine expression in order to select the populations with desired characteristics. For example, it may be of interest to evaluate the expression of interleukins, growth factors, interferon, tumor necrosis factors, colony stimulating factors and lipoproteins in said populations. Thus, in one embodiment of the method as disclosed herein, said at least 3 assays further comprise least one assay measuring the protein expression and/or cytokine expression by said MSCs, such as the expression of one or several proteins or cytokines selected from the group consisting of interleukins, growth factors, interferons, tumor necrosis factors, colony stimulating factors and lipoproteins. In another embodiment, said at least one assay measuring the protein expression and/or cytokine expression measures the expression of one or several proteins or cytokines selected from the group consisting of, IL-2, IL-4, IL-6, IL-8, IL-10, IL-12, IL- 12/13, IL-17A, IL-21, IL-22, IL-29, IL-31, TGF$\beta$, VEGF, FGF, GM-CFS, IFN$\alpha$, IFN$\gamma$, apo E and TNF$\alpha$, such as the group consisting of IL-2, IL-4, IL-6, IL-8, IL-12, IL- 12/13, IL-17A, IL-21, IL-22, IL-29, IL-31, TGF$\beta$, VEGF, FGF, GM-CFS, IFN$\alpha$, IFN$\gamma$, apo E and TNF$\alpha$, such as the group consisting of IL-6, IL-8, GM-CSF and TGF$\beta$, such as the group consisting of at least IL-6. In one particular embodiment, the expression of at least 2, such as at least 3, such as at least 4, such as at least 5, such as at least 6, such as at least 7, such as at least 8, such as at least 9, such as at least 10, such as at least 11, such as at least 12, such as at least 13, such as at least 14, such as at least 15, such as at least 16, such as at least 17, such as at least 18, such as all 19 of said proteins and/or cytokines are measured. Furthermore, the skilled person will appreciate the expression of said proteins and/or cytokines may be measured in the absence of any stimuli and/or in the presence of at least one stimulus. In one embodiment, the expression of said proteins and/or cytokines is measured in the presence of at least one stimulus or several stimuli, such as two, three, four or more stimuli. In one embodiment, said stimulus/stimuli is/are immune response modifying stimulus/stimuli. Non-limiting examples of said immune response modifying stimuli include PBMCs, stimulated PBMCs (such as PBMCs stimulated with PHA), IL10, gamma-aminobutyric acid (GABA), interferon gamma (IFNy) and other. Thus in one embodiment, said immune response modifying stimulus/stimuli is/are selected from the group consisting of PBMCs, stimulated PBMCs, PBMCs stimulated with PHA, IL10, GABA and IFNy. In one embodiment, said stimulus/stimuli is/are GABA and/or IFNy. In one embodiment, there is provided a method as disclosed herein, wherein the stimulus/stimuli is/are selected from the group consisting of polyinosinic: polycytidylic acid (Poly I:C), resiquimod (r848), GABA and IFNy, such as the group consisting of Poly I:C and IFNy. In one embodiment, said stimuli is PBMCs, such as stimulated or unstimulated PBMCs, such as PHA stimulated PBMCs, such as PHA stimulated T-lymphocytes. In one embodiment, there is provided a method as disclosed herein, wherein the stimulus /stimuli is/are PHA stimulated T-lymphocytes and/or GABA.

In one particular embodiment, said method as disclosed herein comprises measuring IL-10 expression in said MSCs in response to stimulation with PHA stimulation T-lymphocytes and/or GABA.

The skilled person appreciates that said assays may be combined to obtain a specific assay combination of interest depending to the desirable properties of the MSC population(s) assayed. The assays may be selected independently of each other.

It is furthermore of importance that the any MSCs to be pooled to obtain the isolated, allogeneic pooled MSC population obtainable by the method as disclosed herein are cells which have a cell morphology of normal cells. MSC cultures are known to contain a subpopulation of small, round cells that are rapidly selfrenewing, usually identified by flow cytometry as low forward scatter and low side scatter. MSCs isolated from donors with greater colony-forming ability are known to have significantly higher proportion of smaller-sized cells. Collectively, data show that donor MSCs classified as having high-growth capacity have an increased capacity for self-renewal, a higher CFU-F efficiency, and a larger proportion of small-sized cells. Cells may be visually inspected during expansion (culture) as well as immediately before or in connection with harvesting and evaluated based on for example the size of cells; size of nuclei; shape of cells; and ratio between cell size and nuclei size. Thus, in one embodiment of the method as disclosed herein, said at least 3 assays comprise at least one morphological assay. In one embodiment, said morphological assay assays morphological features of cells and/or cells nuclei. In one embodiment, said morphological features of cells and/or cells nuclei are one or more features selected from the group consisting of the size of the cell, the size of the nuclei, the shape of the cell and the ratio between cell and nuclei size.

It is important that the an isolated, pooled allogeneic MSC population comprises as many cells as possible which exhibit healthy and desirable morphology, in other words normal morphology. Thus, in one embodiment of the method as disclosed herein, an individual donor derived MSC population is only eligible for pooling if it exhibits at least to 90%, such as at least 91 %, such as at least 92%, such as at least 93%, such as at least 94%, such as at least 95%, such as at least 96%, such as at least 97%, such as at least 98%, such at least 99%, normal cells and/or nuclei. Thus, if said individual donor derived MSC population comprises less than 90% normal cells, said population is not eligible for pooling.

It will be appreciated that said step of assaying each individual donor derived MSC population using at least 3 assays may be performed at any of passages 0 (p0) to p8. For example, said assays may be performed when said individual donor derived MSC populations are in the same passage as when they are pooled in order to ensure that said individual donor derived MSC populations then exhibit the desirable properties at the relevant time point. It is also possible that the assays are performed at an earlier passage than the passage at which they are pooled. It will also be appreciated that different assays may be performed at different passages, provided that a particular assay is performed on each individual donor derived MSC population in the same passage to ensure that the assay results obtained for each individual donor derived MSC population may be compared.

Thus, in one embodiment of the present method, the step of assaying each individual donor derived MSC population using at least 3 assays is performed when the MSC population is in passage 0 (p0) - passage 8 (p8), such as in p1 - p 5, such as in p1 - p4, such as in p2 - p4 or in p1 - p4, such as in p1, p2 and/or p3, such as in p2 and/or p3. In one embodiment, at least one assay, such as at least two assays, such as at least three assays, such as all assays, is/are performed when the cells are in the same passage as when they are pooled. In another embodiment, at least two assays are performed at different passages.

In one embodiment of the present method, said each individual donor derived MSC population is assayed by at least one morphology assay. In one embodiment of the present method, said each individual donor derived MSC population is assayed by at least one assay measuring indoleamine-2,3-dioxygensase (IDO) activity. In one embodiment, said each individual donor derived MSC population is assayed by at least one assay measuring the effect of said MSCs on the proliferation of PBSCs. In one embodiment, said each individual donor derived MSC population is assayed by at least one assay measuring prostaglandin E2 secreted by said MSCs.

In one embodiment of said method, said each individual donor derived MSC population is assayed by at least one morphology assay; an assay measuring IDO activity; and an assay measuring the effect of said MSCs on the proliferation of PBSCs. In one embodiment of said method, said each individual donor derived MSC population is assayed by at least a morphology assay; an assay measuring IDO activity; an assay measuring the effect of said MSCs on the proliferation of PBSCs; and an assay measuring prostaglandin E2 secreted by said MSCs. In one embodiment, there is provided a method as disclosed herein, wherein said each individual donor derived MSC population is further assayed by an assay measuring HLA-G expression in said MSCs. In one embodiment, there is provided a method, wherein said each individual donor derived MSC population is further assayed by at least one assay, such as at least two assays, such as at least three assays, such as at least four assays, measuring the expression of at least one, such as two, such as three, such as all four, factor(s) selected from IL-6, IL-8. GM-CFS and TGFβ. It will be understood that each assay may measure the expression of one of IL-6, IL-8. GM-CFS and TGFβ. In one embodiment, there is provided a method, wherein said each individual donor derived MSC population is further assayed by an assay measuring HLA-G expression in said MSCs and by at least one assay, such as at least two assays, such as at least three assays, such as at least four assays, measuring the expression of at least one, such as two, such as three, such as all four factor(s) selected from IL-6, IL-8. GM-CFS and TGFβ. It will be understood that each assay may measure the expression of one of IL-6, IL-8. GM-CFS and TGFβ.

The present method comprises a step of allocating a total score value to each individual donor derived MSC population. In this step a total score value is allocated to each individual donor derived MSC population based on said at least three individual ranking score values. In the case when a higher ranking score value is indicative of more desirable assay result, a higher total score value is indicative of more desirable population properties. Alternatively, in the case when a lower ranking score value is indicative of more desirable assay result, a lower total score value is indicative of more desirable population properties. The skilled person will appreciate that the ranking score value system and/or the total score value system may be modified without departing from the scope of the present disclosure, provided that said systems allow for a comparison between the individual donor derived MSC populations in terms of desirable properties. In one embodiment of the method disclosed herein, the wherein the individual ranking score value for at least one assay is allocated to said each individual donor derived MSC population based on a comparison of the assay result for said each individual donor derived MSC population to the results for the remaining individual donor derived MSC populations. Thus, individual ranking score values may be allocated based on comparison between the individual donor derived MSC populations analyzed. In one embodiment, wherein the individual ranking score value for at least one assay is allocated to said each individual donor derived MSC population based on absolute assay result obtained for said individual donor derived MSC population. Thus, a desired threshold value for an assay may be chosen. In one embodiment, the assay result is deemed desirable and an individual ranking score value that reflects the obtained desirable assay result is allocated, when said absolute result corresponds to at least a predetermined value or at most a predetermined value.

It will be appreciated that the step of allocating an individual ranking score value to the results from one, two, three or more of said at least 3 assays involves allocating an individual ranking score value, which individual ranking score value is non binary. A not binary score value is a score value which is selected from at at least three levels, in other words at least three different scores. Non limiting examples of non binary score values is 1, 2 and 3; 0, 1 and 2; and 1, 3 and 5. It will be appreciated the non binary ranking score values may be represented by any three numbers X, Y, Z, wherein said X, Y and Z are different numbers. The allocation of non binary score values allows for a higher resolution of ranking the assay results compared to binary score values. Thus, in one embodiment of the method as disclosed herein, allocation an individual ranking score value to each individual donor derived MSC population based on the assay result involves allocating a score value selected from at least three ranking score values, such as at least four ranking score values, such as at least five ranking score values. For example, said individual ranking score value may be selected from 5, 6, 7, 8, 9, 10 or even more possible score values. The skilled person will appreciate that the ranking score values may be numeric or not numeric.

The total score value may be an additive score value obtained by addition of ranking score values for each individual donor derived MSC population. Alternatively, the total score value may be a weighed total score value, obtained by 1) assigning a weight to the ranking score value for each assay and 2) adding the weighed ranking score values for individual donor derived MSC population. In this way it is possible to allocate a relatively higher weight (or importance) to one or several assay result of choice compared to the remaining assay results. The skilled person will appreciate that one or several assay results may be weighed and the weight allocated to each assay result may be chosen independently. Thus, in one embodiment there is provided a method as disclosed herein, wherein said total score value allocated to said each individual donor derived MSC population is an additive total score value obtained by addition of ranking score values for each individual donor derived MSC population. In another embodiment, said total score value allocated to said each individual donor derived MSC population is a weighed total score value obtained by 1) assigning a weight to the ranking score value for each assay and 2) adding the weighed ranking score values for individual donor derived MSC population.

Based on the total score values, a subset of individual donor derived MSC populations with desirable population properties is selected. In this step, it is envisioned that at least 3, such as at least 4, such as at least 5, such as at least 6, such as at least 7, such as at least 8, such as at least 9, such as at least 10 individual donor derived MSC populations are selected. As used herein, the term "subset" refers to all or fewer than all of the assayed individual donor derived MSC populations.

In one embodiment of the method as disclosed herein, the step of selecting a subset of individual donor derived MSC populations with desirable population properties comprises selecting the individual donor derived MSC populations with total score values which correspond to at least a predetermined total score value in the case wherein a higher total score value is indicative of more desirable population properties; or to at most a predetermined total score value in the case wherein a lower total score value is indicative of more desirable population properties. In another embodiment, the step of selecting a subset of individual donor derived MSC populations with desirable population properties comprises selecting a predetermined number of the individual donor derived MSC populations, which populations exhibit a higher total score value relative the remaining individual donor derived MSC populations in the case wherein a higher total score value is indicative of more desirable population; or which populations exhibit a lower total score value relative the remaining individual donor derived MSC populations in the case wherein a lower total score value is indicative of more desirable population properties.

In the next step of the inventive method, the selected individual donor derived MSC populations are pooled to obtain the isolated, pooled allogeneic MSC population. As explained above, it is considered beneficial that the isolated, pooled allogeneic MSC population comprises similar numbers or numbers in the same range of cells derived from each individual donor, such that cells from one donor are not significantly dominating in said pooled population. Thus, in one embodiment of the method as disclosed herein, the number in said the isolated, pooled allogeneic MSC population of cells derived from any one donor does not exceed about 45%, such as does not exceed 40%, such as does not exceed about 35%, of the total cell number in said isolated, pooled allogeneic MSC population and wherein said population comprises MCSs derived from at least 3 donors; such as in which population the number in said isolated, pooled allogeneic MSC population of cells derived from any one donor does not exceed about 40%, such as does not exceed about 35%, such as does not exceed about 30%, of the total cell number in said isolated, pooled allogeneic MSC population and wherein said population comprises MCSs derived from at least 4 donors; such as in which population the number in said isolated, pooled allogeneic MSC population of cells derived from any one donor does not exceed about 35%, such as does not exceed about 30%, such as does not exceed about 25%, of the total cell number in said isolated, pooled allogeneic MSC population and wherein said population comprises MCSs derived from at least 5 donors; such as in which population the number in said isolated, pooled allogeneic MSC population of cells derived from any one donor does not exceed about 30%, such as does not exceed about 25%, such as does not exceed about 20%, of the total cell number in said isolated, pooled allogeneic MSC population and wherein said population comprises MCSs derived from at least 6 donors; such as in which population the number in said isolated, pooled allogeneic MSC population of cells derived from any one donor does not exceed about 25%, such as does not exceed about 22%, such as does not exceed about 20%, of the total cell number in said isolated, pooled allogeneic MSC population and wherein said population comprises MCSs derived from at least 7 donors.; such as in which population the number in said isolated, pooled allogeneic MSC population of cells derived from any one donor does not exceed about 20 %, such as does not exceed about 18 %, such as does not exceed about 16 %, of the total cell number in said isolated, pooled allogeneic MSC population and wherein said population comprises MCSs derived from at least 8 donors; such as in which population the number in said isolated, pooled allogeneic MSC population of cells derived from any one donor does not exceed about 18 %, such as does not exceed about 15 %, such as does not exceed about 13 %, of the total cell number in said isolated, pooled allogeneic MSC population and wherein said population comprises MCSs derived from at least 9 donors; such as in which population the number in said isolated, pooled allogeneic MSC population of cells derived from any one donor does not exceed about 16 %, such as does not exceed about 14 %, such as does not exceed about 12 %, of the total cell number in said isolated, pooled allogeneic MSC population and wherein said population comprises MCSs derived from at least 10 donors. In one particular embodiment of said method, the number of MSC derived from any one individual donor does not exceed about four times, such as about three times, such as about two times the number of the cells derived any other donor.

It will be appreciated that in order to maintain the desired distribution of MSCs derived from individual donors, no further culture of the MCSs is performed after pooling of the selected subset of individual donor derived MSC populations. Without being bound by theory, it is envisioned that the distribution of MSCs derived from individual donors in the pooled population is of importance for obtaining a HLA mismatch expected to ensure no or low HLA immunization in patients administered the isolated, pooled allogeneic MSC population. Therefore, the isolated, pooled allogeneic MSC population as disclosed herein is not further cultured after pooling according to the present method. In one embodiment of the present aspect, there is provided method as disclosed herein, wherein said population is not further cultured after the pooling step.

As mentioned above, only individual donor derived MSC populations which fulfill the desired requirements as assayed by said at least said 3 assays are eligible for pooling in the pooling step. The non-eligible cells are thus discarded. It is possible to compare the assay results obtained for the individual donor derived MSC populations, thus the properties of said cells, to the assay results obtained an earlier obtained isolated, pooled allogeneic MSC population, which earlier population was obtained by the method as disclosed herein. Hence, the earlier population serves as an internal quality control in the present method. Thus, in one embodiment of the present method, the method further comprises the step of discarding an individual donor derived MSC population from the pooling step if the assay results for said individual donor derived MSC population are less desirable than the corresponding assay results for a pooled allogeneic MSC population previously obtained by the same method.

Importantly, the method according to the present disclosure leads to the reduction of variation in the overall assessment of the isolated pooled allogeneic MCS population. To clarify, the variation *within* a batch is reduced compared at a batch comprising MSC pooled form all donors.

To illustrate, the variation in assessment can be calculated by the following formula:

$$\text{Variation} = 1 - \frac{\text{selected (max} - \text{min)}}{\text{all (max- min)}}$$

, wherein the maximum and minimum values are the maximum and minimum assay results obtained.

The overall assessment may be calculated as the delta selection algorithm score for selected donors, in this example 6-3 =3, divided by the delta selection algorithm score for all donors evaluated, in this example 6-1 = 5.

$$\text{Variation} = 1 - \frac{\text{selected (max} - \text{min)}}{\text{all (max- min)}} = 1 - \frac{3}{5} = 0.4$$

Thus, in one embodiment of the method for obtaining the isolated, pooled allogeneic MSC population as disclosed herein, the variation in the overall assessment within a batch is reduced by at least 30%, such as at least 35 %, such as at least 40 % such as at least 45 %, such as at least 50 %, such as at least 60 %, when comparing the assay results for all the individual donor derived MSC populations assayed and the for selected a subset of individual donor derived MSC populations.

As explained in detail for the second aspect of the present disclosure, the method as disclosed herein allows for obtaining batches of isolated, pooled allogeneic mesenchymal stem cell (MSC) population as disclosed herein, which batches show no statistically significant batch-to-batch variability.

In a second aspect of the present disclosure, there is provided an isolated, pooled allogeneic MSC population obtainable by the method as disclosed herein, wherein said population is not further cultured after pooling. Furthermore, in one embodiment, said isolated, pooled allogeneic MSC population exhibits no statistically significant batch-to-batch variability. The present method allows for obtaining an isolated, pooled allogeneic MSC population which exhibits advantageous properties. It is considered advantageous that large batches of MSCs may be obtained due to the step of pooling cells, and additionally this allows for reduction of manufacturing costs. Due to the pooling of individual donor derived MSC populations, it is possible to maintain the cells at low passage numbers as described above, whereby the obtained isolated, pooled allogeneic MSC population exhibits high potency as well as low risk of genetic instability. Hence, large batches of genetically stable cells with high potency can be obtained. Furthermore, the isolated, pooled allogeneic MSC population obtainable by the method as disclosed herein exhibits no statistically significant batch-to-batch variability, due to the method steps employed.

As used herein, the term "batch" refers to an isolated, pooled allogeneic MSC population obtained by the method as disclosed herein.

As used herein, the term "batch-to-batch variability" refers to the difference in properties between an isolated, pooled allogeneic MSC population obtained by the method as disclosed herein and another isolated, pooled allogeneic MSC population obtained by the method as disclosed herein.

Said batch-to-batch variability may be quantified by comparing the results from one or several of said at least three assays which were used for assaying the individual donor derived MSC populations. Alternatively one or several different assays may be employed.

As used herein, the term "no statistically significant batch-to-batch variability" is to be interpreted as the difference between the assay results from one batch and the assay results from a different batch is not statistically significant (for example using a probability value of P>0.05). Said statistical significance may be justified as the coefficient of variance between batches is equal or below the inter and/or intra assay coefficient of variance. The skilled person is familiar suitable statistical calculations.

Thus, in one embodiment, there is provided an isolated, pooled allogeneic MSC population as disclosed herein, which population exhibits no statistically significant batch-to-batch variability. In one embodiment, said no statistically significant batch-to-batch variability is between two consecutively produced batches. In one embodiment, said no statistically significant batch-to-batch variability is between any two batches. In one embodiment, said no statistically significant batch-to-batch variability is between a produced batch and reference batch, wherein said reference batch is an isolated, pooled allogeneic MSC population previously produced by the method as disclosed herein.

In one embodiment, said no statistically significant batch-to-batch variability is associated with the probability value (P) of > 0.05, such as for example P of > 0.04, such as for example P of > 0.03, such as for example P of > 0.02, such as for example P of > 0.01, such as for example P of > 0.005, such as for example P of > 0.001.

In one embodiment, said batch-to-batch variability is quantified based on the assay results from at least one assay selected from the group consisting of the IDO assay as described herein, the PGE2 assay as described herein and the proliferation assay as described herein; such as at least two assays selected from the group consisting of of the IDO assay as described herein, the PGE2 assay as described herein and the proliferation assay as described herein; such as all three of the IDO assay as described herein, the PGE2 assay as described herein and the proliferation assay as described herein. Optionally, the batch-to-batch variability may be quantified based one or more additional assays.

The isolated, pooled allogeneic MSC population as disclosed herein exhibits desired functional and morphological properties, high potency, no statistically significant batch-to-batch variability and is also obtainable in large batches. This allows for predictability and low variability when said population is used as a medicament. Thus, the present isolated, pooled allogeneic MSC population may be used in a standardized medical treatment procedure. It is envisioned that there are both logistic and dosing advantages for the isolated, pooled allogeneic MSC population obtainable by the method as disclosed herein, when said population is used as medicament, in particular in regards to the formulation and dose regimen. The logistic chain is to keep the isolated, pooled allogeneic MSC population in cryogenic storage, hence ensuring that the properties of the isolated, pooled allogeneic MSC population are maintained and allowing that the patient receives a predefined cell number as a medicament, in contrast to "giving the patient the number of cells we managed to expand" according to the prior art, is considered important. Thus, the isolated, pooled allogeneic MSC population as disclosed herein is suitable as on "off the shelf" standardized medical product, which offers predictability in terms of therapeutic effect and safety.

It is envisioned that said isolated, pooled allogeneic MSC population as disclosed herein will be useful in the treatment and/or prevention of diseases or conditions selected from the group consisting of inflammatory diseases or conditions, autoimmune disease and transplantation rejection.

Thus, in a third aspect of the present disclosure, there is provided isolated, pooled allogeneic MSC population as disclosed herein, for use as a medicament.

Without being bound by theory, the isolated, pooled allogeneic MSC population is envisioned to be able modulate responses by innate and adaptive immune cells, retain dendritic cells in an immature state, inhibit dendritic cell differentiation and suppressing their proinflammatory cytokine production. Therefore, the present inventive isolated, pooled allogeneic MSC population is this envisioned to be useful for the treatment and/or prevention of inflammatory and autoimmune diseases or conditions, as well as transplant rejections.

Thus, in a related fourth aspect of the present disclosure, there is provided an isolated, pooled allogeneic MSC population as disclosed herein, for use in the treatment and/or prevention of a disease or condition selected from the group consisting of inflammatory diseases or conditions, autoimmune disease and transplantation rejection.

In one embodiment, said use comprises administration of said isolated, pooled allogeneic MSC population as an infusion to patient in need thereof. In one embodiment, said infusion is administered intravenously, intraperitonealy or intralymphatically, intravenously, intrathecal, intracerebral and or through the ommaya reservoir, intraarterially or subcutaneously. In one embodiment, said infusion is administered intravenously, intraperitonealy or intralymphatically, such as intravenously.

The modulatory nature of MSC and the low expression of HLA-DR are two reasons for assuming that allogeneic transplantation of MSC may be accepted without HLA matching between donor and recipient. It is envisioned that, said infusion may be performed repeatedly or only once, depending on the therapeutic needs of the patient. Without being bound by theory, it is envisioned that said administration, by one infusion or repeated infusions, will not lead to clinically relevant levels of anti-HLA antibodies in the treated patients. Hence, said patients will be eligible for several infusion treatments as described herein or for receiving a transplant if in need thereof. Thus, in one embodiment, said infusion is performed only once. In another embodiment, said infusion is performed repeatedly. For example, said infusion may be performed two times, three times, four times or more.

Thus, in one embodiment, said administration induces no or low anti-HLA antibody titers in said patient. As used herein, the term "low or no anti-HLA antibody titers" refers to titers which are considered clinically irrelevant. Antibody analysis by solid phase multiplex technologies have allowed for a more precise definition of the breadth and strength of HLA antibodies. By correlating these results with those obtained by an actual cell-based crossmatch, and eventual graft outcome, clinically relevant antibodies can be defined in a center-specific manner (Zachary et al. Using real data for a virtual crossmatch. Hum Immunol 2009; 70: 574-579). The skilled person will appreciate that clinically irrelevant anti-HLA antibody titers may be defined by LABScreen single antigen beads test with higher mean fluorescence intensity (MFI) for donor specific antibodies than 1000, DSA MFI >1000.

As mentioned above, it is envisioned that the isolated, pooled allogeneic MSC population as disclosed herein will be useful in the treatment and/or prevention of a disease or condition selected from the group consisting of inflammatory diseases or conditions, autoimmune disease and transplantation rejection.

Non-limiting examples of inflammatory diseases or conditions include acne vulgaris, asthma, autoimmune diseases, autoinflammatory diseases, celiac disease, chronic prostatitis, colitis, diverticulitis, glomerulonephritis, hidradenitis suppurativa, inflammatory bowel diseases, interstitial cystitis, pelvic inflammatory disease, reperfusion injury, rheumatic fever, rheumatoid arthritis, sarcoidosis, transplant rejection, type II diabetes and vasculitis. Non-limiting examples of autoimmune diseases or conditions include Alzheimer's disease, amyo lateral sclerosis, autism, systemic autoimmune diseases, such as SLE, Sjögren's syndrome, sarcoidosis, scleroderma, rheumatoid arthritis, cryoglobulinemic vasculitis, and dermatomyositis; and local autoimmune diseases/syndromes or conditions which affect a specific organ or tissue such as endocrine autoimmune diseases or conditions for example diabetes mellitus type 1, Latent Autoimmune Diabetes in the Adult (LADA), Hashimoto's thyroiditis and Addison's disease; gastrointestinal autoimmune diseases or conditions for example Coeliac disease, Crohn's disease and pernicious anaemia; dermatologic autoimmune diseases or conditions for example pemphigus vulgaris and vitiligo; haematologic autoimmune diseases or conditions for example autoimmune haemolytic anaemia and idiopathic thrombocytopenic purpura; and neurological autoimmune diseases or conditions for example multiple sclerosis, myasthenia gravis and encephalitis. Non-limiting examples of transplant rejection diseases or conditions wherein administration of the present isolated, pooled allogeneic MSC population may be useful include Graft versus Host disease, organ transplant, hematopoietic stem cell transplantation, cell transplantation and islet transplant. The skilled person will appreciate that administration of the present isolated, pooled allogeneic MSC population may be useful as a treatment and/or prevention approach in connection with any of the above mentioned diseases and conditions.

In particular embodiments, there is provided an isolated, pooled allogeneic MSC population for use as disclosed herein, wherein said inflammatory disease or condition is selected from the group consisting of autoimmune diseases.

In particular embodiments, there is provided an isolated, pooled allogeneic MSC population for use as disclosed herein, wherein said disease or condition is transplant rejection, such as rejection of any kind of transplant including cell, tissue, organ or implant. In one embodiment, said transplant rejection may be of an organ transplant rejection or an islet transplant rejection. In one embodiment, said organ is selected from the group consisting of kidney, liver, lung and heart. In one embodiment, said transplant is kidney transplant. In one embodiment, said transplant rejection is an islet transplant rejection.

In particular embodiments, there is provided an isolated, pooled allogeneic MSC population for use as disclosed herein, wherein autoimmune disease is selected from the group consisting of amylotrophic lateral sclerosis (ALS), multiple sclerosis, diabetes, Crohn's disease, ulcerative colitis, inflammatory bowel disease and arthritis. In one embodiment, said autoimmune disease is ALS or inflammatory bowel disease. In one particular embodiment, said autoimmune disease is type 1 diabetes or LADA. It is envisioned that the present isolated, pooled allogeneic MSC population may be a particularly useful for prevention and treatment of LADA patients, recently diagnosed type 1 diabetes cases, and in longstanding type 1 diabetes patients with at least some remaining endogenous insulin production. Without being bound by theory, the immunosuppressive properties of the isolated, pooled allogeneic MSC population are envisioned to slow down or hinder the autoimmune destruction of the insulin-producing beta cells in the pancreas. It may be beneficial to administer said isolated, pooled allogeneic MSC population to patients who have at least some endogenous insulin production.

Thus, in one embodiment, there is provided an isolated, pooled allogeneic MSC population according for use as described herein, wherein said type 1 diabetes or LADA is recently diagnosed type 1 diabetes or LADA, such as type 1 diabetes or LADA diagnosed no more than 3 years prior, such as no more than 2 years prior, such as no more than 1 year prior to the administration said infusion, such as no more than 6 months prior to the administration said infusion, such as no more than 5, 4, 3, 2 or 1 month prior to the administration said infusion. Patients with recently diagnosed type 1 diabetes are expected to exhibit at least some endogenous insulin production and thus express C-peptide, which is a short 31-amino-acid polypeptide that connects insulin's A-chain to its B-chain in the proinsulin molecule.

In one embodiment, there is provided an isolated, pooled allogeneic MSC population according for use as described herein, wherein said patient recently started to exhibit clinical symptoms of type 1 diabetes or LADA, such as started to exhibit clinical symptoms of type 1 diabetes or LADA no more than 3 years prior, such as no more than 2 years prior, such as no more than 1 year prior to the administration said infusion, such as no more than 6 months prior to the administration said infusion, such as no more than 5, 4, 3, 2 or 1 month prior to the administration said infusion.

In one embodiment there is provided an isolated, pooled allogeneic MSC population according for use as disclosed herein, wherein said patient expresses C-peptide. In one embodiment, said patient exhibits a fasting plasma C-peptide concentration of $\geq 0.01$ nmol/L, such as $\geq 0.04$ nmol/L, such as $\geq 0.08$ nmol/L, such as $\geq 0.12$ nmol/L.

In one embodiment, said treatment and/or prevention results in a lower decrease rate of plasma C-peptide concentration in patients receiving the treatment compared to untreated patients, for example a lower decrease rate of stimulated plasma C-peptide concentration as measured by a Mixed Meal Tolerance Test (MMTT) in patients receiving the treatment compared to untreated patients. In one embodiment, said decrease rate of plasma C-peptide concentration in patients receiving the infusion is at least 10% lower, such as at least 20%, such as at least 30%, such as at least 40%, such as at least 50% lower than in untreated patients. In another embodiment, said treatment and/or prevention leads to an increase in fasting plasma C-peptide concentration in the patient compared to the fasting plasma C-peptide concentration before the administration. Said increase in fasting or stimulated plasma C-peptide concentration may for example be measured at 3 months, 6 months or 1 year after treatment. In one embodiment, while maintaining HbA1c levels below 7, said treatment and/or prevention leads to reduction in insulin requirement per kg body weight in said patient as compared to the insulin requirement per kg body weight before the administration. In one embodiment, said treatment and/or prevention leads to reduction in insulin requirement per kg body weight in said patient as compared to the insulin requirement per kg body weight before the administration. For example, said reduction in insulin requirement per kg body weight may be measured 3 months, 6 months or 1 year after treatment. In one embodiment, said treatment and/or prevention leads to leads to insulin independence in said patient (according to the ADA criteria), which may for example be measured 3 months, 6 months or 1 year after treatment. In one embodiment, said treatment and/or prevention leads to reduction daily insulin needs in said patient compared to daily insulin needs before the administration. For example, said reduction may lead to an insulin need of <0.50U/kg body weight per day, such as <0.40U/kg body weight per day, such as <0.30U/kg body weight per day, <0.25U/kg body weight per day in treated patients, for example as measured 3 months, 6 months or 1 year after treatment. The isolated, pooled allogeneic MSC population as described herein is to be administered in a therapeutically effective dose. In one embodiment, there is provided an isolated, pooled allogeneic MSC population for use as discloses herein, wherein said use comprises administration to said patient a dose of at least about $3 \times 10^6$ cells, such as at least about $5 \times 10^6$ cells, such as at least about $10 \times 10^6$ cells, such as at least about $15 \times 10^6$ cells, such as at least about $20 \times 10^6$ cells at least about $25 \times 10^6$ cells, such as approximately at least about $50 \times 10^6$ cells, such as approximately at least about $75 \times 10^6$ cells, such as approximately at least about $100 \times 10^6$ cells, such as approximately at least about $125 \times 10^6$ cells, such as approximately at least about $150 \times 10^6$ cells, such as approximately at least about $175 \times 10^6$ cells, such as approximately at least about $200 \times 10^6$ cells. In one embodiment, said use comprises administration to said patient a dose of at least about $0,1 \times 10^6$ cells/kg bodyweight, such as at least about $0.5 \times 10^6$ cells/kg bodyweight, such as at least about $1 \times 10^6$ cells/kg bodyweight, such as at least about $5 \times 10^6$ cells/kg bodyweight, such as at least about $10 \times 10^6$ cells/kg bodyweight. In one embodiment, said use said use comprises administering to said patient a dose approximately from about $0.1 \times 10^6$ cells/kg bodyweight to about $10 \times 10^6$ cells/kg bodyweight, such as from about $0.25 \times 10^6$ cells/kg bodyweight to about $5 \times 10^6$ cells/kg bodyweight, such as from about $0.25 \times 10^6$ cells/kg bodyweight to about $4 \times 10^6$ cells/kg bodyweight, such as from about $0.5 \times 10^6$ cells/kg bodyweight to about $4 \times 10^6$ cells/kg bodyweight, such as from about $1 \times 10^6$ cells/kg bodyweight to about $4 \times 10^6$ cells/kg bodyweight, such as for example from about $1 \times 10^6$ cells/kg bodyweight to about $3 \times 10^6$ cells/kg bodyweight, such as from about $2 \times 10^6$ cells/kg bodyweight to about $3 \times 10^6$ cells/kg bodyweight or from about $1 \times 10^6$ cells/kg bodyweight to about $2 \times 10^6$ cells/kg bodyweight.

It is envisioned that said isolated, pooled allogeneic MSC population will be useful as a pharmaceutical composition.

Thus, in a related fifth aspect of the present disclosure, there is provided a pharmaceutical composition comprising an isolated, pooled allogeneic MSC population as disclosed herein and at least one pharmaceutically acceptable excipient or carrier. Said pharmaceutical composition may be useful a medicament, for example for treatment of a disease or condition selected from the group consisting of inflammatory diseases or conditions, autoimmune disease and transplantation rejection. It will be appreciated that the pharmaceutical composition may be useful in the treatment and/or prevention of any one of the diseases or conditions listed in connection with the fourth aspect of the present disclosure, which diseases or conditions will for the sake of brevity not be repeated here. In one embodiment, said pharmaceutical composition comprises approximately at least about $3 \times 10^6$ cells, such as at least about $5 \times 10^6$ cells, such as at least about $10 \times 10^6$ cells, such as at least about $15 \times 10^6$ cells, such as at least about $20 \times 10^6$ cells at least about $25 \times 10^6$ cells, such as approximately at least about $50 \times 10^6$ cells, such as approximately at least about $75 \times 10^6$ cells, such as approximately at least about $100 \times 10^6$ cells, such as approximately at least about $125 \times 10^6$ cells, such as approximately at least about $150 \times 10^6$ cells, such as approximately at least about $175 \times 10^6$ cells, such as approximately at least about $200 \times 10^6$ cells. Thus, one dosage of said composition comprises the above mentioned number of cells.

In one embodiment, said pharmaceutical composition is formulated for infusion, such as for intravenous infusion, intraperitoneal infusion, intralymphatical infusion, intravenous infusion, intrathecal infusion, intracerebral infusion, intraarterial infusion, subcutaneous infusion or infusion through the ommaya reservoir. In one embodiment, said pharmaceutical composition is formulated intravenous, intraperitoneal or intralymphatic infusion.

In a related sixth aspect, there is provided a method treatment and/or prevention of a disease or condition selected from the group consisting of inflammatory diseases or conditions, autoimmune disease and transplantation rejection, comprising administering a therapeutically effective dose of an isolated, pooled allogeneic MSC population as disclosed herein or a pharmaceutical composition as disclosed herein to a patient in need thereof. The skilled person will appreciate that any embodiments mentioned in connection with the fourth aspect of the present disclosure are equally applicable to the inventive method of treatment and/or prevention. For the sake of brevity, said embodiments will not be repeated here or will only be mentioned briefly. In one embodiment of said method, the administration of said MSC population is by infusion, such as intravenous infusion, intraperitoneal infusion, intralymphatical infusion, intravenous infusion, intrathecal infusion, intracerebral infusion, intraarterial infusion, subcutaneous infusion or infusion through the ommaya reservoir. In one embodiment of said method, the administration is by intravenous, intraperitoneal or intralymphatic infusion.

In one embodiment, said infusion is performed repeatedly. In another embodiment, said infusion is performed one time only. In one embodiment of the method for treatment and/or prevention as disclosed herein, said administration induces no or low anti-HLA antibody titers in the patient. In one embodiment, said disease or condition is transplant rejection, such as rejection of any kind of transplant including cell, tissue, organ or implant. In one embodiment, said transplant rejection may be of an organ transplant rejection or an islet transplant rejection, In one embodiment, said organ is selected from the group consisting of kidney, liver, lung and heart. In one embodiment, said transplant is kidney transplant. In one embodiment, said transplant rejection is an islet transplant rejection. In one embodiment, said inflammatory disease or condition is selected from the group consisting of autoimmune diseases. In another embodiment, said autoimmune disease is selected from the group consisting of amyotrophic lateral sclerosis, multiple sclerosis, diabetes, Crohn's disease, ulcerative colitis, inflammatory bowel disease and arthritis, for example such as type 1 diabetes or LADA. In one embodiment, said autoimmune disease is ALS or inflammatory bowel disease. In one embodiment, said type 1 diabetes is recently diagnosed type 1 diabetes or LADA, such as type 1 diabetes or LADA diagnosed no more than 3 years prior, such as no more than 2 years prior, such as no more than 1 year prior to the to the administration, such as no more than 6 months prior to the administration said infusion, such as no more than 5, 4, 3, 2 or 1 month prior to the administration, of said infusion. In one embodiment, said patient expresses C-peptide. In a particular embodiment, said patient exhibits a fasting plasma C-peptide concentration of about $\geq 0.01$ nmol/L, such as about $\geq 0.04$ nmol/L, such as about $\geq 0.08$ nmol/L, such as about $\geq 0.12$ nmol/L.

In one embodiment of the method as disclosed herein, said patient recently started to exhibit clinical symptoms of type 1 diabetes or LADA, such as started to exhibit clinical symptoms of type 1 diabetes or LADA no more than 3 years prior, such as no more than 2 years prior, such as no more than 1 year prior to the administration said infusion, such as no more than 6 months prior to the administration said infusion, such as no more than 5, 4, 3, 2 or 1 month prior to the administration said infusion.

In one embodiment, said treatment and/or prevention results in a lower decrease rate of plasma C-peptide concentration in patients receiving the infusion compared to untreated patients. In one embodiment, said decrease rate of plasma C-peptide concentration in patients receiving the infusion is at least about 10% lower, such as at least about 20%, such as at least about 30%, such as at least about 40%, such as at least 50% lower than in untreated patients. In another embodiment, said treatment and/or prevention leads to an increase in fasting plasma C-peptide concentration in the patient compared to the fasting plasma C-peptide concentration before said administration. For example, said increase in fasting plasma C-peptide concentration may be measured about 3 months, about 6 months or about 1 year after treatment. In one embodiment, said treatment and/or prevention leads to reduction in insulin requirement per kg body weight in said patient as compared to the insulin requirement per kg body weight before the administration. For example, said reduction in insulin requirement per kg body weight may be measured about 3 months, about 6 months or about 1 year after treatment. In one embodiment, said treatment and/or prevention leads to leads to insulin independence in said patient (according to the ADA criteria), which may for example be measured 3 months, 6 months or 1 year after treatment. In one embodiment, said treatment and/or prevention leads to reduction daily insulin needs in said patient compared to daily insulin needs before the administration. For example, said reduction may lead to an insulin need of about <0.50U/kg body weight per day, such as about <0.40U/kg body weight per day, such as about <0.30U/kg body weight per day, about <0.25U/kg body weight per day in treated patients, for example as measured about 3 months, about 6 months or about 1 year after treatment.

In one embodiment, said method comprises administering to said patient a dose of at least about $3 \times 10^6$ cells, such as at least about $5 \times 10^6$ cells, such as at least about $10 \times 10^6$ cells, such as at least about $15 \times 10^6$ cells, such as at least about $20 \times 10^6$ cells at least about $25 \times 10^6$ cells, such as approximately at least about $50 \times 10^6$ cells, such as approximately at least about $75 \times 10^6$ cells, such as approximately at least about $100 \times 10^6$ cells, such as approximately at least about $125 \times 10^6$ cells, such as approximately at least about $150 \times 10^6$ cells, such as approximately at least about $175 \times 10^6$ cells, such as approximately at least about $200 \times 10^6$ cells. In one embodiment, said method comprises administering to said patient a dose of at least about $0.1 \times 10^6$ cells/kg bodyweight, such as at least about $0.5 \times 10^6$ cells/kg bodyweight, such as at least about $1 \times 10^6$ cells/kg bodyweight, such as at least about $5 \times 10^6$ cells/kg bodyweight, such as at least about $10 \times 10^6$ cells/kg bodyweight. In one embodiment, said method comprises administering to said patient a dose approximately from about $0.1 \times 10^6$ cells/kg bodyweight to about $10 \times 10^6$ cells/kg bodyweight, such as from about $0.25 \times 10^6$ cells/kg bodyweight to about $5 \times 10^6$ cells/kg bodyweight, such as from about $0.25 \times 10^6$ cells/kg bodyweight to about $4 \times 10^6$ cells/kg bodyweight, such as from about $0.5 \times 10^6$ cells/kg bodyweight to about $4 \times 10^6$ cells/kg bodyweight, such as from about $1 \times 10^6$ cells/kg bodyweight to about $4 \times 10^6$ cells/kg bodyweight, such as for example from about $1 \times 10^6$ cells/kg bodyweight to about $3 \times 10^6$ cells/kg bodyweight, such as from about $2 \times 10^6$ cells/kg bodyweight to about $3 \times 10^6$ cells/kg bodyweight or from about $1 \times 10^6$ cells/kg bodyweight to about $2 \times 10^6$ cells/kg bodyweight.

In yet another aspect of the present disclosure, there is provided a use of the isolated, pooled allogeneic MSC population as disclosed herein, in the manufacture of a medicament for the treatment of a disease or conditions selected from the group consisting of inflammatory diseases or conditions, autoimmune disease and transplantation rejection.

In another aspect of the present disclosure, there is provided a method for evaluating of potency of a MSC population, comprising the step of:

culturing or providing a MSCs population;

assaying said MSC population using at least 3 assays to obtain said at least 3 assay results;

for each assay allocating a score value to said MSC population based on the assay result, wherein a higher score value is indicative of more desirable assay result; or wherein a lower score value is indicative of more desirable assay result;

allocating a total score value to said MSC population based on the score values allocated to each assay, wherein in the case of a higher score value being indicative of more desirable assay result, a higher total score value is indicative of more desirable population properties; or wherein in the case of a lower score value being indicative of more desirable assay result, a lower total score value is indicative of more desirable population properties;

qualifying the MSC population as potent if said total score value is above a predetermined threshold value in the case of a higher score value being indicative of more desirable assay result, or qualifying the MSC population as potent if said total score value is below a predetermined threshold value in the case of a lower score value being indicative of more desirable assay result. In one embodiment of said method, the method employs at least 3 assays as defined herein.

The isolated, pooled allogeneic MSC population may furthermore be useful for culturing cells to be used in *ex vivo* therapy, for example the MSC population may be used as feeder cells or to providing factor or signals of interest. Thus, in yet another aspect of the present disclosure the use of the isolated, pooled allogeneic MSC population as disclosed herein for co-culture of immune cells is provided. For example said MSC population may be used as feeder cells in culture for *ex vivo* expansion and/or stimulation of immune cells, for example but not limited to dendritic cells, natural killer cells, lymphocytes (such as B-cells or T-cells), monocytes and mast cells. Said MSC population may be used as exosome producing cells and/or paracrine factor producing cells and/or for cell to cell stimulation between MSC and immune cells in culture. In one embodiment, there is provided the use of the isolated, pooled allogeneic MSC population as disclosed herein as feeder cells for co-culture of immune cells. In one embodiment, there is provided the use of the isolated, pooled allogeneic MSC population as disclosed herein for stimulation of immune cells co-cultured with said population. It is envisioned that said immune cells which have been co-cultured with the isolated, pooled allogeneic MSC population as disclosed herein will be useful in therapy.

While the invention has been described with reference to various exemplary aspects and embodiments, it will be understood by those skilled in the art that various changes may be made and equivalents may be substituted for elements thereof without departing from the scope of the invention. In addition, many modifications may be made to adapt a particular situation, MSC population or compositions to the teachings of the invention without departing from the essential scope thereof. Therefore, it is intended that the invention not be limited to any particular embodiment contemplated, but that the invention will include all embodiments falling within the scope of the appended claims. The invention will be further illustrated by the following non-limiting Examples.

## BRIEF DESCRIPTION OF THE FIGURES

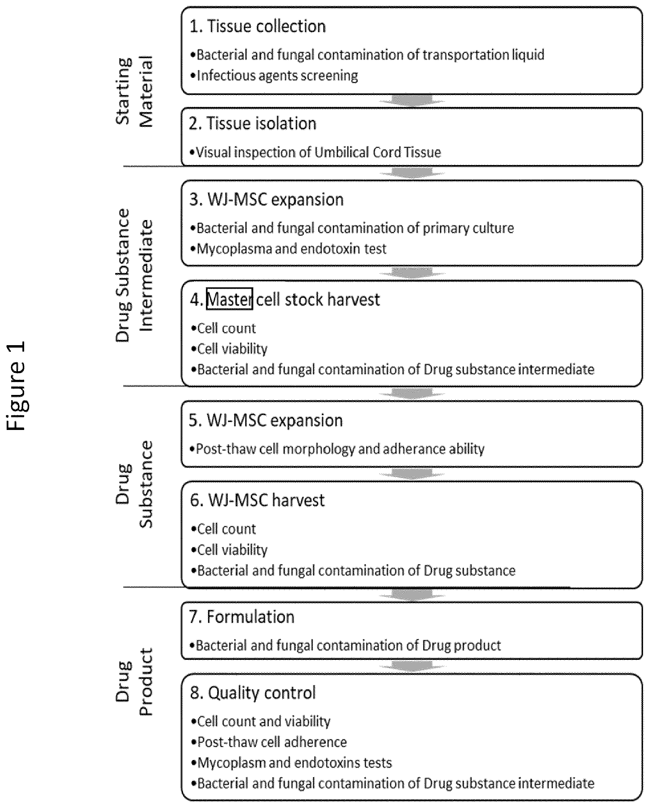

Figure 1 is a flowchart illustrating the manufacturing process of isolated, pooled allogeneic MSC population according to the present disclosure.

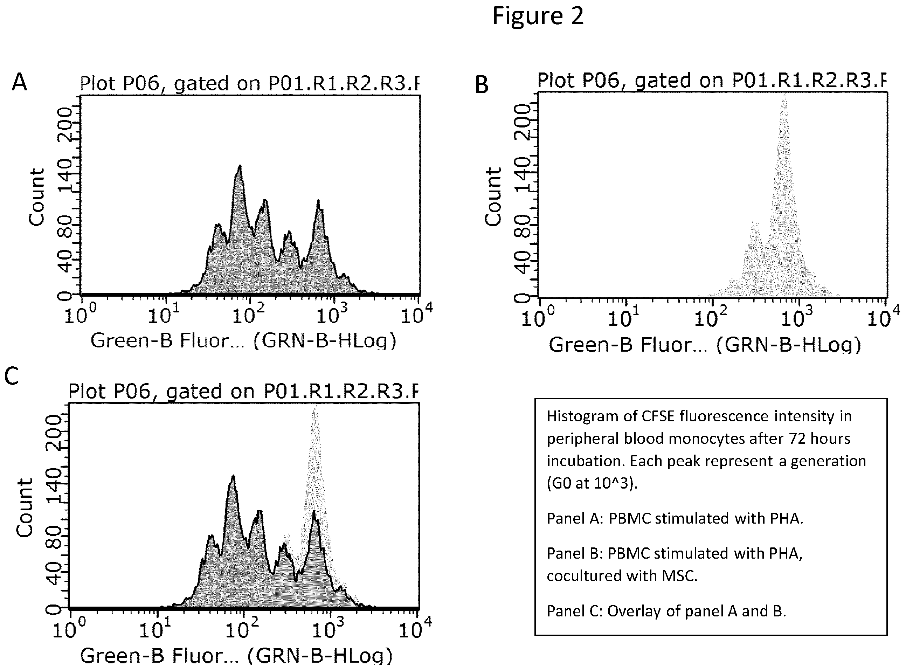

Figure 2 shows result from the proliferation assay described in Example 2. Histogram of CFSE fluorescence intensity in peripheral blood monocytes after 72 hours incubation. Each peak represents a generation (G0 at 10^3). Panel A: PBMC stimulated with PHA. Panel B: PBMC stimulated with PHA, cocultured with MSC. Panel C: Overlay of panel A and B.

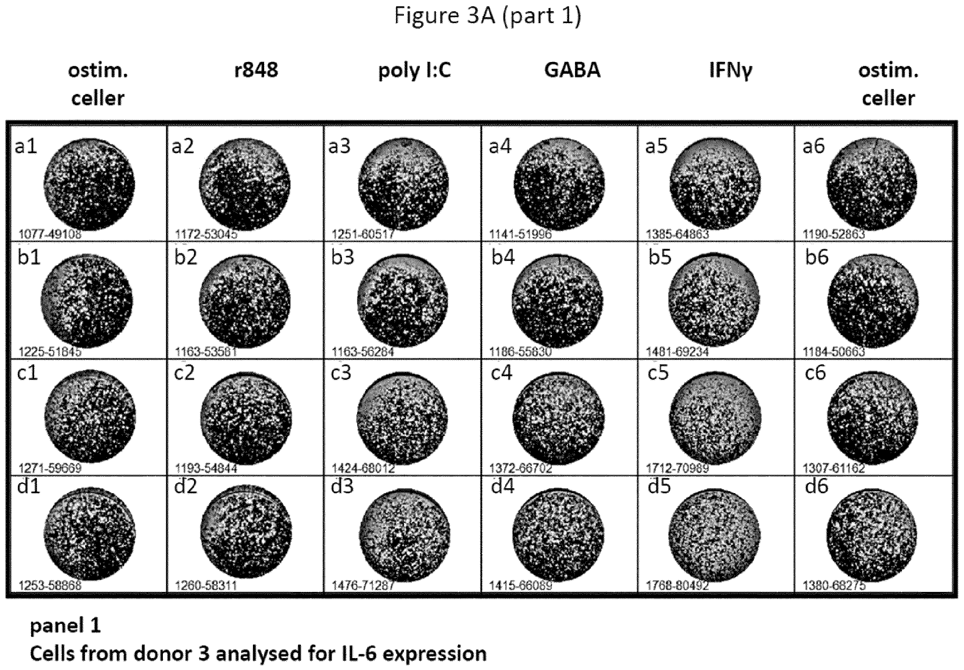

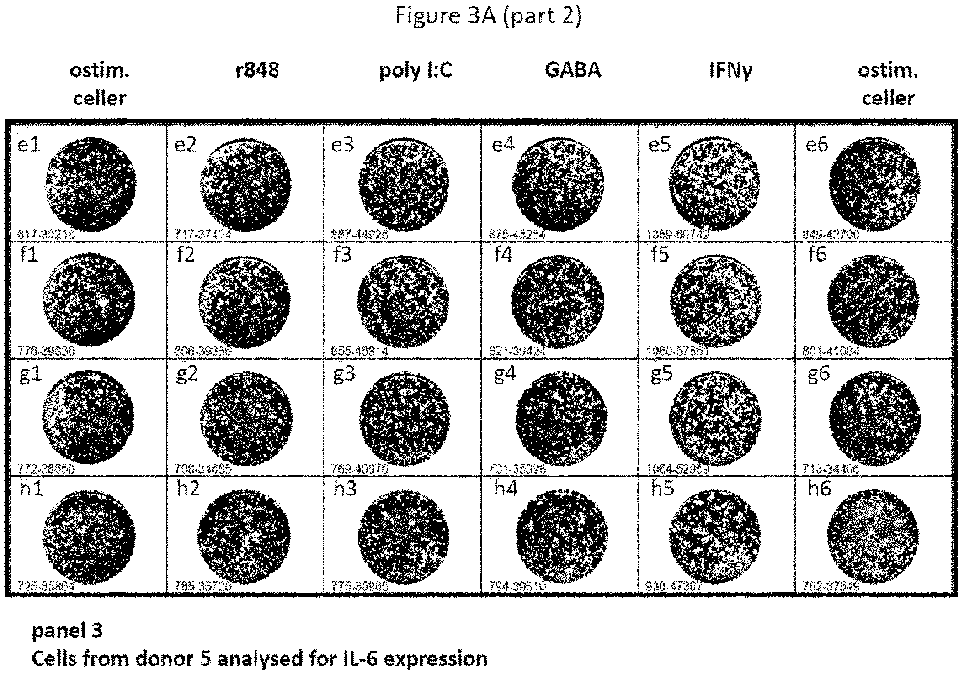

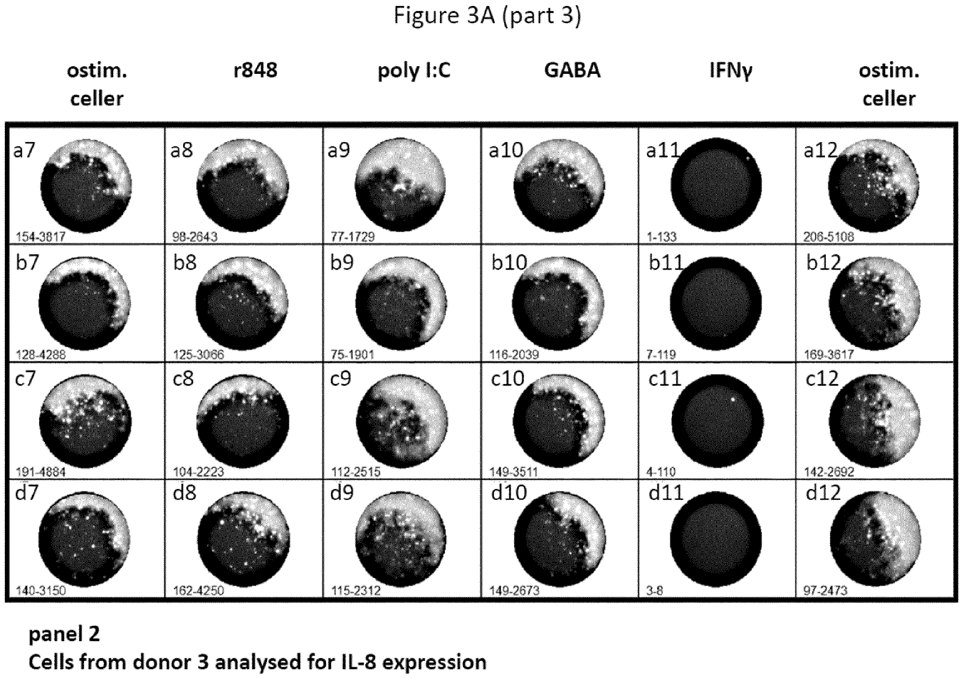

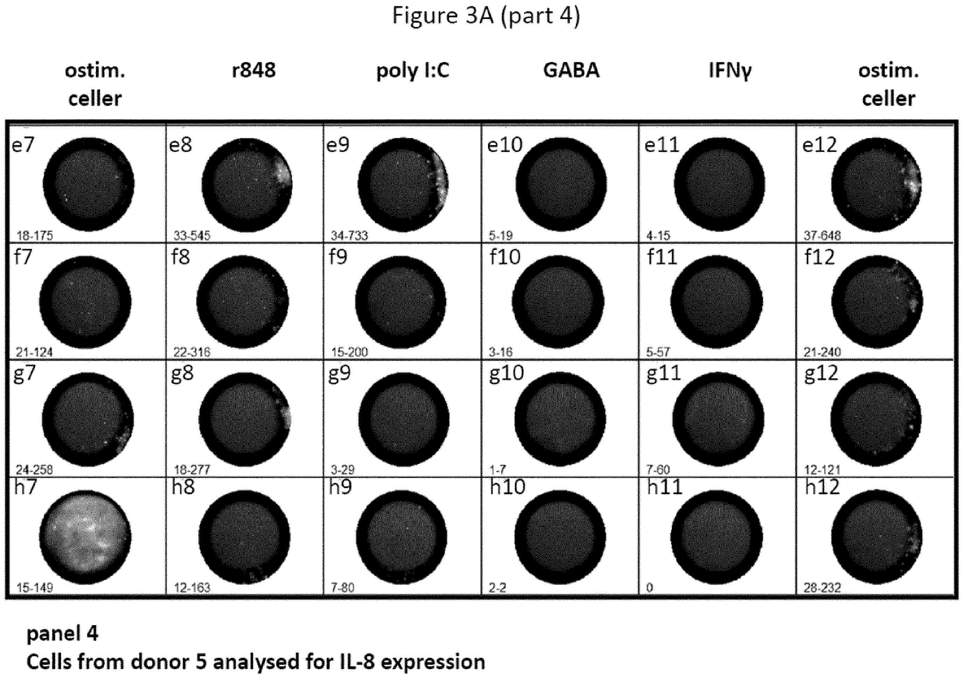

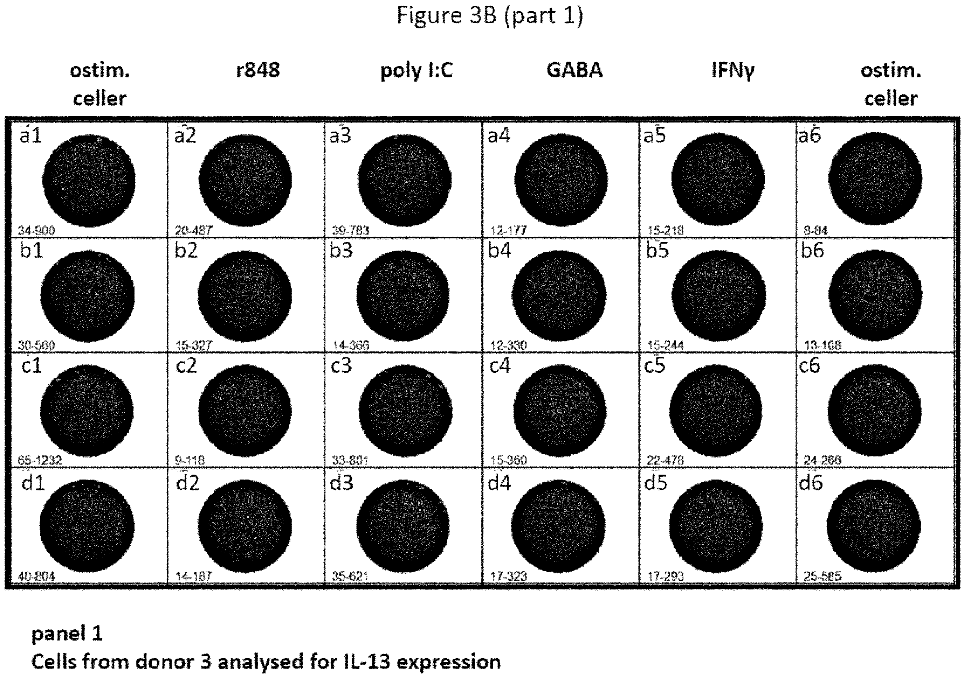

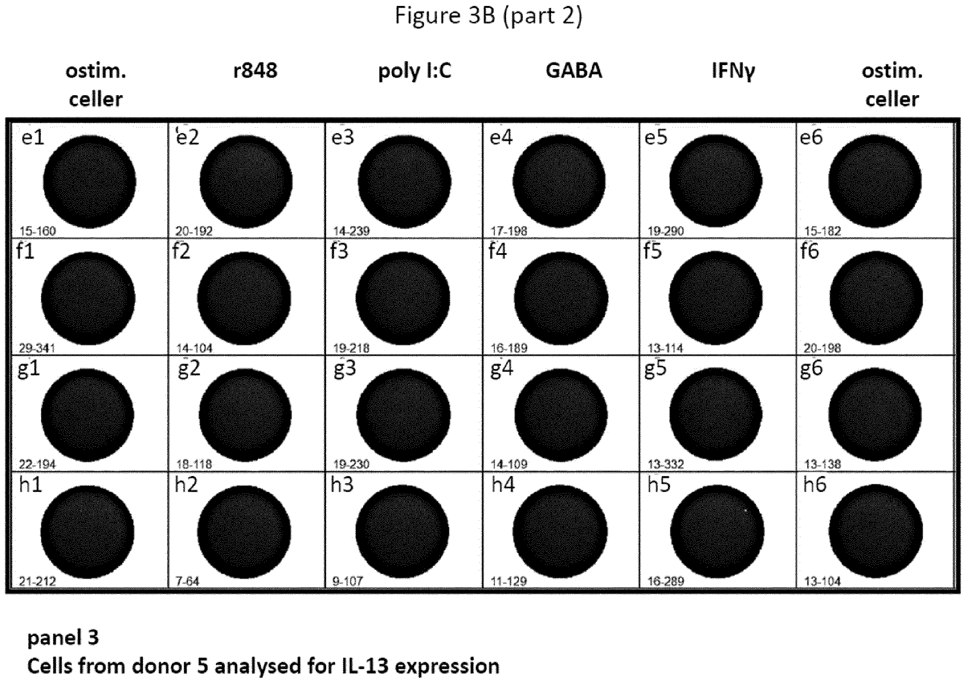

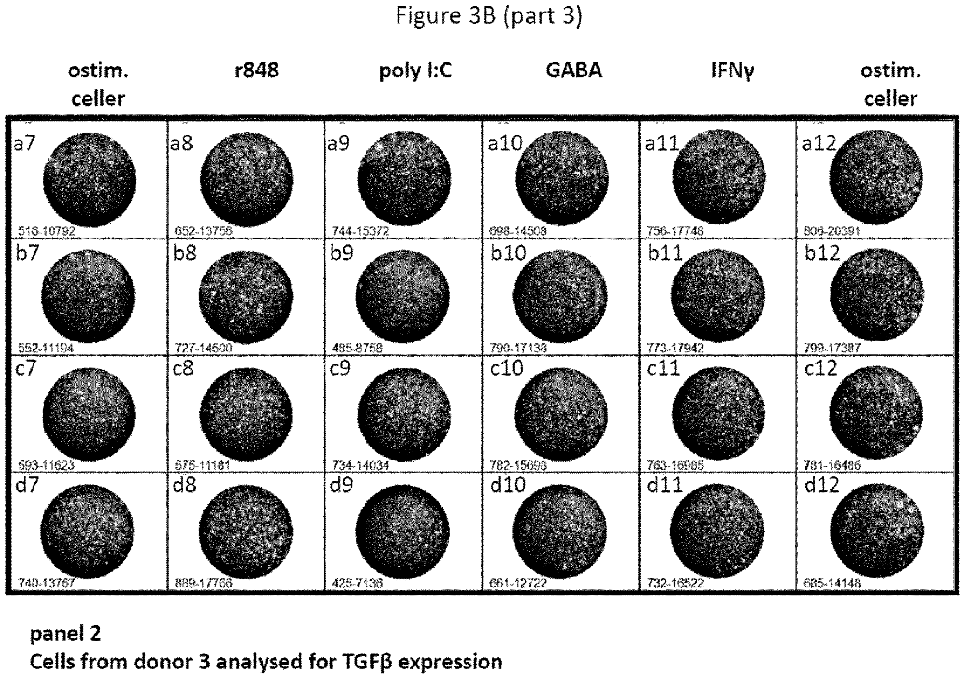

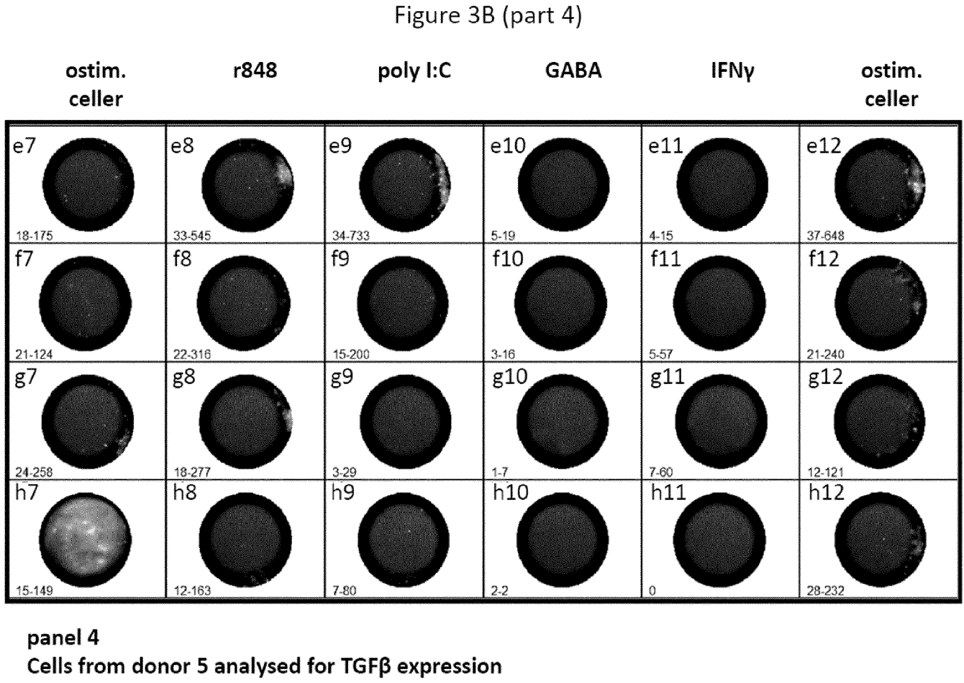

Figure 3A and 3B show results from Fluorospot-assay according to Example 4. Cells from individual donors have been seeded in 96 well Fluorospot assay plates. The cells are unstimulated or stimulated with; r484, poly I:C, GABA, IFNy as indicated. Figure 3A: 1st and 2nd quadrant contain cells from donor no 3 analyzed for expression of IL-6 and IL-8, respectively. 3rd and 4th contain cells from donor no 5 analyzed for expression of IL-6 and IL-8, respectively. Figure 3B: 1st and 2nd quadrant contain cells from donor no 3 analyzed for expression of IL-13 and TGFβ, respectively. 3rd and 4th quadrant contain cells from donor no 5 analyzed for expression of IL-13 and TGFβ, respectively.

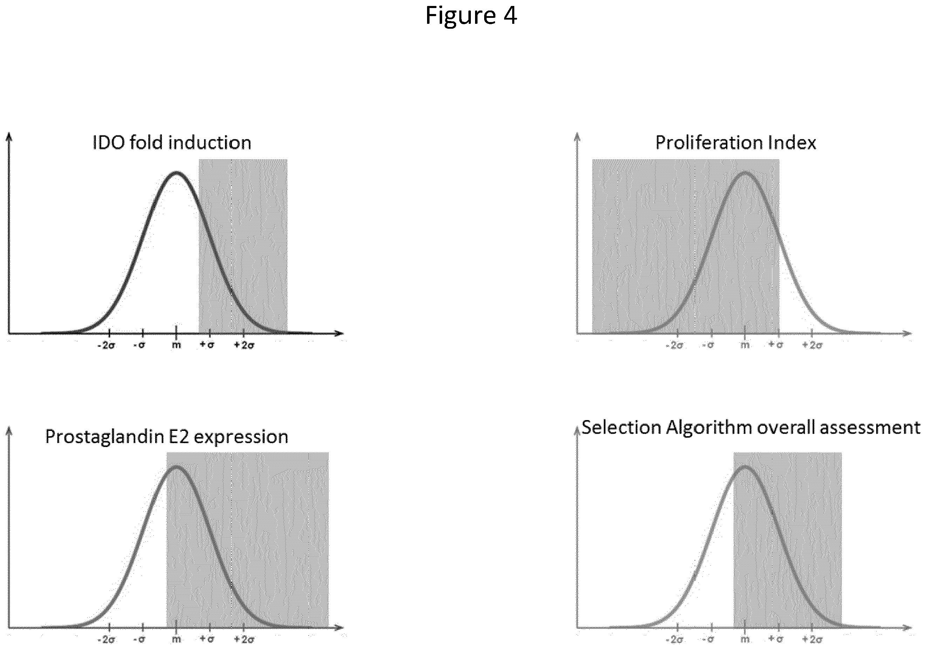

Figure 4 is an illustration of the variation analysis of IDO fold induction, proliferation index and PGE2 assay results of the selected individual donor derived MSC populations and the variation in the overall assessment of the scoring of all analyzed cells and the scoring of the selected cells according to the selection algorithm disclosed herein. Standard distribution is shown and grey boxes indicate the selected donors. As illustrated in the panels, the variation of analysis result for a specific assay is reduced by selecting only donors with the most desired properties. The variation reduction ranged from 53 % for IDO fold induction to 14 % for proliferation index. The reduction of donor variation is reflected in the overall assessment, i.e. in the selection algorithm outcome were the variation is decreased by 40 % in this example.

## EXAMPLES

The present non-limiting Examples describe the generation of the inventive pooled allogeneic MSC composition of *in vitro* expanded mesenchymal stromal cells, including characterization of cells, selection of appropriate donor derived populations of cells and pooling of said donor derived populations of cells to obtain said composition. Examples 1-6 describe the process of obtaining the inventive pooled allogeneic MSC composition. Examples 7-10 describe a clinical study using said pooled allogeneic MSC composition for treatment and/or prevention of type 1 diabetes, in particular for preserving endogenous insulin production in patients diagnosed with type 1 diabetes.

As used in the Example section the following terms have the meaning as explained below:

*Master Cell Stock* - Term used to define Drug Substance Intermediate at certain passage. In the example presented herein, Master Cell Stock is the Drug Substance intermediate at passage 1.

*Drug Substance Intermediate* - Term used to define MSCs from a single donor that are in production, hence being expanded. Meeting in process quality criteria but has not yet been evaluated with the selection algorithm. Drug Substance Intermediate corresponds to individual donor derived MSC population as disclosed herein, which individual donor derived MSC population has not yet been selected for pooling.

*Drug Substance* - Term used to define MSCs from a single donor that meet in the manufacturing quality criteria and have been identified as having desired characteristics by the selection algorithm. Hence, not subject to further culturing or expansion. Drug Substance thus corresponds to individual donor derived MSC population as disclosed herein, which individual donor derived MSC population have been selected for pooling.

*Drug Product* - The term Drug Product refers to a cell suspension of *ex vivo* expanded Wharton's jelly derived mesenchymal stem cells (WJMSCs) from multiple donors which have been identified as having desired characteristics by the selection algorithm. Drug Product corresponds to the isolated, pooled allogeneic MSC populations as disclosed herein.

*Final Product* - The term Final Product refers to a pharmaceutical composition comprising the Drug Product and at least one pharmaceutically acceptable excipient or carrier.

## Example 1

The present Example describes the process of harvesting, transportation, *ex vivo* expansion, and cryopreservation of MSCs from Wharton's Jelly. Additionally, maternal blood is tested for infections agents. Furthermore, culture conditions are described.

## Materials and methods

The manufacturing for the Master Cell Stock of Wharton's Jelly-derived MSC is a continuous process from the donor qualification and subsequent *ex vivo* expansion in xeno-free culture system.

Umbilical cord (UC) samples are collected after natural delivery as well as caesarian sections after placenta expulsion and umbilical cord blood collection (for infectious agents screening). Maternal peripheral blood samples are also collected.

For minimizing the risk of contamination, disposable, sterile scissors and forceps are used and fragments of the umbilical cord are placed into sterile transportation containers filled with transportation liquid (99% Sodium Chloride (0.9% sol.)

(Fresenius Cat.no: PK03XE050PL) supplemented with 1% Antibiotic/Antimycotic solution (Gibco Cat.no: 15240-062)). Samples are transported inside of protective boxes to the Manufacturer's Laboratory. Transport conditions are monitored and UC tissue is processed within 72 hours of child delivery. Isolation of Wharton's Jelly tissue and culture of explants for cell isolation are performed in GMP lab with use of xeno-free, serum-free media and compounds.

Qualification of UC tissue as a source material requires providing complete responses to a medical questionnaire and submission of maternal peripheral blood sample collected within 7 days of the UC collection for infectious agents testing. Donor sampling, testing and screening (medical health questionnaire) is in accordance with Annex II of Directive 2006/17/EC. All donor test kits are validated for intended use. Infectious agent tests performed before umbilical cord qualification are listed in Table 1. Approximately 10-25% of collected samples qualify for further production.

*Table 1. Infectious agent tests performed from maternal blood (MB) samples. \*In case of reactive results CMV IgG of maternal blood screening, additional test of Real Time PCR is performed from primary culture. Negative result of RT PCR CMV are required for product release.*

| Agent/Disease | Test specification | Results required |
| --- | --- | --- |
| HIV I, HIV II | anti-H IV I/II | (-) nonreactive |
| HIV I | HIV I Nucleic Acid Test (NAT) | (-) negative |
| HBV | anti-HB core | (-) nonreactive |
| HBV | HBs-Ag | (-) nonreactive |
| HBV | HBV Nucleic Acid Test (NAT) | (-) negative |
| HCV | anti-HCV | (-) nonreactive |
| HCV | HCV Nucleic Acid Test (NAT) | (-) negative |
| CMV | anti-CMV M | (-) nonreactive |
| CMV | anti-CMV G | (-) nonreactive* |
| Toxoplasmosis | anti-Toxo M | (-) nonreactive |
| Toxoplasmosis | anti-Toxo G | (-) nonreactive |
| Syphilis | Specific treponemal antibody assay | (-) nonreactive |

Upon arrival at the Manufacturer's Laboratory, UC fragments are removed from transportation container and washed in a sterile transportation liquid. UC is dissected and blood vessels are removed. Wharton Jelly tissue is minced into 1-2 mm$^3$ scraps with a sterile lancet and placed in xeno-free, serum-free media into culture flask coated with Attachment Solution (1% MSC Attachment Solution Stock 99% D-PBS) for primary explants cultures. Flask are incubated at 37°C in 5% $CO_2$. After 1-2 weeks cultures are examined for the presence of adherent, fibroblast-like cells. All non-adherent cells presence in cultures are washed out. The cell culture medium comprises 94% NutriStem® XF (Biological Science, Cat no: 05-200-1A), 5% NutriStem® XF Supplement Mix (Biological Science, Cat no: 05-201-1U) and 1% Antibiotic/Antimycotic solution (Gibco Cat.no: 15240-062). Adherent cells from primary are passaged (controlled enzyme digestion of cultures) upon reaching 90% confluence and reseeded at 1-1.5 × 10$^5$ per cm$^2$ for further expansion in p.1 culture. After 2-3 weeks of expansion, a Master Cell Stock (which is passage 1 adherent cells,) is harvested and

(i) cryopreserved in the presence of cryoprotectant solution (70-80% Human Serum Albumin (5% sol.) (CSL Behring Cat.no: Alburex 5) and 20-30% Dimethyl Sulfoxide (WAK Chemie, Cat.no: WAK-DMSO-50)) for vapor phase of liquid nitrogen storage or
(ii) immediately reseeded for further expansion.

When cells after passage 1 are reseeded for further expansion, the Master Cell Stock exist few hours only. Regardless of this short lifespan of MCS, all tests mentioned in Fig. 1 are performed.

*Table 2. Solutions used in steps of manufacturing process according to the Figure 1.*

| Name of solution | Composition | Step of process |
| --- | --- | --- |
| Transportation Liquid | 99% Sodium Chloride (0.9% sol.)<br>1 % Antibiotic/Antimycotic solution | 1 |
| Culture Medium | 94% NutriStem® XF<br>5% NutriStem® XF Supplement Mix<br>1 % Antibiotic/Antimycotic solution | 2-6 |
| Attachment Solution | 1% MSC Attachment Solution Stock 99% D-PBS | 2-6 |

(continued)

| Name of solution | Composition | Step of process |
|---|---|---|
| Cryoprotectant Solution* | 70-80% Human Serum Albumin (5% sol.) 20-30% Dimethyl Sulfoxide | 7 |
| *when Master Cell Stock is intended to be cryopreserved | | |

There are no animal-origin raw materials used in the manufacturing process from umbilical cord tissue collection until Drug Product release.

During primary cultures of explants, and at the end of manufacturing of Master Cell Stock each cell passage, samples are taken to determine the presence of bacterial and fungal contamination, mycoplasma and endotoxins. The number and viability of cells is evaluated in the Master Cell Stock and Drug Product. Microbial culture, mycoplasma and endotoxins is evaluated from final product. Additionally, one reference sample of Drug Product is can be thawed and testing cell culture is established. This testing culture serve as a source of material for additional final confirmation of product safety purity (by microbial culture and mycoplasma and endotoxins test, karyotype etc.), potency (cell number, adherence efficiency and viability) and identity (cytometric immunophenotyping). Cultures fulfilling the approval criteria listed in Table 3 qualify for next steps of processing or cryopreservation and analytical procedures for evaluation of cultures are explained in Example 2.

## Results

Manufacturer demonstrated the microbiological safety of WJMSC derived from umbilical cords obtained after natural deliveries. The addition of antibiotic/antimycotic solution to the transportation liquid and during next steps of manufacturing resulted in the absence of microorganism (bacteria and fungi) in Master Cell Stock as well as in Drug Product.

The retrospective analysis the characteristic of cells obtained from different donors allow to get criteria to be met by all MCSs for manufacturing comparable Drug Products as is referred in Table 4.

Results of analysis of five Master Cell Stocks derived from different donors, presented in the Table 6 reveals high similarity of cells characteristics. Thus, the present procedures provide highly uniform MSC populations which fulfill the required safety criteria.

*Table 3. Tests and acceptance specification*

| Test or Specification | Goal | Criteria for approval |
|---|---|---|
| Sterility of in-process media | safety, purity | bacteria/fungi not detected |
| Sterility of Master Cell Stock | safety, purity | bacteria/fungi not detected |
| Mycoplasma | safety, purity | not detected |
| Endotoxins | safety, purity | not detected (<0.25 IU/ml) |
| Cell count | potency | $\geq 200 \times 10^6$ |
| Cell viability | potency | $\geq 80\%$ |
| Surface adherence | potency, identity | $\geq 90\%$ of adherent cells |
| Karyotype | safety | 46 and sex chromosomes |
| CD45 antigen expression | identity | < 5% |
| CD34 antigen expression | identity | < 5% |
| CD14 antigen expression | identity | < 5% |
| CD19 antigen expression | identity | < 5% |
| CD3 antigen expression | identity | < 5% |
| CD73 antigen expression | identity | $\geq 70\%$ |
| CD90 antigen expression | identity | $\geq 70\%$ |
| CD105 antigen expression | identity | $\geq 70\%$ |

*Table 4. Analysis of different batches of Master Cell Stock*

| Test | Batch # | | | | | | | | |
| --- | --- | --- | --- | --- | --- | --- | --- | --- | --- |
| | TST 112 | TST 115 | TST 116 | TST 145 | TST 171 | TST 188 | TST 213 | TST 198 | TST 392 |
| Inf. agents | neg* | neg | neg* | neg* | neg | neg | neg* | neg | neg |
| Sterility | neg | neg | neg | neg | neg | neg | neg | neg | neg |
| Mycoplasma | neg | neg | neg | neg | neg | neg | neg | neg | neg |
| Endotoxins | neg | neg | neg | neg | neg | neg | neg | neg | neg |
| Viability (%) | 95% | 96% | 95% | 95% | 95% | 95% | 95% | 94% | 94% |
| CD45** | 0.69% | 3.27% | 0.07% | 2.68% | 0.37% | 4.45% | 2.3% | 1.14% | 1.45% |
| CD34** | 0.69% | 3.27% | 0.07% | 2.68% | 0.37% | 4.45% | 2.3% | 1.14% | 1.45% |
| CD14** | 0.69% | 3.27% | 0.07% | 2.68% | 0.37% | 4.45% | 2.3% | 1.14% | 1.45% |
| CD19** | 0.69% | 3.27% | 0.07% | 2.68% | 0.37% | 4.45% | 2.3% | 1.14% | 1.45% |
| HLA-DR** | 0.69% | 3.27% | 0.07% | 2.68% | 0.37% | 4.45% | 2.3% | 1.14% | 1.45% |
| CD73 | 99% | 99% | 89% | 92% | 89% | 99% | 98% | 96% | 99% |
| CD90 | 99% | 99% | 98% | 99% | 99% | 99% | 84% | 99% | 99% |
| CD105 | 97% | 94% | 76% | 96% | 78% | 83% | 93% | 88% | 99% |
| * Positive results of CMV IgG from maternal blood. Real time PCR test from primary culture has confirmed the lack of viral DNA in culture cells<br>** Tests performed in one antibody panel | | | | | | | | | |

## Example 2

The present Example describes characterization of MSCs from donors based on morphology, proliferative capacity and expression of markers for MSC according to the criteria of the ISCT. Furthermore, the cells are screened for the presence of mycoplasma, endotoxins, bacterial contaminants, fungal contaminants, viral contaminants and/or endotoxins and karyotype testing is performed. The described characterization results in identification of MSC populations derived from Drug Substance Intermediates, which MSCs fulfill quality criteria for pooling.

### Materials and methods:

First, MSCs must be plastic-adherent when maintained in standard culture conditions. Only plastic adherent cells are subject to the analytical procedures described below. Cultures are screened according to the analytical procedures given below.

### *Analytical Procedures*

*Infectious Agents.*

Sampling. The source material for WJ-MSC manufacturing (placental part of the umbilical cord) is obtained within several minutes after placenta expulsion. That is why the only way of infectious agent transmission is from maternal blood via placenta. Two samples of donor-mother's peripheral blood are collected at the day of delivery and source tissue harvest.

Analysis. ABBOTT ARCHITECT 2000 for chemiluminescent immunoassay and Procleix PANTHER System for NAT assay are used according to manufacturer's instructions. The following test using the Abbott ACHITECT for chemiluminescent immunoassay are performed HIV Ag/Ab Combo; HBsAg Qualitative II; Anti-HBc II; Anti-HCV; CMV IgM; CMV IgG; Toxo IgM; Toxo IgG; and Syphilis TP. Additionally, Proclex Utrio Plus Assay is used to qualitative screen *in vitro* nucleic acid amplification for HIV-1 RNA, hepatitis C virus (HCV) RNA and hepatitis B virus (HBV) DNA in plasma and serum specimens from human donors.

Acceptance criteria. Results of test must be "negative", "non-reactive" or "not detected" for infectious agents (except CMV IgG: having positive results of this test Manufacturer performs confirm the lack of CMV DNA in the product by RealTime PCR test).

*Sterility.*

<u>Sampling</u>. A sample (10 mL) of cells and supernatant from cultures after enzymatic harvest.
<u>Analysis</u>. Sample is seeded into two BACTEC bottles intended for growth of anaerobic and aerobic bacteria as well as for detection of fungal contamination. Bottles are placed in BACTEC FX400 microbial analyzer for 14 days.
<u>Acceptance criteria</u>. Results of test must be "negative" or "not detected" for aerobic anaerobic bacteria as well as for fungal microorganisms after 14 days incubation.

*Mycoplasma.*

<u>Sampling</u>. A sample (0.1 mL) of cells and supernatant from cultures after enzymatic harvest
<u>Analysis</u>. The Venor®GeM Classic Assay (Merck KGaA, cat no MP0025) is based on PCR amplification is uses according to the manufacturer's instructions.
<u>Acceptance criteria</u>. Results of test must be "not detected" for amplification product in the gel slot.

*Endotoxin.*

<u>Sampling</u>. A sample (0.5 mL) of cells and supernatant from cultures after enzymatic harvest
<u>Analysis</u>. The Endosafe®-PTS™, (Charles River Laboratories, cat no PTS2005F) real-time endotoxin testing system, is used according to the manufacturer's recommendations.
<u>Acceptance criteria</u>. Results of test must be "not detected"

*Cell Count.*

<u>Sampling</u>. A sample (0.5 mL) of cells from cultures after enzymatic harvest Analysis. Light microscopic analysis of cell number with use of Malassez' chamber.
<u>Acceptance criteria</u>. Not less than 98% of required number of cells

*Cell Viability.*

<u>Sampling</u>. A sample (0.5 mL) of cells from cultures after enzymatic harvest <u>Analysis</u>. Flow cytometric analysis of 7-AAD (Becton, Dickinson and Company, Cat.no. 559925) stained cell suspension is performed using a FACS Calibur flow cytometer.
<u>Acceptance criteria</u>. More than 80% of viable cells.

*Immunophenotyping.*

<u>Sampling</u>. A sample (0.5 mL) of cells from cultures after enzymatic harvest <u>Analysis</u>. Flow cytometric analysis of cells previously labelled with monoclonal antibodies is performed using a FACS Calibur flow cytometer Antigens tested a listed in Table 4.
<u>Acceptance criteria</u>- Expression of CD73, CD90 and CD105 on more than 70% of cells. Lack of expression of lineage antigens (CD45, CD34, CD14 or CD11b, CD79alpha or CD19 and HLA-DR surface molecules).

*Karyology.*

<u>Sampling</u>. Cell culture performed especially for this assay.
<u>Analysis</u>. Culture is blocked with Colcemid and stained with Giemsa. The number of chromosomes and structural aberrations are evaluated.
<u>Acceptance criteria</u>. 46 chromosomes, XY; no visible aberrations.

*Differentiation* assay

Cells are subject to differentiation
<u>Analysis</u>. Differentiation assays are used according to manufacturer's instructions. Human Mesenchymal Stem Cell Functional Identification Kit, Catalog Number SC006, R&D Systems, Inc. designed for the identification of human MSCs based on their ability to differentiate into multiple mesenchymal lineages. This kit contains specially formulated adipogenesis, chondrogenesis, and osteogenesis media supplements, which can be used to effectively differentiate MSCs into adipogenic, chondrogenic, or osteogenic lineages. A panel of antibodies, consisting of anti-mFABP4, anti-hAggrecan, and anti-hOsteocalcin, are included to define the mature phenotypes of adipocytes, chondrocytes, and osteocytes, respectively. StemPro® Chondrogenesis Differentiation Kit, Catalogue number: A1007101, Thermo Fisher Scientific Inc. developed for the chondrogenic differentiation of mesenchymal stem cells (MSCs) in tissue-culture vessels. The kit contains all reagents required for inducing MSCs to be committed to the chondrogenesis pathway and generate chondrocytes.

Acceptance criteria. Ability to differentiate to osteoblasts, adipocytes and chondroblasts *in vitro.*

**Results**

Obtained MSC populations are plastic-adherent when maintained in standard culture conditions. The MSC express CD105, CD73 and CD90, and lack expression of CD45, CD34, CD14 or CD11b, CD79alpha or CD19 and HLA-DR surface molecules as given in Example 3 and are able to differentiate to osteoblasts, adipocytes and chondroblasts *in vitro.* Thus, MSC populations eligible for pooling are identified.

While these criteria are currently employed, they may require modification as new knowledge unfolds leading to for example alteration of the definition of MSC according to the criteria of the ISCT), the present inventors believe the above minimal set of standard criteria will foster a more uniform characterization of MSC. As used herein, MSCs are defined according to criteria from ISCT.

**Example 3**

The present Example describes the screening assays used to characterize the said MSC populations derived from Drug Substance intermediates for morphological, proliferative and functional characteristics in order to select the MSC populations to be pooled.

**Materials and methods:**

Below follows the description of 6 assays used to characterize the MSC populations.

*Assay 1 -IDO:* IDO assay is used to analyze the immunosuppressive capacity of Drug Substance Intermediate or Drug Substance, i.e. mesenchymal stem/stroma cells (MSC).

The UC-MSC immunomodulatory potential is reported as a measure of indoleamine 2,3-dioxygenase (IDO) activity, determined by measuring tryptophan and kynurenine in the culture supernatant. Indoleamine-pyrrole 2, 3-dioxygenase (IDO or INDO EC 1.13.11.52) is a heme-containing enzyme that in humans is encoded by the IDO1 gene. The IDO enzyme converts L-tryptophan to N-formylkynurenine (or kynurenine), an immunosuppressive molecule that acts as an inhibitor of immune cell proliferation - including T cells. The IDO activity is the ratio of kynurenine/tryptophan and can be determined by calculating the amount of tryptophan and kynurenine present in cell culture supernatants using an ELISA kit. When stimulated with interferon gamma (IFNy), mesenchymal stem/stroma cells (MSC) secrete more IDO than when they are unstimulated.

Inducible IDO activity indicates that the cells released have functional potency, related to immunomodulation.

*MSC culturing:* Seed 10 000 MSC / well in 48-well cell culture plates in 100 $\mu$l assay medium (DMEM, low glucose, GlutaMAX™ Supplement, pyruvate (ThermoFisher Scientific, cat no. 21885025) + 10% Fetal Bovine Serum, qualified, heat inactivated (ThermoFisher Scientific, cat no. 16140071)). Dilute IFNy from stock, 1 mg/ml (ThermoFisher Scientific, cat no. PHC4033). The final concentration of IFNy / well is 100 ng/ml. Add 100 $\mu$l of 200 ng/ml IFN$\gamma$ to the wells. Add 100 $\mu$l assay medium to non-stimulated cells (no IFN$\gamma$). Incubate cell culture plate at 37°C, 5% $CO_2$ for 72 hours. Remove the supernatant from each well and store in micro tubes at -20°C until further processing for ELISA analysis.

Tryptophan and kynurenine measurements are done according to manuals provided by the ELISA-kit manufacturer (Immundiagnostik AG, cat no. K 3730 and K 3728). Both tryptophan and kynurenine ELISA are performed on the same day but at separate occasions. The two ELISAs are conducted according to manufacturer's instructions; see the manuals for respective ELISA.

Absorption at 450 nm with background subtraction at 620 nm is measured in a Spectramax microplate reader (Molecular Devices, Spectramax 190).

*Analyzing results:* Amount of absorbance measured is inversely proportional to the amount of amino acid present in the sample; i.e. the lower the OD450, the more kynurenine or tryptophan there is. The 4PL-algorithm (Four Parameter Logistic Regression) is used to calculate results (software SoftMax Pro 7.0.2, Molecular Devices), as recommended by kit manufacturer. Concentrations are determined directly from the standard curve. The control samples provided with the kits should are evaluated for acceptability: if outside the acceptable range according to the manufacturer of the kit, the samples need to be re-assayed.

*Results*

Relative IDO bioactivity of IFNy treated cells from Drug Substance Intermediates are used for ranking of the samples according to the selection algorithm (Example 4). The donors with the highest increase in bioactivity get the highest ranking score. The ranking score (Table 5) is later used in the final selection of donor (see Example 4).

*Table 5. Illustrative example of ranking score based on IDO fold increase.*

| Donor | IDO fold increase | Ranking score | Donor | IDO fold increase | Ranking score |
|-------|-------------------|---------------|-------|-------------------|---------------|
| D5 | 150 | 3 | D3 | 112 | 2 |
| D7 | 130 | 3 | D2 | 100 | 1 |
| D4 | 125 | 3 | D8 | 90 | 1 |
| D6 | 124 | 2 | D10 | 85 | 0 |
| D1 | 112 | 2 | D9 | 80 | 0 |

*Assay 2: Proliferation assay*

This method is used to quantitatively measure the immunosuppressing effect of the Drug Substance Intermediate and/or Drug Substance, i.e. umbilical cord derived MSCs have on the proliferation of peripheral blood mononuclear cells (PBMC). MSC have been shown to suppress T-lymphocyte proliferation. Mixed lymphocyte reactions with MSC are frequently used to demonstrate the immunosuppressive activity of MSC.

Phytohaemagglutinin (PHA) is used as a mitogen which activates proliferation of T-lymphocytes. The immunosuppressive activity of Drug Substance Intermediate and/or Drug Substance is quantified as the decrease in proliferation of PHA stimulated T-lymphocytes.

*Culturing and CFSE priming:* 500 $\mu$l of working medium (RPM11640 (ThermoFisher Scientific, cat no. 12633012 + 2 mM Glutamax (ThermoFisher Scientific, cat no. 35050061) + 100U/ml Pest (ThermoFisher Scientific, cat no. 15140122) + 10% FBS (ThermoFisher Scientific, cat no. 16140071) MSC (2x $10^5$ cells/well) is seeded in 12-well cell culture plates. The plates are incubated at 37°C + 5% $CO_2$ for 2 hours for plastic adherence of cells. Lymphoprep™ kit is used for isolation of mononuclear cells from donated peripheral blood, retrieved from the blood central, according to manufacturer's instructions (Stem Cell Technologies, cat no. 07801). PBMC are suspended in RPMI 1640, $22 \times 10^6$ cells/ml. CellTrace™ CFSE Cell Proliferation Kit (ThermoFisher Scientific, cat no. C34554) is used for analysing the proliferation according to manufacturer's instruction.

*Analysis:* CFSE positive cells are analyzed by flow cytometry (Merck, Guava easyCyte 5HT). CFSE histogram includes three or four peaks and the first top from the right represents undivided cells (G0). The following tops show different generations (G1-G4). Proliferation Index (PI) is calculated as the total number of cells of all generations divided by the number of parent cells that entered cell division.

*Table 6. Illustrative example of ranking score based on proliferation index.*

| Donor | Proliferation Index | Ranking score | Donor | Proliferation Index | Ranking score |
|-------|---------------------|---------------|-------|---------------------|---------------|
| D7 | 0.90 | 3 | D1 | 1.02 | 2 |
| D5 | 0.92 | 3 | D8 | 1.03 | 1 |
| D6 | 0.98 | 3 | D2 | 1.03 | 1 |
| D4 | 1.00 | 2 | D9 | 1.05 | 0 |
| D3 | 1.00 | 2 | D10 | 1.11 | 0 |

*Results*

The average proliferation index for each Drug Substance Intermediates is used for relative comparison of the donors. The donors with the lowest PI get the highest ranking score. The ranking score (Table 6) is later used in the final selection of donor (see Example 4).

*Assay 3*: *Prostaglandin E2*

Prostaglandin E2 (PGE2) assay evaluates Drug Substance Intermediate and/or Drug Substance secretion of PGE2 when co-cultured with peripheral blood mononuclear cells (PBMCs) activated with Phytohemagglutinin (PHA).
*Cell culturing:* Cells are cultured in assay medium (DMEM, low glucose, GlutaMAX™ Supplement, pyruvate (ThermoFisher Scientific, cat no. 21885025) + 10% Fetal Bovine Serum, qualified, heat inactivated (ThermoFisher Scientific, cat no. 16140071)) for 3 days in co-culture cell ratio MSC-PBMC 1/5, in presence and absence of PHA (Merck, cat no. 11082132001). 40 000 MSCs are seeded per well in 12-well cell culture plates. Cell culture plates are incubated at 37°C, 5% $CO_2$ for 2 h to allow the cells to adhere.
Lymphoprep™ kit is used for isolation of mononuclear cells from donated peripheral blood, retrieved from the blood central, according to manufacturer's instructions (Stem Cell Technologies, cat no. 07801). PBMC are suspend in assay medium, $0.5 \times 10^6$ cells/ml and 400 µl is seeded into wells intended for PBMC. 500 µl assay medium is added to wells without PBMC. 100 pl/well of 100 pg/ml is added to PHA to PBMC containing wells and the cell culture plate is incubated at 37°C, 5% $CO_2$ for 72 hours. The supernatant is removed from each well and centrifuged in 5 min 500 g to remove particulates. The supernatant is frozen and stored at -20°C until further processing for ELISA analysis.
The Parameter™ Prostaglandin E2 Immunoassay kit is used for PGE2 expression detection according to manufacturer's instruction (Bio-Techne, cat no. KGE004B) and is analyzed with Spectramax microplate reader (Molecular Devices, Spectramax 190). The 4PL-algorithm (Four Parameter Logistic Regression) is used to calculate results (software SoftMax Pro 7.0.2, Molecular Devices).

*Results*

The average expression of PGE2 in pg/ml for each Drug Substance Intermediate is used for relative comparison of the donors. The donors with the highest expression level get the highest ranking score. The ranking score (Table 7) is later used in the final selection of donor (see Example 4).

*Table 7. Illustrative example of ranking score based on PGE2 expression.*

| Donor | PGE2 expression | Ranking score | Donor | PGE2 expression | Ranking score |
|-------|-----------------|---------------|-------|-----------------|---------------|
| D5 | 14900 | 3 | D3 | 12400 | 2 |
| D7 | 13000 | 3 | D2 | 12000 | 1 |
| D4 | 12900 | 3 | D8 | 11100 | 1 |
| D6 | 12600 | 2 | D10 | 10000 | 0 |
| D1 | 12500 | 2 | D9 | 9000 | 0 |

*Assay 4*: *HLA-G*

The HLA-G assay evaluates Drug Substance Intermediate and/or Drug Substance expression of soluble and/or intracellular HLA-G in response to IFNy, IL-10 and/or PHA.
*Cell culture:* 50 000 MSC and 25 000 JEG-3 cells (positive control cells, ATCC, cat no. ATCC® HTB-36™) are seeded per well in 12-well cell culture plates in 1 ml assay medium (DMEM, low glucose, GlutaMAX™ Supplement, pyruvate (ThermoFisher Scientific, cat no. 21885025) + 10% Fetal Bovine Serum, qualified, heat inactivated (ThermoFisher Scientific, cat no. 16140071)) with a final concentration of 10-50 ng/ml IL-10 (Miltenyi Biotec, cat no. 130-108-985) or 25-100 ng/ml IFNγ (ThermoFisher Scientific, cat no. PHC4033) or without stimulation. Next, cells are incubated at 37°C, 5% CO2 for 48h to 72h. The supernatant is removed from each well and stored at -20°C for ELISA analysis of soluble HLA-G. *Intracellular HLA-G:* The adherent cells are washed with DPBS twice and detach with TrypLE Express (Thermo Scientific, cat no. 12604021). The BD Cytofix/Cytoperm™ is used for fixation and permeabilization of cells according to manufacturer's instruction (Becton, Dickinson and Company, cat no. 554714). Cells are stained with HLA-G (PE) antibody (EXBIO Praha, cat no. 1P-431-C100) according to manufacturer's instruction and analyzed by flow cytometry (Merck, Guava easyCyte 5HT).
*Soluble HLA-G:* The concentration of HLA-G in the supernatant is analyzed with sHLA-G ELISA kit (Enzo Life Sciences, cat no. ALX-850-309-KI01) according to manufacturer's instruction.

*Results*

The Drug Substance Intermediates and/or Drug Substances are analyzed for both intracellular and soluble HLA-G expression and receive a score based on the relative expression compared to with the other samples analyzed. The total score from intracellular and soluble HLA-G expression is summarized and the Drug Substance Intermediates receive a ranking score (Table 8) that is used for the final selection of donors (see Example 4).

*Table 8. Illustrative example of ranking score based on sHLA-G and iHLA-G scores.*

| Donor | Soluble HLA-G | sHLA-G score | Intracellular HLA-G | iHLA-G score | HLA-G score | Ranking score |
|---|---|---|---|---|---|---|
| D5 | 1110 | 8 | 0% | 9 | 17 | 3 |
| D7 | 1100 | 7 | 1% | 6 | 13 | 3 |
| D4 | 990 | 4 | 0% | 9 | 13 | 3 |
| D6 | 1200 | 9 | 6% | 1 | 10 | 2 |
| D1 | 1002 | 6 | 1% | 6 | 12 | 2 |
| D3 | 980 | 3 | 0% | 9 | 12 | 2 |
| D2 | 1000 | 5 | 8% | 0 | 5 | 1 |
| D8 | 937 | 2 | 5% | 3 | 5 | 1 |
| D10 | 825 | 1 | 5% | 3 | 4 | 0 |
| D9 | 600 | 0 | 2% | 4 | 4 | 0 |

*Assay 5: Morphology*

Cell morphology of the Drug Substance Intermediate and/or Drug Substance cultures are continuously surveilled. Cells are being visually inspected during expansion as well as before harvesting and evaluated based on:

| | |
|---|---|
| • Size of cell | normal/big |
| • Size of nuclei | normal/big |
| • Shape of cell | normal/abnormal |
| • Ratio between cell and nuclei size | normal/abnormal |

*Results*

Drug Substance Intermediate cells are visually assessed based on the criteria above. Only samples with more than 90% normal cells are accepted. The frequency of abnormal cells is used for ranking (Table 9) the Drug Substance Intermediates (see Example 4).

*Table 9. Illustrative example of ranking score based on morphology.*

| Donor | Percentage abnormal cells | Ranking score | Donor | Percentage abnormal cells | Ranking score |
|---|---|---|---|---|---|
| D5 | 0% | 3 | D4 | 2% | 2 |
| D6 | 0% | 3 | D3 | 5% | 1 |
| D7 | 0% | 3 | D8 | 5% | 1 |
| D1 | 1% | 2 | D9 | 6% | 0 |
| D2 | 1% | 2 | D10 | 8% | 0 |

*Assay 6: Fluorospot*

Drug Substance Intermediate and/or Drug Substance are cultured in Fluorospot specific 96 well plates pre-coated with antibodies (service provided by MabTech). 1000 - 3000 cells are seeded per well in 100 μl assay medium (DMEM, low glucose, GlutaMAX™ Supplement, pyruvate (ThermoFisher Scientific, cat no. 21885025) + 10% Fetal Bovine Serum, qualified, heat inactivated (ThermoFisher Scientific, cat no. 16140071)) and incubated for 48 hours in absence or presence of stimuli. Stimulations used are Poly I:C (Invivogen, cat no. tlrl-pic), r848 (Invivogen, cat no. tlrl-r848), GABA (Diamyd Medical) and IFNγ (ThermoFisher Scientific, cat no. PHC4033). The expression of IL-2, IL-4, IL-6, IL-8, IL-12, IL-12/13, IL-17A IL-21, IL-22, IL-29, IL-31, TGFβ1, GM-CFS, IFNα, IFNγ, apoE and TNFα is analyzed by Fluorospot (MabTech).

Earlier batches of the pooled allogeneic MSC composition, i.e. Drug Product, are used as reference. The assay contains both proteins and cytokines considered desirable and undesirable. For example, it considered positive if the cells are expressing IL-6 but negative if they express IFNγ.

*Table 10: Antibodies used in said Fluorospot-assay.*

| Fluorospot-assay | Detection antibody | Fluorospot-assay | Detection antibody |
|---|---|---|---|
| IL-2 | human mAb MT8G10-biotin, 0.5 mg/ml | IL-29 | human mAb MT6G4-biotin, 0.5 mg/ml |
| IL-4 | human mAb IL-4 II-biotin, 1 mg/ml | IL-31 | human mAb MT158-biotin, 0.5 mg/ml |
| IL-6 | human mAb 39C3-biotin, 1 mg/ml | IFNα (pan) | human mAbs MT2/4/6-biotin, 1 mg/ml |
| IL-8 | human mAb MT8F19-biotin, 0.5 mg/ml | IFNγ | human mAb 7-B6-1-biotin, 1 mg/ml |
| IL-13 | human mAb IL13-3-biotin, 0.5 mg/ml | TNFα | human mAb TNF5-biotin, 0.5 mg/ml |
| IL-21 | human mAb MT21.3m-biotin, 0.5 mg/ml | GM-CSF | human mAb 23B6-biotin, 1 mg/ml |
| IL-22 | human mAb MT7B27-biotin, 0.5 mg/ml | TGFβ1 (latent form) | human mAb MT517-biotin, 0.5 mg/ml |

*Results*

The results are analyzed with the software provided with the Fluorspot reader. The program generates both visual and numeric output (see Figure 3).

The Drug Substance Intermediate samples are scored in relation to the reference sample (numeric value). A threshold value for positive vs. negative is predefined for each marker and the Drug Substance Intermediates are scored according to Table 12.

*Table 1 1: Marker translation to numeric scores.*

| Type of marker | Result | Numeric score | Type of marker | Result | Numeric score |
|---|---|---|---|---|---|
| Positive markers | Negative | 0 | Negative markers | Negative | 0 |
| | Positive | 1 | | Positive | -2 |
| | Higher than reference | 2 | | | |

The final ranking of the Drug Substance Intermediates is based on the summarized score from all markers analyzed with and/or without stimuli (Table 12).

*Table 12. Illustrative example of ranking score for all markers analyzed.*

| Donor | M1 | M1 S1 | M2 | M3 | M4 S1 | M4 S2 | M4 S3 | Summarized score |
|---|---|---|---|---|---|---|---|---|
| **D1** | 1 | 1 | 0 | 0 | 1 | 1 | 1 | 5 |
| **D2** | 1 | 1 | 0 | 0 | 1 | 1 | 1 | 5 |

(continued)

| Donor | M1 | M1 S1 | M2 | M3 | M4 S1 | M4 S2 | M4 S3 | Summarized score |
|---|---|---|---|---|---|---|---|---|
| D3 | 1 | 1 | -1 | 0 | 1 | 1 | 1 | 4 |
| D4 | 1 | 1 | 0 | 0 | 1 | 1 | 1 | 5 |
| D5 | 2 | 1 | 0 | 0 | 2 | 1 | 1 | 7 |
| D6 | 2 | 2 | 0 | 0 | 1 | 2 | 1 | 8 |
| D7 | 1 | 2 | 0 | 0 | 2 | 1 | 1 | 7 |
| D8 | 0 | 0 | 0 | 0 | 1 | 1 | 1 | 3 |
| D9 | 0 | 0 | 0 | 0 | 0 | 0 | 1 | 1 |
| D10 | 1 | 1 | 0 | 0 | 0 | 0 | 1 | 3 |

*D1 = donor 1, D2 = donor 2 etc. M1 = marker 1, M2 = marker 2 etc. S1 = stimuli 1,*
*S2 = stimuli 2 etc. M1 and M4 are positive markers. M2 and M3 are negative markers.*

The ranking of the Drug Substance Intermediates is based on the score which generates are ranking score for the Fluorospot assay. The ranking score is later used for the overall selection of donors described in Example 4. The Drug Substance Intermediate sample with the highest score will also get the highest ranking score (see Table 13):

*Table 13. Illustrative example of ranking score based on Summarized score from the Fluorospot assay.*

| Donor | Score | Ranking score | Donor | Score | Ranking score |
|---|---|---|---|---|---|
| D6 | 8 | 3 | D4 | 5 | 2 |
| D5 | 7 | 3 | D3 | 4 | 1 |
| D7 | 7 | 3 | D8 | 3 | 1 |
| D1 | 5 | 2 | D9 | 3 | 0 |
| D2 | 5 | 2 | D10 | 1 | 0 |

Furthermore, it is also possible to use some or all of the Fluorospot results as input in the selection algorithm, i.e. data from each analyzed protein as a separate component in the selection algorithm.

## Example 4

The present Example describes the process of selection of the MSC populations derived from the donors based on the characteristics described in Example 3 resulting in a subset of cells populations for pooling to obtain the inventive pooled allogeneic composition.

## Material and methods:

*Analysis and ranking*: The Drug Substance Intermediate samples are analyzed with the assays described above (IDO, proliferation, PGE2, HLA-G, Morphology and Fluorospot). Ranking of samples is performed as described below:

1. The IDO assay described above is conducted two times with duplicate cell culture samples and each sample is analyzed in triplicates with ELISA. Earlier batches pooled allogeneic MSCs are used as reference samples. The IDO assay contains control samples, analyzed with each run and results generated from the analysis of control samples are evaluated for acceptability using appropriate statistical methods. Acceptable range of the two controls are according to manufacturer's specification. An example of acceptable ranges is: Kynurenine ($\mu$mol/L) control 1: 0.53 -1,33 and control 2: 1.78-4.15; Tryptophan ($\mu$mol/L) control 1: 15.0-35.0 and control 2: 31.2-72.8. Quality criteria employed for assay are: IDO controls are within the specified range and IDO activity (reference sample) > 60-fold, i.e. the fold induction of IDO activity between interferon gamma (IFN$\gamma$) reference sample compared to unstimulated reference sample.

The Drug Substance Intermediates are ranked based on their relative IDO expression.

2. The Proliferation assay described above is conducted two times with duplicate cell culture samples and each sample is analyzed in triplicates with FACS. The samples impact on PBMC proliferation is presented as proliferation index, PI. Earlier batches pooled allogeneic MSCs are used as reference samples and PBMC stimulated with PHA in absence of MSC is used as positive control. Quality criteria employed for assay are: Proliferation Index (positive control) > 1.5 and Proliferation Index (reference) 0.9-1.3.

The Drug Substance Intermediates are ranked based on their relative Proliferation Index

3. The PGE2 assay is conducted two times with duplicate cell culture samples and each sample is analyzed in triplicates with ELISA. The kit includes standards for establishing a standard curve for each experiment. Earlier batches pooled allogeneic MSCs are used as reference samples and the samples are compared based on the level of PGE2 expression in presence of PBMC activated by PHA. Quality criteria employed for assay are: PGE2 expression (reference) 5-15 ng/ml and Standard curves $R^2 > 0.95$.

The Drug Substance Intermediates are ranked based on their relative PGE2 expression.

4. The HLA-G assay is conducted two times with duplicate cell culture samples and each sample is analyzed in triplicates with ELISA or FACS. Earlier batches pooled allogeneic MSCs are used as reference samples and the samples are compared based on the level of HLA-G expression in presence of PBMC activated by PHA. The ELISA kit includes standards for establishing a standard curve for each experiment. Quality criteria employed for assay are: Soluble HLA-G expression (reference) >3 U/ml, Standard curves $R^2 > 0.95$ and Intracellular HLA-G expression (reference) >5%, The Drug Substance Intermediates are ranked based on their relative intracellular and/or soluble expression of HLA-G.

5. The morphology assessment is conducted by laboratory personnel with long experience in MSC culturing. Earlier batches pooled allogeneic MSCs are used as reference samples and the samples assessed based on: size of cell (normal or big); size of nuclei (normal or big); shape of cell (normal or abnormal); and ration between cell and nuclei size (normal or abnormal). Quality criteria employed for assay are: >90% normal cells according to all four criteria. Reference sample has > 90% normal cells. The Drug Substance Intermediates that have more than 10% abnormal cells are disqualified. The Drug Substance Intermediates are ranked based on the frequency of abnormal cells.

*Outliers and disqualification of samples*: ELISA and FACS are analyzed in triplicates from each cell culture well. Only one of the three triplicates can be regarded as an outlier. Outlier analysis is based on delta optical density for ELISA ($\Delta$OD) and Proliferation Index and HLA-G positive percentage for FACS. Measurements from a cell culture well are analyzed for outliers if the coefficient of variance (CV) is > 10%, The replicate which is deviating most from the average is considered an outlier if the exclusion of the replicate will decrease CV with > 3% when removed from the analysis. Such outliers are excluded from the analysis without further justifications.

The analysis of a single cell culture well is disqualified if the CV > 20% after outlier analysis has been conducted. Three or more disqualified cell culture wells in the same experiment will disqualify the experiment.

*Overall assessment:* The selection of Drug Substance Intermediates is an overall assessment of the assays according to a point system presented in Table 14 below. Each assay generates a ranking score and in this final selection, the ranking score of all the assays is summarized by addition. Selecting 5 donors from the 10 donors described in Example 3 is performed with each assay contributing equally to the total score and final decision as in Table 14. Ranking values are added for each assay to obtain an additive total score.

*Table 14. Example of selection based on additive total score.*

| Donor (DX) | IDO | PI | PGE2 | HLA-G | Morph. | FluoroS | Total score | Selected |
|---|---|---|---|---|---|---|---|---|
| D1 | 2 | 2 | 2 | 2 | 2 | 2 | 12 | yes |
| D2 | 1 | 1 | 1 | 1 | 2 | 2 | 8 | no |
| D3 | 2 | 2 | 2 | 2 | 1 | 1 | 10 | no |
| D4 | 3 | 2 | 3 | 3 | 2 | 2 | 15 | yes |
| D5 | 3 | 3 | 3 | 3 | 3 | 3 | 18 | yes |
| D6 | 2 | 3 | 2 | 2 | 3 | 3 | 15 | yes |
| D7 | 3 | 3 | 3 | 3 | 3 | 3 | 18 | yes |
| D8 | 1 | 1 | 1 | 1 | 1 | 1 | 6 | no |
| D9 | 0 | 0 | 0 | 0 | 0 | 0 | 0 | no |
| D10 | 0 | 0 | 0 | 0 | 0 | 0 | 0 | no |

Alternatively selecting 5 of the 10 donors is done by assigning a weight the assays, thus allowing an analysis to influence the selection of more or less donors. An example would be to put a factor two on morphology and decrease the importance of HLA-G assay to half. Weighed ranking scores are added to obtain a weighed total score. The results from Example 3 based on weighed total score are shown in Table 15:

*Table 15. Example of selection based on weighed total score.*

| Donor (DX) | IDO | PI | PGE2 | HLA-G | Morph. | FluoroSpot | Total score | Selected |
|---|---|---|---|---|---|---|---|---|
| D1 | 2 | 2 | 2 | 1 | 4 | 2 | 13 | yes |
| D2 | 1 | 1 | 1 | 0,5 | 4 | 2 | 9,5 | no |
| D3 | 2 | 2 | 2 | 1 | 2 | 1 | 10 | no |
| D4 | 3 | 2 | 3 | 1,5 | 4 | 2 | 15,5 | yes |
| D5 | 3 | 3 | 3 | 1,5 | 6 | 3 | 19,5 | yes |
| D6 | 2 | 3 | 2 | 1 | 6 | 3 | 17 | yes |
| D7 | 3 | 3 | 3 | 1,5 | 6 | 3 | 19,5 | yes |
| D8 | 1 | 1 | 1 | 0,5 | 2 | 1 | 6,5 | no |
| D9 | 0 | 0 | 0 | 0 | 0 | 0 | 0 | no |
| D10 | 0 | 0 | 0 | 0 | 0 | 0 | 0 | no |

## Results

The 5 Drug Substance Intermediates (DX) with the highest total score (additive/simple or weighed) are selected for manufacturing of the isolated pooled allogeneic MSC population, i.e. the Drug Product, as disclosed herein. Thus, the isolated, pooled allogeneic population comprises MSCs derived from 5 different donors, which MCSs fulfil the functional, morphological and safety criteria as disclosed herein.

## Example 5

The present Example describes the process of manufacturing the Final Product, which is a single cell suspension comprising excipients as described below. Said Final Product is filled in transfer bags suitable for cryopreservation and frozen according to predefined temperature curves as described below.

*Table 16. Composition packaging of Final Product* Alburex 5 (CSL Behring), 50G/L; human serum albumin 50 g/L; purity of protein > 96%, sodium N-acetyltryptophanate , sodium caprylate, sodium chloride*

| CELL SUSPENSION COMPONENTS | | | |
|---|---|---|---|
| Component | Function | Quantity | Quality |
| Allogeneic WJMSCs | Active Substance | $100 \times 10^6$ cells | In-house |
| 5% Human Serum Albumin* | Component of Cryoprotectant | 90% vol/vol (4.5 mL) | Ph.Eur |
| Dimethyl Sulfoxide (DMSO) ** | Component of Cryoprotectant | 10% vol/vol (0.5 mL) | Ph.Eur |
| PACKAGING COMPONENTS | | | |
| Component | Description | Function | Quality |
| Cryobags | Bag; ethylene vinyl acetate | Primary container | Ph.Eur |
| Cassette | Container; aluminum alloy | Protective container | In-house |
| Label | Cryo label | Information | In-house |
| ** WAK Chemie, Cat.no. WAK-DMSO-50. | | | |

## Materials and Methods

*Pooling of donors*

Only donor samples that pass all acceptance criteria are considered for pooling. The Drug Substances used are selected according to Example 3 and 4. The pooling of Drug Substance in passage 3 is directly followed by cryopreservation. The Drug Product is thus obtained. Importantly, the Drug Product is not subjected to any further culturing or expansion.

*Formulation and packaging of Drug Product*

The Final Product is a 5 mL of cell suspension and is presented in cryobags.
The composition of cryopreserved Final Product, comprising the Drug product, is shown in Table 16:

## Results

Thus, a resulting Final Product is obtained as disclosed herein.

## Example 6

The present Example describes evaluation of the stability of the Final Product after cryopreservation. It shows that the Final Product is stable for at least 2 hours post thawing.

## Materials and Methods

The inventive composition is shipped on liquid nitrogen or on dry ice in cryo bags containing 5 ml of cell suspension with 100 million cells per bag, the Final Product. The cryo bag is thawed in water bath (37°C) and directly transferred to infusion bag, usually containing 100 ml sodium chloride solution. The infusion solution of 105 ml is then ready for infusion. The viability of the cells is analyzed by taking a sample from the infusion bag at different time points. The function of the cells is further analyzed by taking samples of approximately $1 \times 10^6$ cells and culture them at 5 min, 1 h and 2 h after thawing in 95% NutriStem® XF (Biological Science, Cat no: 05-200-1A), 5% NutriStem® XF Supplement Mix (Biological Science, Cat no: 05-201-1U) - NutriStem is only used once, thereafter it will be exchanged to assay medium (DMEM, low glucose, GlutaMAX™ Supplement, pyruvate (ThermoFisher Scientific, cat no. 21885025) + 10% Fetal Bovine Serum, qualified, heat inactivated (ThermoFisher Scientific, cat no. 16140071)). Plastic adherence of the cells is monitored visually and micrographs are taken at appropriate time intervals. Viability analysis of cells after thawing is done by flow cytometry (Merck, Guava easyCyte 5HT) analysis of 7-Amino-Actinomycin D (7-AAD, Becton, Dickson and Company, cat no. 559925) according to manufacturer's instruction. Phenotypic characterization by CD90 (Becton, Dickson and Company, cat no. 561557) and HLA-DR (Exbio, cat no. IF-690-T100) is analyzed according to manufacturer's instruction.

## Results

*Viability:* Cells are considered stable when viability is >80%, hence CB1 is stable 24 h after thawing, when stored at RT, as shown in Table 17.

Table 17. Viability results for CB1 at post-thaw times 22 min - 24 h.

| Time post-thaw | Viability % | SD % | CV % |
|---|---|---|---|
| 22 min | 94.9 | 1.9 | 2 |
| 40 min | 99.2 | 0.15 | 0.15 |
| 1 h | 98.4 | 0.27 | 0.27 |
| 2 h | 98.0 | 0.11 | 0.11 |
| 3 h | 97.5 | 0.13 | 0.13 |
| 24 h | 92.7 | 0.11 | 0.11 |

*Phenotypic characterization:* CB1 cells grown in MSC Nutristem basal medium as well as in DMEM showed a high percentage of CD90 surface markers, > 99%, regardless of time in RT before start of culture. HLA-DR was found to be negative (see Table 18).

*Table 18. Single staining of CD90 and HLA-DR surface markers in CB1 cells cultured in MSC Nutristem basal medium (NS) or DMEM + 10% FBS (DMEM).*

|  | NS 5 min | NS 1 h | NS 2 h | DMEM 5 min | DMEM 1 h | DMEM 2 h | Acceptable result |
|---|---|---|---|---|---|---|---|
| CD90 % | 99.62 | 99.72 | 99.48 | 99.65 | 99.55 | 99.82 | $\geq 70$ |
| HLA-DR % | 0.01 | 0.02 | 0.02 | 0.14 | 0.17 | 0.06 | < 5 |

*Surface adherence:* Cells attached already after 1 h culture to the surface of T-75 flasks when grown in MSC Nutristem basal medium. Cells grown in DMEM + 10% FBS attached after 24 h culture.

*Conclusion:* The analyzed batch the inventive composition fulfills quality criteria with cell viability > 97% after 3h at RT and > 92% after 24 h at room temperature. Also, CD90 surface marker is positive and HLA-DR staining is negative in cells cultured 5 min, 1 h and 2 h after thawing. Surface adherence was faster with cells cultured in the MSC-specific medium (MSC Nutristem basal medium) than when cultured in conventional cell growth medium. After 24h, cells from all "post-thaw times" had attached. Since all criteria tested in-house are well within acceptable results, the high quality of this batch is verified.

## Example 7

The present Example provides a summary of the clinical study design of infusion of the inventive pooled allogeneic MSC composition into patients diagnosed with type 1 diabetes. Safety and tolerance as well as changes in beta-cell function, metabolic control and diabetes treatment satisfaction are examined. Any adverse events will be reported and potential causal relationship with Final Product will be investigated.

*Study design:* A combined phase I and phase II study is performed. The first part is an open, dose escalating study consisting of 9 male patients, 18-40 years of age. The second part is a randomized, double-blinded, placebo-controlled, phase I/II study in parallel design comparing treatment with the pooled allogeneic MSC composition as disclosed herein to placebo in adult patients diagnosed with type 1 diabetes. Safety, preservation of endogenous insulin production (measured as C-peptide concentrations) together with metabolic control, diabetes treatment satisfaction and immunological profile are assessed.

A total number of 24 patients are enrolled in the study (9 patients in the first part and 15 patients in the second part) and followed for one year after Final Product/placebo treatment. Inclusion and exclusion criteria are described in Example 8.

In the first part of the study patients 1-3 receive a single dose of 25 million cells, patients 4-6 receive 100 million cells and patients 7-9 receive 200 million cells. The 15 patients in the second part of the study will be allocated with a ratio of 1:1:1 to one of three arms: A. Allogeneic infusion with WJMSCs (batch 1), i.e. Final product batch 1); B. Allogeneic infusion with WJMSCs (batch 2) i.e. Final Product batch 2); and C. placebo infusion. In the statistical analysis group A and B will be pooled and compared with group C.

In the second part of the study all patients will receive a fixed single dose of Final Product. A preliminary proposed dose is 100 million MSCs, but no dosing will occur in part 2 before data from part 1 is evaluated to confirm the preliminary proposed dose.

The study starts with a screening period to obtain informed consent, screening, and inclusion to the study. Inclusion to the study must be within two years of type 1 diabetes diagnosis. Throughout the study, all patients will continue their insulin treatment, with insulin doses adjusted to maintain optimal blood glucose control as per clinical practice. All patients, 1-24, will follow the set visit schedule and during each visit a set of tests and procedures will take place according to Table 19.

*Table 19. Overview of study. Visits 3 and 4 may be performed +/- 3 days, visit 5 may be performed +/- 7 days and 6-8 may be performed +/- 14 days from day indicated above.*

|  | Screening visit | Baseline visit | Treatment | Follow up | | | | |
|---|---|---|---|---|---|---|---|---|
| Visit number | 1 | 2 | 3 | 4 | 5 | 6 | 7 | 8 |

(continued)

| | Screening visit | Baseline visit | Treatment | Follow up | | | | |
|---|---|---|---|---|---|---|---|---|
| Days | -14 -0 | 0 | 7 | 37 | 97 | 187 | 277 | 372 |
| **Informed consent** | X | | | | | | | |
| **Eligibility criteria** | X | | | | | | | |
| **Demography** | X | | | | | | | |
| **Medical history** | X | | | | | | | |
| **Concomitant medications** | X | X | X | X | X | X | X | X |
| **Baseline symptoms** | X | X | | | | | | |
| **Adverse events** | | X | X | X | X | X | X | X |
| **Optimizing diabetes care** | X | X | X | X | X | X | X | X |
| **Randomization** | | X | | | | | | |
| **Insulin requirement** | | X | | | | X | | X |
| **MMTT** | | X | | | | | | X |
| **CGM** | | X | | | | | | X |
| **WJMSC/placebo infusion** | | | X | | | | | |
| **DTSQ** | | X | | | | | | X |
| **Immunology tests** | | X | | X | X | X | | X |
| **Clinical chemistry** | X | X | | X | X | | | X |
| **HLA class I genotype** | | X | | | | | | |
| **HbA1c** | X | X | | X | X | X | X | X |
| **Pregnancy test /HCG)** | X | | | | | | | |
| **Vital signs (heart rate, blood pressure)** | X | X | X | X | X | X | X | X |
| **Dispensing diary card** | | X | X | X | X | X | X | |
| **Collecting diary card** | | | X | X | X | X | X | X |

The end of study is defined as the last participant's last follow up.

Throughout the study, patient safety is of importance. Each serious adverse effect (SAE) that is at least possibly related to the Final Product is to be classified by the investigator as expected or unexpected and followed up according to protocol.

### Results

Clear insights into the medical situation of the 24 included patients. This includes safety and adverse events parameters and will allow to gain insights into the set efficacy endpoints.

No or only minor side effects of MSC treatment have previously been observed in clinical studies for a number of diseases such as graft versus host disease, tissue regeneration after myocardial infarct or liver cirrhosis, or in osteogenesis imperfecta. No increased risk of tumor development in patients is known, and no ectopic tissue formation has been observed (von Bahr et al., (2012) Biol Blood Marrow Transplant; 18: 557-564, von Bahr L et al.,(2012). Stem Cells; 30: 1575-1578). Similarly, in first studies in adult patients newly diagnosed for type 1 diabetes no side effects were observed (Hu J et al., (2013). Endocr J; 60: 347-357, Carlsson PO et al., (2015) Diabetes. 2015; 64(2):587-92).

WJMSC from the manufacturer used for the present study have previously been used in hospital exemption procedures for various conditions and the safety profile of the cells is consistent and well tolerated with only mild and transient adverse reactions related to the product. However, serious adverse events and deaths has been reported, caused by the underlying disease, for instant terminal ALS patients and patients suffering grade 4 GVHD, receiving therapy. In the present study, any expected adverse events are mild and transient flu-like symptoms.

The present inventive treatment with the Final product, which comprises the Drug Product, is an allogeneic transplantation of cells from multiple donors and HLA mismatch is guaranteed. There is a theoretical risk of HLA immunization of the patients that might be devastating if the T1DM patient later in life is in need of a kidney transplantation.

Antibodies against foreign HLA without clinical relevance are expected in up to 20% of the patients.

A successful intervention would be highly beneficial for subjected patients likely providing them with a lower HbA1c, less blood glucose fluctuations, and diminished risk of ketoacidosis. It would also substantially decrease the risks of severe hypoglycemic events and late complications. The decrease in C-peptide concentration pre-treatment and 12 month post treatment is expected to be less for patients receiving the inventive Final Product as compared to patients receiving placebo (control).

## Example 8

The present Example describes the selection criteria for the study population. Each patient enrolled in the study has to fulfill all inclusion criteria and none of the exclusion criteria.

*Inclusion and Exclusion criteria:* Subjects will be recruited from the population of newly diagnosed type 1 diabetes patients.

*Patients eligible for inclusion in this study must fulfill the following criteria:* 1. Given written informed consent for participation of the study; 2. Clinical history compatible with type 1 diabetes diagnosed less than 2 years before enrolment; 3. In the first part of the study patients 1-9 only male patients between 18-40 years of age will be included. In the second part of the study, patients 10-24, both male and female patients 18 to 40 years of age (inclusive at both ends) will be included; 4. Fasting plasma C-peptide concentration >0.12 nmol/L; and 5. Nonpregnant women and using approved method of contraception/abstinence.

*Patients fulfilling any of the following criteria at screening are not eligible for inclusion in this study:* 1. Inability to provide informed consent; 2. Patients with body mass index (BMI) > 30, or weight >100 kg; 3. Patients with weight <50 kg; 4. Patients with unstable cardiovascular status incl. NYHA class III/IV or symptoms of angina pectoris, uncontrolled hypertension (≥160/105 mmHg), active on-going infections,
tuberculosis, or at risk of tuberculosis or mycosis, HIV, Treponema pallidum, hepatitis B antigen or hepatitis C, demyelinating disease, proliferative retinopathy and previous or known malignancy; 5. Patients with any immune suppressive treatment; 6. Pregnant or lactating women; 7. Taking oral anti-diabetic therapies or any other concomitant medication which may interfere with glucose regulation other than insulin; 8. Patients with GFR <80 ml/min/1.73 m2 body surface; 9. Patients with known hypersensitivity against any excipients, i.e. dimethyl sulfoxide (DMSO).

### Results

Study population of 24 individuals is selected based on the criteria described above.

## Example 9

The present Example describes how the clinical study is performed. Herein the inventive pooled allogeneic MSC composition is referred to as Drug Product and the pharmaceutical composition as Final Product.

### Material and Methods

The Final Product was a cell suspension with MSC's produced from donated Wharton's Jelly from umbilical cord tissue and expanded in adherent culture over approximately 4-5 weeks (a maximum of 3 passages) as described in Examples 1-5. The treatment was allogenic and consists of pooled expanded MSC from 5-10 different donors. Minimum 72 hours before infusion, the investigator sent a requisition to the Manufacturer for delivery of the Final Product. On the day of infusion, the applicable Final Product was transported by the Manufacturer to the investigator site. The Final Product was stored in a cryo bag in liquid nitrogen transportation canister. The Final Product was thawed bed-side in a water bath with sterile saline solution and diluted in 100ml saline resulting in an infusion volume of 105 ml. The Final Product was administered to the patient within 30 minutes after preparation.

Both the subjects and the study personnel were blinded to the subject's treatment. Placebo was provided in identical tissue bags as the Final Product. Subjects, the investigator, study site personnel and sponsor personnel involved with data review and analysis, remained blinded to study treatment throughout the study until the data base is clean and locked.

All patients received standard insulin treatment subcutaneously. Insulin treatment was be prescribed and collected by the patients from the pharmacy per clinical routine practice. Study patient receiving concomitant medication which may interfere with glucose regulation other than insulin treatments or other immunosuppressive treatments were withdrawn from the study e.g. oral anti-diabetic therapies, GLP-1 RA's. Subjects were allowed to take other medications which do not interfere with glucose regulation. All medications and significant non-drug therapies (including physical therapy and blood transfusions) administered after the patient starts treatment with study drug were reported.

For part I of the study, male patients 1-3 received $25 \times 10^6$ cells, patients 4-6, $100 \times 10^6$ cells and patients 7-10, $200 \times 10^6$ cells. A safety evaluation was performed between each dose cohort, with recommendation of continuing, modifying or stopping the trial.

For part II of the study: Male and female patients are assigned to one of the following three treatment arms in a ratio of 1:1:1; Either infusion with Final Product batch 1, batch 2 or a placebo infusion. The primary endpoint is evaluated in a ratio 1:2, patients treated with Final Product (batch 1 and 2) are compared to patients treated with placebo. Patients receive the Final Product as one infusion in a concentration of $20 \times 10^6$ cells/ml in an isotonic Sodium chloride (NaCl) solution with 5% HSA and 10% DMSO as defined in Example 5. Placebo infusions consist of an isotonic NaCl solution with 5% HSA and 10% DMSO but without cells and the volume is corresponding (5 ml). All infusions are administrated over approximately 20 minutes. The patients are observed for any acute reactions after the infusion for 3 hours. In the second part of the study, the fixed dose of $200 \times 10^6$ cells corresponds to $2 - 4 \times 10^6$ cells/kg, which is in the range of established doses. Therefore, no dose adjustment will be performed.

The study is completed at one year follow up after infusion of Final Product or placebo. The patients will thereafter continue standard insulin treatment.

A subgroup analysis of the patients receiving Final Product batch 1 will be compared with patients receiving Final Product batch 2. Both safety and efficacy will between the batches will be compared.

**Results**

The above described approach assures the proper application of the product and allows to study the safety and efficacy of the product.

In the Protrans study part 1 (9 treated patients) no major adverse events related to the study drug have been observed (Table 14).

*Table 14a. Adverse events after infusion of WJMSC in the first part of the trial*

| Number of patients | AE description | mild | moderate | severe | Relation-ship to Study Drug | Outcome |
|---|---|---|---|---|---|---|
| 1 | Headache | x | | | unlikely | resolved |
| 1 | Common cold with fever | x | | | unlikely | resolved |
| 1 | Tingling numbness in the body(intermittent) | x | | | unlikely | resolved |
| 1 | Symptoms of stress | x | | | unlikely | resolved |
| 1 | Elevated P-ASAT (aspartat-amino-transferase) Elevated P-ALAT (alanin-amino-transferase | x | | | unlikely | resolved |
| 1 | Food poisoning with vomiting | x | | | unlikely | resolved |
| 1 | Cut in palm | x | | | not related | resolved |

| 1 | Lumbago | x | | | not related | resolved |
| 1 | Unilateral conjunctival infection in left eye | x | | | not related | resolved |
| 1 | Upper respiratory viral infection with fever | | x | | unlikely | resolved |
| 1 | Viral rhinitis | x | | | unlikely | resolved |
| 1 | Disturbing smell of corn | x | | | definitely | resolved |
| 1 | Viral infection | | x | | unlikely | resolved |

*Table 14b.* Adverse events occurring the same day after infusion of WJMSC

| Number of patients | AE description |
|---|---|
| 1 | Confirmed no AE |
| 1 | Headache |
| 1 | Disturbing smell of corn |
| 1 | Unilateral conjunctival infection in left eye |

*Table 14c. Adverse events occurring one day or more after infusion of WJMSC.*

| Number of patients | AE description |
|---|---|
| 1 | Confirmed no AE |
| 1 | Food poisoning with vomiting |
| 1 | Lumbago |
| 1 | Viral rhinitis |
| 1 | Common cold with fever |
| 1 | Viral infection |
| 1 | Symptoms of stress |
| 1 | Upper respiratory viral infection with fever |
| 1 | Elevated P-ASAT (aspartat- amino transferase) and P-ALAT (alanin- amino-transferase) |
| 1 | Tingling numbness in the body(intermittent) |

### Example 10

The present Example describes the parameters investigated in the clinical study.

### Material and methods

The following tests are used for evaluation of the present clinical study.

*Mixed meal tolerance test:* Measurement of endogenous insulin production as C-peptide concentrations in response to a mixed meal tolerance test are performed. After an overnight fasting period, the patient performs a standardized MMTT. The patient drinks a solution of resource protein (Novartis Nestle, 6 ml/kg, maximal dose 360 ml). Blood samples are taken for analysis at time-point 0, 15, 30, 60, 90 and 120 minutes. C-peptide values in the peripheral venous blood plasma, collected at each of the time points are measured and AUC C-peptide concentration is calculated for the test.

*Optimizing diabetes care:* Continuous glucose monitoring (CGM) is performed for 72 hours using patient-blinded system. Study subjects records plasma glucose concentrations by own measurement of plasma glucose by glucose reagent strips as 7-point profiles for three consecutive days prior to visits in the study.

Insulin doses and measured plasma glucose values are written down in the subject's diary every day by the subject him/herself. Amount of insulin, recorded plasma glucose values, time and date will be included. This is used to calculate:

- Number of insulin independent patients (ADA criteria) at days 187 and 372.
- Number of patients with daily insulin needs <0.25U/kg at days 187 and 372.
- Insulin requirement/kg BW at days 187 and 372.

*Treatment Satisfaction and Quality of life*: Diabetes treatment satisfaction changes in the study will be measured using a diabetes treatment satisfaction questionnaire. Quality of life and treatment satisfaction is analyzed by a standardized Diabetes Treatment Satisfaction questionnaire at day 372 after infusion. Efficacy of treatment is measured as delta change in this parameter when compared to before start of treatment.

*Clinical safety assessments*: Physical examinations, which are performed several times during the study and prior to treatment. Physical exam includes the examination of general appearance, heart and lung auscultation. Vital signs include BP and pulse measurements. Electrocardiography is performed at all visits during the study. An ophthalmological examination with retinal inspection is performed by a specialized ophthalmologist at visits 1, 4 and 8.

*Hematology:* Hemoglobin, hematocrit, red blood cell count, white blood cell count, neutrophil count, lymphocyte count, monocyte count and platelet count are measured.

*Clinical chemistry, serology:* C-reactive protein (CRP), creatinine, cystatin C, GFR, total bilirubin, AST, ALT, alkaline phosphatase (ALP), sodium, potassium, calcium, phosphorous, albumin, and uric acid is measured prior to study. Screening for chronic infections is performed by tests for HIV, Treponema pallidum (syphilis), Hepatitis B and C. CRP, AST, ALT, ALP, total bilirubin, lipids (total cholesterol, LDL, HDL, triglycerides), sodium, potassium, calcium, phosphorous, albumin, creatinine, cystatin C, GFR and uric acid are analyzed prior to treatment and repeatedly at visits thereafter. Before and repeatedly during study, measurements are performed on P-glucose, HbA1c, C-peptide, HLA class I ab, GAD ab, and IA2 ab.

Separate blood samples are collected for measurements of circulating levels of different cytokines (IL-2, IL-6, IL-10, IL-17, IL-21, IL-33, IL-35, IFN-gamma and TGF-beta) analyzed in plasma samples by using commercially available ELISA techniques (RnD Systems, Abingdon, UK) or Mesoscale techniques. The PBMCs are further studied for determining the proportion of different immune cells such as antigen presenting cells, T-cells, regulatory T-cells, regulatory B-cells and other innate immune cells by using flow cytometry. Also, PBMCs are used for determining the immune regulatory genes expression and *in vitro* assays. Peripheral blood from the patient is used for measuring circulating levels of unmethylated INS DNA measure of ongoing beta-cell death.

**Results**

It is expected that the present example will show that treatment with Final Product formulated using the Selection algorithm as disclosed herein and pooled MSCs derived from multiple individual donors as disclosed herein, will show one or more of the following results: lower decrement of C-peptide as compared to placebo over 12 months; sustained C-peptide in the Final Product treated population; increase of C-peptide over 12 months; lower insulin dependency and lower insulin requirements compared to the control. It is expected that patients treated according to the present disclosure will exhibit a higher treatment satisfaction and patient Quality of Life compared to the control group.

Additionally, the present treatment have been shown to not generate any de novo alloimmunization. The blood sample analyzed from 9 subjects at the 4th visit, i.e. one-month post treatment, did not result in any alloimmunization irrespective of dose given. Two subjects had HLA low titers of HLA antibodies before treatment. The titer after treatment was sustained and more importantly, was not triggered by the pooled MSC. Thus, the treatment does not induce or if any,only induced low anti-HLA antibody titers in the patient. Hence, no clinically relevant induction of anti-HLA antibodies occurred. Development of HLA antibodies is known to be dose dependent. By having multiple donors, the concentration of any allogenic HLA will be lower than if cells from a single donor was used, resulting in low alloimmunization, such as clinically irrelevant antibody titers in respect to HLA immunization. There is no universal threshold value for what is considered clinically relevant antibody titers in respect to HLA immunization. However, antibody analysis by solid phase multiplex technologies has allowed for a more precise definition of the breadth and strength of HLA antibodies. By correlating these results with those obtained by an actual cell-based crossmatch, and eventual graft outcome, clinically relevant antibodies can be defined in a center-specific manner (Zachary et al. Using real data for a virtual crossmatch. Hum Immunol 2009; 70: 574-579). Furthermore, isolated pooled allogenic MSC populations according to the present disclosure are expected to show no statistically significant batch to batch variability a high tolerability level. No serious adverse events are expected, due to the selection of cells with desired functionalities and characteristics.

**Example 11**

The present Example describes how the second clinical study is performed. Herein the inventive pooled allogeneic MSC composition is referred to as Drug Product and the pharmaceutical composition as Final Product. Subjects previously treated with the Drug Product are asked for participation after completion of the clinical trial described in Example 9. The purpose of the trial is to investigate safety and efficacy of repeated treatment with the Drug Product.

**Material and Methods**

The Final Product is a cell suspension with MSC's produced from donated Wharton's Jelly from umbilical cord tissue and expanded in adherent culture over approximately 4-5 weeks (a maximum of 3 passages) as described in Examples 1-5. The treatment is allogenic and consists of pooled expanded MSC from 5-10 different donors. Minimum 72 hours before infusion, the investigator will send a requisition to the Manufacturer for delivery of the Final Product. On the day of infusion, the applicable Final Product is transported by the Manufacturer to the investigator site. The Final Product is stored in a cryo bag in liquid nitrogen transportation canister. The Final Product are thawed bed-side in a water bath with sterile saline solution and diluted in 100ml saline resulting in an infusion volume of 105 ml. The Final Product is administered to the patient within 30 minutes after preparation.

Both the subjects and the study personnel are blinded to the subject's treatment. Placebo is provided in identical tissue bags as the Final Product. Subjects, the investigator, study site personnel and sponsor personnel involved with data review and analysis, will remain blinded to study treatment throughout the study until the data base is clean and locked. Only in case of emergency, such as SAE, may unblinding occur before completion of study.

All patients will receive standard insulin treatment subcutaneously. Insulin treatment will be prescribed and collected by the patients from the pharmacy per clinical routine practice. Study patient receiving concomitant medication which may interfere with glucose regulation other than insulin treatments or other immunosuppressive treatments will be withdrawn from the study e.g. oral anti-diabetic therapies, GLP-1 RA's. Subjects are allowed to take other medications which do not interfere with glucose regulation. All medications and significant non-drug therapies (including physical therapy and blood transfusions) administered after the patient starts treatment with study drug are reported.

Only patients that completed the first (dose escalation) part of the first clinical trial will be enrolled for treatment with a second dose of the Drug Product at the same dose as they received earlier, patients 1-3 receive $25 \times 10^6$ cells, patients 4-6, $100 \times 10^6$ cells and patients 7-10, $200 \times 10^6$ cells. Additionally, 9 patients with matching baseline data will be followed as control group without any intervention.

### Example 12

The present Example describes the analysis of within batch variability. Comparison is made between the assay results from the individual donor derived MSC populations selected for pooling by the selection algorithm as disclosed herein and all the individual donor derived MSC populations (including not selected populations).

### Methods and Results

Individual donor derived MSC populations were analyzed by the IDO assay, the proliferation assay and the PGE2 assay as described in Example 3. Variation in assessment is be calculated by the following formula:

$$\text{Variation} = 1 - \frac{\text{selected (max} - \text{min)}}{\text{all (max- min)}}$$

, wherein the maximum and minimum values are the maximum and minimum assay results obtained.

The total range of IDO fold induction from cells from individual donors were 50 - 200 fold, the donors finally selected with the selection algorithm ranged from 130-200 fold. The total range of proliferation index from cells from individual donors were 1.14 - 1.07, the donors finally selected with the selection algorithm ranged from 1.13 - 1.07.The total range of PGE2 expression induced by cells from individual donors were 0.29 - 1.76, the donors finally selected with the selection algorithm ranged from 0.61 - 1.42.

Variation between donor derived MSC populations was quantified according to formula above. The results show that for IDO-assay variation decreases by 53 %, for the proliferation assay variation decreases by 14 % and for the PGE2 assay variation decreases by 45 % (see Figure 4).

The variation in the overall assessment calculated with the selection algorithm reduced the variation by 40 % when comparing the scoring of all analyzed cells and the scoring of the selected (see Figure 4).

### Example 13

The present Example describes the analysis of batch-to-batch variability between isolated pooled allogeneic MSC populations as disclosed herein.

### Methods and Results

Isolated pooled allogeneic MSC populations (Drug Product) as disclosed herein were analyzed by the IDO assay, the proliferation assay and the PGE2 assay as described in Example 3. Assay results from the Drug Product from separate batches were compared. Three batches of Drug Product has been manufactured according to method as disclosed herein with cells from pooled donors selected by the selection algorithm. The nomenclature of the Drug Product batches are, TB1 (Technical batch number 1, intended for non-clinical usage), CB1 and CB2 (Clinical batch 1 and 2, respectively, used in the clinical trial described in example 9 and 11). Drug Product from three batches TB1, CB1 and CB2, was subjected to analysis.

Each experiment is conducted two times and every batch is analysed in two replicates in each experiment.

Intra assay standard deviation and coefficient of variation was calculated based on two replicates in the same assay, i.e. the standard deviation between the two replicates, the coefficient of variance is calculated as the standard deviation divided by the mean value.

Inter assay standard deviation and coefficient of variation were calculated as the standard deviation and coefficient of variation between the average from two experiments.

The batch-to-batch standard deviation and coefficient of variance were calculated based on the average value from all replicates in both experiments for IDO, PGE2 and PI, respectively.

Results are presented in Table 15 a-c.

*Table 15a. Results from comparison of results of IDO assay.*

| IDO assay | Average IDO fold induction | Intra assay STDEV | Inter assay STDEV | Intra assay CV % | Inter assay CV % |
|---|---|---|---|---|---|
| TB1 | 316 | 23 | 11 | 7% | 3% |
| CB1 | 341 | 26 | 51 | 8% | 15% |
| CB2 | 332 | 50 | 42 | 15% | 13% |
| Average | 330 | 33 | 34 | 10% | 10% |
| Batch-to-batch standard deviation = 13, CV = 4 % | | | | | |

*Table 15b. Results from comparison of results of PGE2 assay.*

| PGE2 assay | Average PGE2 expression | Intra assay STDEV | Inter assay STDEV | Intra assay CV % | Inter assay CV % |
|---|---|---|---|---|---|
| TB1 | 4507 | 320 | 330 | 7% | 7% |
| CB1 | 5719 | 576 | 1490 | 10% | 26% |
| CB2 | 5544 | 847 | 67 | 15% | 1% |
| Average | 5256 | 581 | 629 | 11% | 12% |
| Batch-to-batch standard deviation = 655, CV = 12% | | | | | |

*Table 15c. Results from comparison of results of proliferation assay.*

| Proliferation assay | Average Proliferation Index | Intra assay STDEV | Inter assay STDEV | Intra assay CV % | Inter assay CV % |
|---|---|---|---|---|---|
| TB1 | 1,17 | 0,04 | 0,01 | 3% | 1% |
| CB1 | 1,16 | 0,01 | 0,03 | 1% | 3% |
| CB2 | 1,20 | 0,03 | 0,01 | 2% | 1% |
| Average | 1,18 | 0,02 | 0,02 | 2% | 1% |
| Batch-to-batch standard deviation = 0,02, CV = 2% | | | | | |

*Table 15d. Summary of variability analysis*

| | Intra assay CV % | Inter assay CV % | Batch-to-batch CV % |
|---|---|---|---|
| IDO | 10% | 10% | 4% |
| PGE2 | 11% | 12% | 12% |
| PI | 2% | 1% | 2% |

In summary, the present Example shows that the intra assay standard deviation between replicates and the inter assay standard deviation (between average from separate analysis), is higher than standard deviation between different batches. Thus, it is shown that that there is no difference between the batches and therefore the is no statistically significant batch-to-batch variability between the different batches of isolated pooled allogeneic MSC populations as disclosed herein.

## ITEMIZED LIST OF EMBODIMENTS

1. Method for obtaining an isolated, pooled allogeneic mesenchymal stem cell (MSC) population comprising MSCs derived from at least 3 individual donors, wherein the number of cells derived from any one donor does not exceed 50% of the total cell number and wherein said MSCs have at most been subject to eight passages; comprising the steps of:

- culturing or providing MSCs from more than said at least 3 individual donors to obtain more than at least 3 individual donor derived MSC populations;
- assaying each individual donor derived MSC population using at least 3 assays to obtain at least 3 assay results for said each individual donor derived MSC population, wherein said at least 3 assays comprise at least one assay measuring the immunosuppressive capacity each individual donor derived MSC population;
- for each assay allocating an individual ranking score value to said each individual donor derived MSC population based on the assay result and thus obtaining at least 3 individual ranking score values for each individual donor derived MSC population, wherein a higher ranking score value is indicative of more desirable assay result; or wherein a lower ranking score value is indicative of more desirable assay result;
- allocating a total score value to each individual donor derived MSC population based on said at least 3 individual ranking score values, wherein in the case of a higher ranking score value being indicative of more desirable assay result, a higher total score value is indicative of more desirable population properties; or wherein in the case of a lower ranking score value being indicative of more desirable assay result, a lower total score value is indicative of more desirable population properties;
- selecting a subset of individual donor derived MSC populations with desirable population properties based on their total score values; and
- pooling said selected individual donor derived MSC populations to obtain an isolated, pooled allogeneic mesenchymal stem cell (MSC) population.
- and

  wherein said pooled allogeneic MSC population is not further cultured after the pooling step and wherein said at least 3 assays comprise one assay measures indoleamine-2,3-dioxygensase (IDO) activity; one assay measuring prostaglandin E2 secreted by said MSCs; and one assay measuring the effect of said MSCs on the proliferation of peripheral blood mononuclear cells (PBMCs).

2. Method for obtaining an isolated, pooled allogeneic MSC population according to item 1, wherein the individual ranking score value for at least one assay is allocated to said each individual donor derived MSC population based on a comparison of the assay result for said each individual donor derived MSC population to the results for the remaining individual donor derived MSC populations.

3. Method for obtaining an isolated, pooled allogeneic MSC population according to item 1 or 2, wherein the individual ranking score value for at least one assay is allocated to said each individual donor derived MSC population based on absolute assay result obtained for said individual donor derived MSC population.

4. Method for obtaining an isolated, pooled allogeneic MSC population according to item 3, wherein the assay result is deemed desirable and an individual ranking score value that reflects the obtained desirable assay result is allocated, when said absolute result corresponds to at least a predetermined value or at most a predetermined value.

5. Method for obtaining an isolated, pooled allogeneic MSC population according to any one of items 1-4, wherein the step of selecting a subset of individual donor derived MSC populations with desirable population properties comprises selecting the individual donor derived MSC populations with total score value which corresponds to at least a predetermined value in the case wherein a higher total score value is indicative of more desirable population properties; or to at most a predetermined value lower total score value in the case wherein a lower total score value is indicative of more desirable population properties.

6. Method for obtaining an isolated, pooled allogeneic MSC population according to any one of items 1-4, wherein the step of selecting a subset of individual donor derived MSC populations with desirable population properties comprises selecting a predetermined number of the individual donor derived MSC populations, which populations exhibit a higher total score value relative the remaining individual donor derived MSC populations in the case wherein a higher total score value is indicative of more desirable population; or which populations exhibit a lower total score value relative the remaining individual donor derived MSC populations in the case wherein a lower total score value is indicative of more desirable population properties.

7. Method for obtaining an isolated, pooled allogeneic MSC population according to any one of item 1-6, wherein said MSCs have at most been subject to seven passages, such as at most six passages, such as at most five passages, such as at most four passages, such as at most three passages, such as one, two or three passages, such as two or three passages.

8. Method for obtaining an isolated, pooled allogeneic MSC population according to any one of items 1-7, wherein said MSCs are selected from the group consisting of bone marrow derived MSCs, peripheral blood derived MSCs, adipose tissue derived MSCs, dental tissue derived MSCs, placenta derived MSCs, umbilical cord derived MSCs, amniotic fluid derived MSC, cord blood derived MSCs, Wharton Jelly derived MSCs, decidua derived MSCs, chondrion membrane derived MSCs and amnion membrane derived MSCs; such as the group consisting of placenta derived MSCs, umbilical cord derived MSCs, amniotic fluid derived MSC, cord blood derived MSCs, Wharton Jelly derived MSCs, decidua derived MSCs, chondroid membrane derived MSCs, dental pulp derived MSCs and amnion membrane derived MSCs.

9. Method for obtaining an isolated, pooled allogeneic MSC population according to item 8, wherein said MSCs are selected from the group consisting of umbilical cord derived MSCs and Wharton Jelly derived MSCs, such as wherein said MSCs are Wharton Jelly derived MSCs.

10. Method for obtaining an isolated, pooled allogeneic MSC population according to any one of items 1-9, wherein said population comprises MSCs derived from at least four individual donors, such as at least five individual donors, such as at least six individual donors, such as at least seven individual donors, such as at least eight individual donors, such as at nine individual donors, such as at least ten individual donors.

11. Method for obtaining an isolated, pooled allogeneic MSC population according to any one of items 1-9, wherein said population comprises MSCs derived from 3-20 individual donors, such as 3-15 individual donors, such as 3-10 individual donors, such as 4-8 individual donors, such as 5-7 individual donors, such as 5, 6 or 7 individual donors.

12. Method for obtaining an isolated, pooled allogeneic MSC population according to any one of items 1-11, wherein the step of assaying each individual donor derived MSC population comprises assaying at least 1-4 times, such as 2-4 times, such as 2-3 or 3-4 times, as many individual donor derived MSC population as the number of individual donor derived MSC populations pooled in the pooling step.

13. Method for obtaining an isolated, pooled allogeneic MSC population according to any one of items 1-12, wherein the step of assaying each individual donor derived MSC population comprises assaying at least 3, such as at least 4, such as at least 5, such as at least 6, such as at least 7, such as at least 8, such as at least 9, such as at least 10, such as at least 11, such as at least 12, such as at least 13, such as at least 14, such as at least 15, such as at least 16, such as at least 17, such as at least 18, such as at least 19, such as at least 20 individual donor derived MSC populations.

14. Method for obtaining an isolated, pooled allogeneic MSC population according to any one of items 1-12, wherein the step of assaying each individual donor derived MSC population comprises assaying 3-50 individual donor derived MSC populations, such as 4-50, such as 5-50, such as 6-50, such as 6-30, such as 6-20, such as 6-15, such as 8-12 individual donor derived MSC population.

15. Method for obtaining an isolated, pooled allogeneic MSC population according to any one of items 1-14, the step assaying each individual donor derived MSC population using at least 3 assays comprises using as least one functional assay, such as at least two functional assays, such as at least three functional assays, such at least four functional assays, such least five functional assays.

16. Method for obtaining an isolated, pooled allogeneic MSC population according to any one of item 1-15, wherein said proliferation of PBMCs is the proliferation of T-lymphocytes, such as proliferation of phytohaemagglutinin (PHA) stimulated T-lymphocytes.

17. Method for obtaining an isolated, pooled allogeneic MSC population according to any one of items 1-16, wherein said at least one assay measuring prostaglandin E2 secreted by said MSCs comprises measuring prostaglandin E2 secreted by said MSCs when co-cultured with PBMCs, such as PHA stimulated PBMCs, such as PHA stimulated T-lymphocytes.

18. Method for obtaining an isolated, pooled allogeneic MSC population according to any one of items 1-17, wherein said at least 3 assays further comprise at least one assay measuring HLA-G expression in said MSCs in response to IFNγ, IL-10, PHA and/or GABA, such as in response to IFNγ, IL-10 and/or PHA.

19. Method for obtaining an isolated, pooled allogeneic MSC population according to any one of items 1-18, wherein said at least 3 assay further comprise at least one assay measuring the protein expression and/or cytokine expression.

20. Method for obtaining an isolated, pooled allogeneic MSC population according to item 19, wherein said at least one assay measuring the protein expression and/or cytokine expression measures the expression of one or several proteins or cytokines selected from the group consisting of IL-2, IL-4, IL-6, IL-8, IL-12, IL- 12/13, IL17A, IL-21, IL-22, IL-29, IL-31, TGFβ, VEGF, FGF, GM-CFS, IFNα, IFNγ, apo E and TNFα, such as the group consisting of IL-6, IL-8, GM-CSF and TGFβ, such as the group consisting of at least IL-6.

21. Method for obtaining an isolated, pooled allogeneic MSC population according to item 20, wherein the expression of at least 2, such as at least 3, such as at least 4, such as at least 5, such as at least 6, such as at least 7, such as at least 8, such as at least 9, such as at least 10, such as at least 11, such as at least 12, such as at least 13, such as at least 14, such as at least 15, such as at least 16, such as at least 17, such as at least 18, such as all 19 of said proteins and/or cytokines are measured.

22. Method for obtaining an isolated, pooled allogeneic MSC population according to any one of items 19-21, wherein said expression is measured in absence and/or presence of at least one stimuli.

23. Method for obtaining an isolated, pooled allogeneic MSC population according to item 22, wherein said stimuli is an immune response modifying stimuli.

24. Method for obtaining an isolated, pooled allogeneic MSC population according to item 23, wherein said immune response modifying stimuli is selected from the group consisting of PBMCs; stimulated PBMCs, such as PBMCs stimulated with PHA; IL10; gamma-aminobutyric acid (GABA); and interferon gamma (IFNy).

25. Method for obtaining an isolated, pooled allogeneic MSC population according to item 24, wherein said immune response modifying stimuli is gamma-aminobutyric acid (GABA).

26. Method for obtaining an isolated, pooled allogeneic MSC population according to item 24, wherein said stimuli is a cytokine, such as interferon gamma (IFNy).

27. Method for obtaining an isolated, pooled allogeneic MSC population according to item any one of items 22-26, wherein stimuli is selected from the group consisting of polyinosinic:polycytidylic acid (Poly I:C), resiquimod (r848), gamma-aminobutyric acid (GABA) and IFNγ, such as the group consisting of Poly I:C and IFNγ.

28. Method for obtaining an isolated, pooled allogeneic MSC population according to item any one of items 22-23, wherein stimuli is PBMCs, such as stimulated or unstimulated PBMCs, such as PHA stimulated PBMCs, such as PHA stimulated T-lymphocytes.

29. Method for obtaining an isolated, pooled allogeneic MSC population according to any one of items 1-28, wherein said at least 3 assays comprises at least one morphological assay.

30. Method for obtaining an isolated, pooled allogeneic MSC population according to item 29, wherein said morphological assay assays morphological features of cells and/or cells nuclei.

31. Method for obtaining an isolated, pooled allogeneic MSC population according to item 30, wherein said morphological features of cells and/or cells nuclei are one or more features selected from the group consisting of the size of the cell, the size of the nuclei, the shape of the cell and the ratio between cell and nuclei size.

32. Method for obtaining an isolated, pooled allogeneic MSC population according to any one of items 29-31, wherein an individual donor derived MSC population is only eligible for pooling if it exhibits more than or equal to 90%, such as at least 91%, such as at least 92%, such as at least 93%, such as at least 94%, such as at least 95%, such as at least 96%, such as at least 97%, such as at least 98%, such at least 99% normal cells and/or nuclei.

33. Method for obtaining an isolated, pooled allogeneic MSC population according to any one of items 1-32, wherein step of assaying each individual donor derived MSC population using at least 3 assays is performed when the MSC population is in passage 0 (p0) - passage 8 (p8), such as in p1 - p 5, such as in p1 - p4, such as in p2 - p4 or in p1 - p4, such as in p1, p2 and/or p3, such as in p2 and/or p3.

34. Method for obtaining an isolated, pooled allogeneic MSC population according to any one of items 1-33, at least one assay, such as at least two assays, such as at least three assays, such as all assays, is/are performed when the cells are in the same passage as when they are pooled.

35. Method for obtaining an isolated, pooled allogeneic MSC population according to any one of items 1-33, wherein at least two assays are performed at different passages.

36. Method for obtaining an isolated, pooled allogeneic MSC population according to any one of items 1-35, wherein said total score value allocated to said each individual donor derived MSC population is an additive total score value obtained by addition of ranking score values for each individual donor derived MSC population.

37. Method for obtaining an isolated, pooled allogeneic MSC population according to any one of items 1-35, wherein said total score value allocated to said each individual donor derived MSC population is a weighed total score value obtained by 1) assigning a weight to the ranking score value for each assay and 2) adding the weighed ranking score values for individual donor derived MSC population.

38. Method for obtaining an isolated, pooled allogeneic MSC population according to any one of items 1-37, wherein the step of selecting a subset of individual donor derived MSC populations with desirable population properties comprises selecting at least 3, such as at least 4, such as at least 4, such as at least 5, such as at least 6, such as at least 7, such as at least 8, such as at least 9, such as at least 10 individual donor derived MSC populations.

39. Method for obtaining an isolated, pooled allogeneic MSC population according to any one of items 1-38, in which population the number of cells derived from any one donor does not exceed 45%, such as does not exceed 40%, such as does not exceed 35%, of the total cell number and wherein said population comprises MCSs derived from at least 3 donors; such as in which population the number of cells derived from any one donor does not exceed 40%, such as does not exceed 35%, such as does not exceed 30%, of the total cell number and wherein said population comprises MCSs derived from at least 4 donors; such as in which population the number of cells derived from any one donor does not exceed 35%, such as does not exceed 30%, such as does not exceed 25%, of the total cell number and wherein said population comprises MCSs derived from at least 5 donors; such as in which population the number of cells derived from any one donor does not exceed 30%, such as does not exceed 25%, such as does not exceed 20%, of the total cell number and wherein said population comprises MCSs derived from at least 6 donors; such as in which population the number of cells derived from any one donor does not exceed 25%, as does not exceed 22 %, such as does not exceed 20 %, of the total cell number and wherein said population comprises MCSs derived from at least 7 donors.

40. Method for obtaining an isolated, pooled allogeneic MSC population according to any one of items 1-39 in which population the number of MSC derived from any one donor does not exceed four times, such as three times, such as two times the number of the cells derived any other donor.

41. Method for obtaining an isolated, pooled allogeneic MSC population according to item any one of items 1-40, further comprising the step of discarding an individual donor derived MSC population from the pooling step if the assay results for said individual donor derived MSC population are less desirable than the corresponding assay results for a pooled allogeneic MSC population previously obtained by the method according to any one of items 1-40.

42. An isolated, pooled allogeneic MSC population, obtainable by the method according to any one of items 1-41, wherein said population is not further cultured after pooling.

43. Isolated, pooled allogeneic MSC population according to item 42, and wherein said population exhibits no statistically significant batch-to-batch variability.

44. Isolated, pooled allogeneic MSC population according to item 43, for use as a medicament.

45. Isolated, pooled allogeneic MSC population according to item 44, for use in the treatment and/or prevention of a disease or condition selected from the group consisting of inflammatory diseases or conditions, autoimmune disease and transplantation rejection.

46. Isolated, pooled allogeneic MSC population according for use according to any one of items 44-45, wherein said use comprises administration of said MSC population as an infusion to patient in need thereof.

47. Isolated, pooled allogeneic MSC population according for use according to item 46, wherein said infusion is administered intravenously, intraperitoneally, intralymphatically, intravenously, intrathecally, intracerebrally, intraarterially, subcutaneously or through the ommaya reservoir; such as intravenously, intraperitoneally or intralymphatically.

48. Isolated, pooled allogeneic MSC population according for use according to any one of items 44-47, wherein said infusion is performed repeatedly.

49. Isolated, pooled allogeneic MSC population according for use according to any one of items 44-47, wherein said infusion performed one time only.

50. Isolated, pooled allogeneic MSC population according for use according to any one of item 44-49, wherein said administration induces no or low anti-HLA antibody titers in the patient.

51. Isolated, pooled allogeneic MSC population according for use according to any one of items 44-50, wherein said inflammatory disease or condition is selected from the group consisting of autoimmune diseases.

52. Isolated, pooled allogeneic MSC population according for use according to item 51, wherein said autoimmune disease is selected from the group consisting of amylotrophic lateral sclerosis, multiple sclerosis, diabetes, Crohn's diesase, ulcerative colitis and arthritis.

53. Isolated, pooled allogeneic MSC population according for use according to item 52, wherein said autoimmune disease is type 1 diabetes or Latent Autoimmune Diabetes in the Adult (LADA).

54. Isolated, pooled allogeneic MSC population according for use according to item 53, wherein said type 1 diabetes or LADA is recently diagnosed type 1 diabetes or LADA, such as type 1 diabetes or LADA diagnosed no more than 3 years prior, such as no more than 2 years prior, such as no more than 1 year prior to the administration said infusion.

55. Isolated, pooled allogeneic MSC population according for use according to any one of items 52-54, wherein said patient expresses C-peptide.

56. Isolated, pooled allogeneic MSC population according for use according to item 55, wherein said patient exhibits a fasting plasma C-peptide concentration of $\geq$0.01 nmol/L, such as $\geq$0.04 nmol/L, such as $\geq$0.08 nmol/L, such as $\geq$0.12 nmol/L.

57. Isolated, pooled allogeneic MSC population according for use according to any one of items 52-56, wherein the treatment and/or prevention results in a lower decrease rate of plasma C-peptide concentration in patients receiving the infusion compared to untreated patients.

58. Isolated, pooled allogeneic MSC population according for use according to any one of items 52-57, wherein said decrease rate of plasma C-peptide concentration in patients receiving the infusion is at least 10% lower, such as at least 20%, such as at least 30%, such as at least 40%, such as at least 50% lower than in untreated patients.

59. Isolated, pooled allogeneic MSC population according for use according to any one of items 52-58, wherein treatment and/or prevention leads to an increase in fasting plasma C-peptide concentration in the patient compared to the fasting plasma C-peptide concentration before said treatment.

60. Isolated, pooled allogeneic MSC population according for use according to item 44-50, wherein said a disease or conditions is transplant rejection.

61. Isolated, pooled allogeneic MSC population according for use according to any one of items 44-60, wherein said use comprises administration to said patient a dose of at least $3 \times 10^6$ cells, such as at least $5 \times 10^6$ cells, such as at least $10 \times 10^6$ cells, such as at least $15 \times 10^6$ cells, such as at least $20 \times 10^6$ cells at least $25 \times 10^6$ cells, such as approximately at least $50 \times 10^6$ cells, such as approximately at least $75 \times 10^6$ cells, such as approximately at least $100 \times 10^6$ cells, such as approximately at least $125 \times 10^6$ cells, such as approximately at least $150 \times 10^6$ cells, such as approximately at least $175 \times 10^6$ cells, such as approximately at least $200 \times 10^6$ cells.

62. Isolated, pooled allogeneic MSC population according for use according to any one of items 44-60, wherein said uses comprises administration to said patient a dose of at least $0,1 \times 10^6$ cells/kg bodyweight, such as at least $0.5 \times 10^6$ cells/kg bodyweight, such as at least $1 \times 10^6$ cells/kg bodyweight, such as at least $5 \times 10^6$ cells/kg bodyweight, such as at least $10 \times 10^6$ cells/kg bodyweight.

63. Isolated, pooled allogeneic MSC population according for use according to any one of items 44-62, wherein said use comprises administering to said patient a dose approximately from $0.1 \times 10^6$ cells/kg bodyweight to $10 \times 10^6$ cells/kg bodyweight, such as from $0.25 \times 10^6$ cells/kg bodyweight to $5 \times 10^6$ cells/kg bodyweight, such as from $0.25 \times 10^6$ cells/kg bodyweight to $4 \times 10^6$ cells/kg bodyweight, such as from $0.5 \times 10^6$ cells/kg bodyweight to $4 \times 10^6$ cells/kg bodyweight, such as from $1 \times 10^6$ cells/kg bodyweight to $4 \times 10^6$ cells/kg bodyweight, such as for example from $1 \times 10^6$ cells/kg bodyweight to $3 \times 10^6$ cells/kg bodyweight, such as from $2 \times 10^6$ cells/kg bodyweight to $3 \times 10^6$ cells/kg bodyweight or from $1 \times 10^6$ cells/kg bodyweight to $2 \times 10^6$ cells/kg bodyweight.

64. Pharmaceutical composition comprising an isolated, pooled allogeneic MSC population according to any one of items 44-63, and at least one pharmaceutically acceptable excipient or carrier.

65. Pharmaceutical composition comprising an isolated, pooled allogeneic MSC population according to item 64, comprising approximately at least $3 \times 10^6$ cells, such as at least $5 \times 10^6$ cells, such as at least $10 \times 10^6$ cells, such as at least $15 \times 10^6$ cells, such as at least $20 \times 10^6$ cells at least $25 \times 10^6$ cells, such as approximately at least $50 \times 10^6$ cells, such as approximately at least $75 \times 10^6$ cells, such as approximately at least $100 \times 10^6$ cells, such as approximately at least $125 \times 10^6$ cells, such as approximately at least $150 \times 10^6$ cells, such as approximately at least $175 \times 10^6$ cells, such as approximately at least $200 \times 10^6$ cells.

66. Pharmaceutical composition comprising an isolated, pooled allogeneic MSC population according to any one of items 64-65, formulated for infusion; such for intravenous infusion, intraperitoneal infusion, intralymphatical infusion, intravenous infusion, intrathecal infusion, intracerebral infusion, intraarterial infusion, subcutaneous infusion or infusion through the ommaya reservoir; such as for intravenous, intraperitoneal or intralymphatic infusion.

67. Method for treatment and/or prevention of a disease or condition selected from the group consisting of inflammatory diseases or conditions, autoimmune disease and transplantation rejection, comprising administering a therapeutically effective dose of an isolated, pooled allogeneic MSC population according to any one of items 44-63 or a pharmaceutical composition according to any one of items 64-66, to a patient in need thereof.

68. Method for treatment and/or prevention according to item 67, wherein said administration of said MSC population is by infusion; such as by intravenous infusion, intraperitoneal infusion, intralymphatical infusion, intravenous infusion, intrathecal infusion, intracerebral infusion, intraarterial infusion, subcutaneous infusion or infusion through the ommaya reservoir; such as by intravenous, intraperitoneal or intralymphatic infusion.

69. Method for treatment and/or prevention according to any one of items 67-68, wherein said infusion is performed repeatedly.

70. Method for treatment and/or prevention according to any one of items 67-68, wherein said infusion is performed one time only.

71. Method for treatment and/or prevention according to any one of items 67-70, wherein said administration induces no or low anti-HLA antibody titers in the patient.

72. Method for treatment and/or prevention according to any one of items 67-71, wherein said inflammatory disease or condition is selected from the group consisting of autoimmune diseases.

73. Method for treatment according to any one of items 67-72, wherein said autoimmune disease is selected from the group consisting of amyotrophic lateral sclerosis, multiple sclerosis, diabetes, Crohn's disease, ulcerative colitis and arthritis.

74. Method for treatment and/or prevention according to item 73, wherein said autoimmune disease is type 1 diabetes or LADA.

75. Method for treatment and/or prevention according to item 74, wherein said type 1 diabetes or LADA is recently diagnosed type 1 diabetes or LADA, such as type 1 diabetes or LADA diagnosed no more than 3 years prior, such as no more than 2 years prior, such as no more than 1 year prior to the administration, such as prior to the administration of said infusion.

76. Method for treatment and/or prevention according to any one of items 74-76, wherein said patient expresses C-peptide.

77. Method for treatment and/or prevention according to item 76, wherein said patient exhibits a fasting plasma C-peptide concentration of ≥0.01 nmol/L, such as ≥0.04 nmol/L, such as ≥0.08 nmol/L, such as ≥0.12 nmol/L.

78. Method for treatment and/or prevention according to any one of items 73-77, wherein the treatment and/or prevention results in a lower decrease rate of plasma C-peptide concentration in patients receiving the infusion compared to untreated patients.

79. Method for treatment and/or prevention according to any one of items 73-78, wherein said decrease rate of plasma C-peptide concentration in patients receiving the infusion is at least 10% lower, such as at least 20%, such as at least 30%, such as at least 40%, such as at least 50% lower than in untreated patients.

80. Method for treatment and/or prevention according to any one of items 73-79, wherein treatment and/or prevention leads to an increase in fasting plasma C-peptide concentration in the patient compared to the fasting plasma C-peptide concentration before said infusion.

81. Method for treatment according to items 71-76, wherein said disease or condition is transplant rejection.

82. Method for treatment and/or prevention according to any one of items 67-81, wherein said method comprises administering to said patient a dose of at least $3 \times 10^6$ cells, such as at least $5 \times 10^6$ cells, such as at least $10 \times 10^6$ cells, such as at least $15 \times 10^6$ cells, such as at least $20 \times 10^6$ cells at least $25 \times 10^6$ cells, such as approximately at least $50 \times 10^6$ cells, such as approximately at least $75 \times 10^6$ cells, such as approximately at least $100 \times 10^6$ cells, such as approximately at least $125 \times 10^6$ cells, such as approximately at least $150 \times 10^6$ cells, such as approximately at least $175 \times 10^6$ cells, such as approximately at least $200 \times 10^6$ cells.

83. Method for treatment and/or prevention according to any one of items 67-82, wherein said method comprises administering to said patient a dose of at least $0,1 \times 10^6$ cells/kg bodyweight, such as at least $0,5 \times 10^6$ cells/kg bodyweight, such as at least $1 \times 10^6$ cells/kg bodyweight, such as at least $5 \times 10^6$ cells/kg bodyweight, such as at least $10 \times 10^6$ cells/kg bodyweight.

84. Method for treatment and/or prevention according to any one of items 67-83, wherein said method comprises administering to said patient a dose approximately from $0.1 \times 10^6$ cells/kg bodyweight to $10 \times 10^6$ cells/kg body-weight, such as from $0.25 \times 10^6$ cells/kg bodyweight to $5 \times 10^6$ cells/kg bodyweight, such as from $0.25 \times 10^6$ cells/kg bodyweight to $4 \times 10^6$ cells/kg bodyweight, such as from $0.5 \times 10^6$ cells/kg bodyweight to $4 \times 10^6$ cells/kg bodyweight, such as from $1 \times 10^6$ cells/kg bodyweight to $4 \times 10^6$ cells/kg bodyweight, such as for example from $1 \times 10^6$ cells/kg bodyweight to $3 \times 10^6$ cells/kg bodyweight, such as from $2 \times 10^6$ cells/kg bodyweight to $3 \times 10^6$ cells/kg bodyweight or from $1 \times 10^6$ cells/kg bodyweight to $2 \times 10^6$ cells/kg bodyweight.

85. Use of an isolated, pooled allogeneic MSC population according to any one of items 44-63, in the manufacture of a medicament for the treatment of a disease or conditions selected from the group consisting of inflammatory diseases or conditions, autoimmune disease and transplantation rejection.

86. Method for evaluating of potency of a MSC population, comprising the step of: culturing or providing an MSCs population;

    assaying said MSC population using at least 3 assays to obtain said at least 3 assay results wherein said at least 3 assays comprise at least one assay measuring the immunosuppressive capacity each individual donor derived MSC population;;
    for each assay allocating a score value to said MSC population based on the assay result, wherein a higher score value is indicative of more desirable assay result; or wherein a lower score value is indicative of more desirable assay result;
    allocating a total score value to said MSC population based on the score values allocated to each assay, wherein in the case of a higher score value being indicative of more desirable assay result, a higher total score value is indicative of more desirable population properties; or wherein in the case of a lower score value being indicative of more desirable assay result, a lower total score value is indicative of more desirable population properties;
    qualifying the MSC population as potent if said total score value is above a predetermined threshold value in the case of a higher score value being indicative of more desirable assay result or qualifying the MSC population as potent if said total score value is below a predetermined threshold value in the case of a lower score value being indicative of more desirable assay result.

87. Method according to item 86, wherein said at least 3 assays comprise wherein said at least 3 assays comprise one assay measures indoleamine-2,3-dioxygensase (IDO) activity; one assay measuring prostaglandin E2 secreted by said MSCs ; and one assay measuring the effect of said MSCs on the proliferation of peripheral blood mononuclear cells (PBMCs).

88. Method according to item 86 or 87, wherein said at least 3 assays are defined according to any one of items 15-32.

89. Use of isolated, pooled allogeneic MSC population according to item 42 or 43 for co-culture of immune cells.

90. Use according to item 89, wherein said isolated, pooled allogeneic MSC population is used as feeder cells for co-culture of immune cells.

91. Use according to item 89, wherein said isolated, pooled allogeneic MSC population is used for the stimulation of immune cells co-cultured with said population.

**Claims**

1. An isolated, pooled allogeneic mesenchymal stem cell (MSC) population comprising MSCs derived from at least 3 individual donors, wherein the number of cells derived from any one donor does not exceed 50% of the total cell number and wherein said MSCs have at most been subject to eight passages, which MSC population is obtainable by the method comprising the steps of:

   - culturing or providing MSCs from more than said at least 3 individual donors to obtain more than at least 3 individual donor derived MSC populations;
   - assaying each individual donor derived MSC population using at least 3 assays to obtain at least 3 assay results for said each individual donor derived MSC population, wherein said at least 3 assays comprise at least one assay measuring the immunosuppressive capacity each individual donor derived MSC population;
   - for each assay allocating an individual ranking score value to said each individual donor derived MSC population based on the assay result and thus obtaining at least 3 individual ranking score values for each individual donor derived MSC population, wherein a higher ranking score value is indicative of more desirable assay result; or wherein a lower ranking score value is indicative of more desirable assay result;
   - allocating a total score value to each individual donor derived MSC population based on said at least 3 individual ranking score values, wherein in the case of a higher ranking score value being indicative of more desirable assay result, a higher total score value is indicative of more desirable population properties; or wherein in the case of a lower ranking score value being indicative of more desirable assay result, a lower total score value is indicative of more desirable population properties;
   - selecting a subset of individual donor derived MSC populations with desirable population properties based on their total score values; and
   - pooling said selected individual donor derived MSC populations to obtain an isolated, pooled allogeneic MSC population.
   - and

   wherein said pooled allogeneic MSC population is not further cultured after the pooling step and
   wherein said at least 3 assays comprise one assay measures indoleamine-2,3-dioxygensase (IDO) activity; one assay measuring prostaglandin E2 secreted by said MSCs; and one assay measuring the effect of said MSCs on the proliferation of peripheral blood mononuclear cells (PBMCs),
   and wherein said population exhibits no statistically significant batch-to-batch variability.

2. An isolated, pooled allogeneic MSC population according to claim 1, wherein said batch-to-batch variability is quantified by comparing the results from one or several of said at least 3 assays which were used for assaying the individual donor derived MSC populations.

3. An isolated, pooled allogeneic MSC population according to claim 1 or 2, wherein said no statistically significant batch-to-batch variability is between two consecutively produced batches.

4. An isolated, pooled allogeneic MSC population according to claim 1 or 2, wherein said no statistically significant batch-to-batch variability is between a produced batch and reference batch, wherein said reference batch is an isolated, pooled allogeneic MSC population previously produced by the method.

5. An isolated, pooled allogeneic MSC population according to any one of claims 1-4, wherein variation in the overall assessment within a batch is reduced by at least 30%, such as at least 35 %, such as at least 40 % such as at least 45 %, such as at least 50 %, such as at least 60 %, when comparing the assay results for all the individual donor derived MSC populations assayed and for the selected subset of individual donor derived MSC populations.

6. Isolated, pooled allogeneic MSC population according to claims 1-4, for use as a medicament.

7. Isolated, pooled allogeneic MSC population according to claim 5, for use in the treatment and/or prevention of a disease or condition selected from the group consisting of inflammatory diseases or conditions, autoimmune disease and transplantation rejection.

8.  Isolated, pooled allogeneic MSC population according for use according to claim 7, wherein said autoimmune disease is selected from the group consisting of amylotrophic lateral sclerosis, multiple sclerosis, diabetes, Crohn's disease, ulcerative colitis and arthritis.

9.  Isolated, pooled allogeneic MSC population according for use according to any one of claims 7-8, wherein said autoimmune disease is type 1 diabetes or Latent Autoimmune Diabetes in the Adult (LADA).

10. Isolated, pooled allogeneic MSC population for use according to claim 9, wherein the patient to be administered said population was diagnosed no more than 3 years prior with as type 1 diabetes or LADA.

11. Isolated, pooled allogeneic MSC population according for use according to any one of claims 9-10, wherein said patient expresses C-peptide.

12. Isolated, pooled allogeneic MSC population according for use according to claim 11, wherein said patient exhibits a fasting plasma C-peptide concentration of ~0.01 nmol/L, such as ~0.04 nmol/L, such as ~0.08 nmol/L, such as ~0.12 nmol/L.

13. Isolated, pooled allogeneic MSC population according for use according to any one of claims 6-12, wherein said use comprises administration to said patient a dose of at least $3 \times 10^6$ cells, such as at least $5 \times 10^6$ cells, such as at least $10 \times 10^6$ cells, such as at least $15 \times 10^6$ cells, such as at least $20 \times 10^6$ cells, such as at least $25 \times 10^6$ cells, such as approximately at least $50 \times 10^6$ cells, such as approximately at least $75 \times 10^6$ cells, such as approximately at least $100 \times 10^6$ cells, such as approximately at least $125 \times 10^6$ cells, such as approximately at least $150 \times 10^6$ cells, such as approximately at least $175 \times 10^6$ cells, such as approximately at least $200 \times 10^6$ cells.

14. Isolated, pooled allogeneic MSC population for use according to any one of claims 6-13, wherein the administration of said isolated, pooled allogeneic MSC population induces no or low anti-HLA antibody titers, such as no clinically relevant anti-HLA antibody titers, in the patient.

15. Pharmaceutical composition comprising an isolated, pooled allogeneic MSC population according to any one of claims 1-5 or an isolated, pooled allogeneic MSC population for use according to any one of claims 6-14, and at least one pharmaceutically acceptable excipient or carrier.

Figure 1

**Starting Material**

**1. Tissue collection**
- Bacterial and fungal contamination of transportation liquid
- Infectious agents screening

**2. Tissue isolation**
- Visual inspection of Umbilical Cord Tissue

**Drug Substance Intermediate**

**3. WJ-MSC expansion**
- Bacterial and fungal contamination of primary culture
- Mycoplasma and endotoxin test

**4. Master cell stock harvest**
- Cell count
- Cell viability
- Bacterial and fungal contamination of Drug substance intermediate

**Drug Substance**

**5. WJ-MSC expansion**
- Post-thaw cell morphology and adherance ability

**6. WJ-MSC harvest**
- Cell count
- Cell viability
- Bacterial and fungal contamination of Drug substance

**Drug Product**

**7. Formulation**
- Bacterial and fungal contamination of Drug product

**8. Quality control**
- Cell count and viability
- Post-thaw cell adherence
- Mycoplasm and endotoxins tests
- Bacterial and fungal contamination of Drug substance intermediate

# Figure 2

**A** Plot P06, gated on P01.R1.R2.R3.F

**B** Plot P06, gated on P01.R1.R2.R3.F

**C** Plot P06, gated on P01.R1.R2.R3.F

Histogram of CFSE fluorescence intensity in peripheral blood monocytes after 72 hours incubation. Each peak represent a generation (G0 at 10^3).

Panel A: PBMC stimulated with PHA.

Panel B: PBMC stimulated with PHA, cocultured with MSC.

Panel C: Overlay of panel A and B.

EP 4 130 252 A1

| ostim. celler | r848 | poly I:C | GABA | IFNγ | ostim. celler |

panel 1
Cells from donor 3 analysed for IL-6 expression

# Figure 3A (part 2)

| ostim.<br>celler | r848 | poly I:C | GABA | IFNγ | ostim.<br>celler |
|---|---|---|---|---|---|
| <br>617-30218 | <br>717-37434 | <br>887-44926 | <br>875-45254 | <br>1059-60749 | <br>849-42700 |
| <br>776-39836 | <br>806-39356 | <br>855-46814 | <br>821-39424 | <br>1060-57561 | <br>801-41084 |
| <br>772-38658 | <br>708-34685 | <br>769-40976 | <br>731-35398 | <br>1064-52959 | <br>713-34406 |
| <br>725-35864 | <br>785-35720 | <br>775-36965 | <br>794-39510 | <br>930-47367 | <br>762-37549 |

**panel 3**
**Cells from donor 5 analysed for IL-6 expression**

EP 4 130 252 A1

# Figure 3A (part 3)

panel 2
Cells from donor 3 analysed for IL-8 expression

## Figure 3A (part 4)

panel 4
**Cells from donor 5 analysed for IL-8 expression**

EP 4 130 252 A1

panel 1
Cells from donor 3 analysed for IL-13 expression

EP 4 130 252 A1

Figure 3B (part 2)

panel 3
Cells from donor 5 analysed for IL-13 expression

## Figure 3B (part 3)

| ostim. celler | r848 | poly I:C | GABA | IFNγ | ostim. celler |
|---|---|---|---|---|---|
| a7 516-10792 | a8 652-13756 | a9 744-15372 | a10 698-14508 | a11 756-17748 | a12 806-20391 |
| b7 552-11194 | b8 727-14500 | b9 485-8758 | b10 790-17138 | b11 773-17942 | b12 799-17387 |
| c7 593-11623 | c8 575-11181 | c9 734-14034 | c10 782-15698 | c11 763-16985 | c12 781-16486 |
| d7 740-13767 | d8 889-17766 | d9 425-7136 | d10 661-12722 | d11 732-16522 | d12 685-14148 |

panel 2
Cells from donor 3 analysed for TGFβ expression

# Figure 3B (part 4)

panel 4
Cells from donor 5 analysed for TGFβ expression

EP 4 130 252 A1

Figure 4

Europäisches
Patentamt
European
Patent Office
Office européen
des brevets

**EUROPEAN SEARCH REPORT**

Application Number

**EP 22 19 7497**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/131618 A1 (STEMPEUTICS RES PRIVATE LTD [IN]; TA MALANCHA [IN]; MAJUMDAR ANISH SEN) 4 October 2012 (2012-10-04) <br> * page 18 - page 26 * <br> * claims 1, 3, 19; figure 2; examples 1-8 * <br> ----- | 1-15 | INV. <br> C12N5/0775 <br><br> ADD. <br> A61K35/28 <br> A61K35/12 <br> C12N5/073 |
| X | NEKANTI U ET AL: "Optimization and scale-up of Wharton's jelly-derived mesenchymal stem cells for clinical applications", <br> STEM CELL RESEARCH, ELSEVIER, NL, <br> vol. 5, no. 3, <br> 1 November 2010 (2010-11-01), pages 244-254, XP027408720, <br> ISSN: 1873-5061 <br> [retrieved on 2010-09-28] | 1 | |
| A | * page 245 - page 248 * <br> * page 250, right-hand column, last paragraph - page 251, left-hand column, paragraph 1 * <br> ----- | 2-15 | |
| | | | TECHNICAL FIELDS SEARCHED (IPC) |
| A | WO 2015/016761 A2 (ISLETONE AB [SE]) 5 February 2015 (2015-02-05) <br> * claim 1 * <br> * page 23 - page 24 * <br> ----- | 1-15 | C12N <br> A61K |
| | -/-- | | |

The present search report has been drawn up for all claims

| Place of search | Date of completion of the search | Examiner |
|---|---|---|
| Munich | 14 December 2022 | Heiduschat, Carola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 22 19 7497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KE CHEN ET AL: "Human umbilical cord mesenchymal stem cells hUC-MSCs exert immunosuppressive activities through a PGE2-dependent mechanism", CLINICAL IMMUNOLOGY, vol. 135, no. 3, 1 June 2010 (2010-06-01), pages 448-458, XP055491581, US ISSN: 1521-6616, DOI: 10.1016/j.clim.2010.01.015 * page 448 * * page 451, right-hand column, last paragraph - page 452, right-hand column, last paragraph * * page 455, left-hand column, last paragraph - right-hand column, paragraph f * | 1-15 | |
| A | NAJAR M ET AL: "Mesenchymal stromal cells use PGE2 to modulate activation and proliferation of lymphocyte subsets: Combined comparison of adipose tissue, Wharton's Jelly and bone marrow sources", CELLULAR IMMUNOLOGY, ACADEMIC PRESS, SAN DIEGO, CA, US, vol. 264, no. 2, 1 January 2010 (2010-01-01), pages 171-179, XP027180763, ISSN: 0008-8749 [retrieved on 2010-06-18] * page 171, right-hand column - page 172, left-hand column * * page 175, right-hand column, last paragraph - page 177, right-hand column, paragraph f * | 1-15 | TECHNICAL FIELDS SEARCHED (IPC) |

-/--

The present search report has been drawn up for all claims

| Place of search | Date of completion of the search | Examiner |
|---|---|---|
| Munich | 14 December 2022 | Heiduschat, Carola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 19 7497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/121894 A2 (ESCAPE THERAPEUTICS INC [US]; HANTASH BASIL [US]) 9 October 2008 (2008-10-09) * paragraph [0063] * * claims 1, 8, 13-15 * * paragraph [0091] * | 1-15 | |
| A | HAIYING CHEN ET AL: "Human umbilical cord Whartons jelly stem cells: Immune property genes assay and effect of transplantation on the immune cells of heart failure patients", CELLULAR IMMUNOLOGY, ACADEMIC PRESS, SAN DIEGO, CA, US, vol. 276, no. 1, 23 March 2012 (2012-03-23), pages 83-90, XP028427466, ISSN: 0008-8749, DOI: 10.1016/J.CELLIMM.2012.03.012 [retrieved on 2012-04-11] * the whole document * | 1-15 | |
| A | MATTHEW D GRIFFIN ET AL: "Anti-donor immune responses elicited by allogeneic mesenchymal stem cells: what have we learned so far?", IMMUNOLOGY AND CELL BIOLOGY, vol. 91, no. 1, 4 December 2012 (2012-12-04), pages 40-51, XP055491590, AU ISSN: 0818-9641, DOI: 10.1038/icb.2012.67 * the whole document * | 1-15 | TECHNICAL FIELDS SEARCHED (IPC) |

-/--

The present search report has been drawn up for all claims

| Place of search | Date of completion of the search | Examiner |
|---|---|---|
| Munich | 14 December 2022 | Heiduschat, Carola |

EPO FORM 1503 03.82 (P04C01)

Europäisches
Patentamt

European
Patent Office

Office européen
des brevets

# EUROPEAN SEARCH REPORT

Application Number

EP 22 19 7497

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEUNING DANIËLLE G ET AL: "Clinical translation of multipotent mesenchymal stromal cells in transplantation", SEMINARS IN NEPHROLOGY UNITED STATES JUL,, vol. 34, no. 4, 1 July 2014 (2014-07-01), pages 351-364, XP009186147, ISSN: 1558-4488, DOI: 10.1016/J.SEMNEPHROL.2014.06.002 * the whole document * | 1-15 | |
| A | KATARINA LE BLANC ET AL: "Multipotent mesenchymal stromal cells and the innate immune system", NATURE REVIEWS IMMUNOLOGY, vol. 12, no. 5, 25 April 2012 (2012-04-25) , pages 383-396, XP055162760, ISSN: 1474-1733, DOI: 10.1038/nri3209 * the whole document * | 1-15 | |
| A | US 2009/324609 A1 (LODIE TRACEY [US] ET AL) 31 December 2009 (2009-12-31) * the whole document * | 9-15 | TECHNICAL FIELDS SEARCHED (IPC) |

The present search report has been drawn up for all claims

| Place of search | Date of completion of the search | Examiner |
|---|---|---|
| Munich | 14 December 2022 | Heiduschat, Carola |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 22 19 7497

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2022

| Patent document cited in search report | | Publication date | Patent family member(s) | | Publication date |
|---|---|---|---|---|---|
| WO 2012131618 | A1 | 04-10-2012 | NONE | | |
| WO 2015016761 | A2 | 05-02-2015 | EP | 3027738 A2 | 08-06-2016 |
| | | | ES | 2909752 T3 | 10-05-2022 |
| | | | PT | 3027738 T | 11-05-2022 |
| | | | US | 2016199413 A1 | 14-07-2016 |
| | | | WO | 2015016761 A2 | 05-02-2015 |
| WO 2008121894 | A2 | 09-10-2008 | US | 2010055785 A1 | 04-03-2010 |
| | | | US | 2014193382 A1 | 10-07-2014 |
| | | | US | 2016152953 A1 | 02-06-2016 |
| | | | WO | 2008121894 A2 | 09-10-2008 |
| US 2009324609 | A1 | 31-12-2009 | AU | 2008287063 A1 | 19-02-2009 |
| | | | CA | 2697265 A1 | 19-02-2009 |
| | | | EP | 2185689 A2 | 19-05-2010 |
| | | | EP | 2578677 A1 | 10-04-2013 |
| | | | EP | 2581441 A1 | 17-04-2013 |
| | | | US | 2009324609 A1 | 31-12-2009 |
| | | | US | 2012121611 A1 | 17-05-2012 |
| | | | WO | 2009023566 A2 | 19-02-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DOMINICI et al.** The International Society for Cellular Therapy position statement. *Cytotherapy,* 2006, vol. 8, 315-317
- **BERMAN et al.** *Diabetes,* 2010, vol. 59, 2558-2568
- **MADSBAD et al.** *Br Med J,* 1979, vol. 2 (6200), 1257-9
- **STEFFES et al.** *Diabetes Care;,* 2003, vol. 26 (3), 832-6
- **LAZARUS et al.** *Bone Marrow Transplant,* 1995, vol. 16, 557-564
- **RASMUSSON et al.** *Exp.Cell.Res,* 2005, vol. 305 (1), 33-41
- **ZACHARY et al.** Using real data for a virtual cross-match. *Hum Immunol,* 2009, vol. 70, 574-579
- **VON BAHR et al.** *Biol Blood Marrow Transplant,* 2012, vol. 18, 557-564
- **VON BAHR L et al.** *Stem Cells,* 2012, vol. 30, 1575-1578
- **HU J et al.** *Endocr J,* 2013, vol. 60, 347-357
- **CARLSSON PO et al.** *Diabetes,* 2015, vol. 64 (2), 587-92